# EUROPEAN PATENT APPLICATION

(11) **EP 4 177 272 A1**
(43) Date of publication of application: **10.05.2023**
(21) Application number: 21885284.6
(22) Date of filing: 29.10.2021
(51) Int. Cl.: C07K 16/30, C07K 19/00, C12N 15/13, C12N 15/62, A61K 39/00, A61P 35/00

(54) **USE OF REGULATOR OF ITPRIPL1 IN PREPARATION OF DRUG THAT REGULATES IMMUNE RESPONSES OR FIGHTS TUMORS**

(30) Priority: 30.10.2020 CN 202011191447; 24.05.2021 CN 202110566040
(71) Applicant: Fudan University, Shanghai 200433 (CN)
(72) Inventor: XU, Jie, Shanghai 200032 (CN); DENG, Shouyan, Shanghai 200032 (CN); SONG, Teng, Shanghai 200032 (CN); WANG, Yiting, Shanghai 200032 (CN); WANG, Yungang, Shanghai 200032 (CN); WANG, Huanbin, Shanghai 200032 (CN); CHI, Hao, Shanghai 200032 (CN)
(74) Representative: Weickmann & Weickmann PartmbB
(86) International application number: PCT/CN2021/127234
(87) International publication number: WO 2022/089557

(57) **Abstract**

Provided is a use of a regulator of ITPRIPL1 in the preparation of a drug that regulates immune responses or resists tumors, the regulator being used to increase or decrease the expression or function of the ITPRIPL1 gene or protein in an organism. Also provided is a pharmaceutical composition, which comprises the regulator used in the use. Also provided are an isolated ITPRIPL1 recombinant protein and an antibody that recognizes and binds to the ITPRIPL1. On the basis of the principle that an ITPRIPL1 protein binds to CD3ε and NRP2 receptors to regulate the functions of different immune cells, and then participates in the regulation of immune responses and a process of immune evasion of a tumor, it is confirmed that a regulator of ITPRIPL1 may be used to prepare a drug or a pharmaceutical composition, and may be applied to suppress tumors, autoimmune disease, transplant rejection, allergies and infections and other diseases.

## Description

This application claims priorities to Chinese Patent Application No. 202011191447.3, entitled "USE OF REGULATOR OF ITPRIPL1 IN PREPARATION OF DRUG THAT REGULATES IMMUNE RESPONSES OR FIGHTS TUMORS", filed to China National Intellectual Property Administration on Oct. 30, 2020, and Chinese Patent Application No. 202110566040.2, entitled "ISOLATED ANTIGENIC ITPRIPL1-BINDING PROTEIN AND USE THEREOF", filed to China National Intellectual Property Administration on May. 24, 2021, the entire contents of which are incorporated herein by reference.

### TECHNICAL FIELD

The present disclosure relates to the field of biomedicine, and specifically to a use of a regulator of ITPRIPL1 in the preparation of a drug that regulates immune responses or resists tumors.

### BACKGROUND OF THE INVENTION

ITPRIPL1-encoded protein is Inositol 1,4,5-trisphosphate receptor-interacting protein-like 1, for which the function of ITPRIPL1 has never been reported. Based on the annotation of UniProtKB database, ITPRIPL1 of human includes 555 amino acids, which are divided into an extracellular domain (1-103 amino acids), a transmembrane domain (104-124 amino acids), and an intracellular domain (125-555 amino acids).

T cells are key effector cells of adaptive immune responses, which have many important roles in eliminating pathogens and autoimmune diseases. There are several subpopulations of T cells, each with a different function. TCRs (T cell receptors) found on the surface of T cells are heterodimers composed of α and β polypeptide chains, which constitute about 95% of the TCR population, or they are composed of γ and δ polypeptide chains (Pitcher and van Oers, 2003). Each kind of polypeptide includes constant (C) and variable (V) regions. The constant regions are anchored in the cell membrane, while the variable regions extend outside the cells and are responsible for binding the antigen. The cytoplasmic short tails of TCRs are lack of the ability to transduce signals. Intracellular signaling is initiated by the CD3 protein complex, which includes intracellular immunoreceptor tyrosine-based activation motifs (ITAMs).

CD3 (Cluster of Differentiation 3) T cell co-receptors are a kind of protein complex consisting of four different chains. In mammals, a complex includes one CD3γ (γ) chain, one CD3δ (δ) chain and two CD3ε (ε) chains. These chains are correlated to TCRs and ζ chain (zeta chain), and produce activation signals in T lymphocytes. The TCRs, ζ chain and CD3 molecules together form the TCR complex. CD3γ, CD3δ and CD3ε chains are highly related cell surface protein of the immunoglobulin superfamily containing a single extracellular immunoglobulin domain. TCRs are not capable of binding free epitopes/antigens. In contrast, TCRs can bind cleaved fragments of larger polypeptides associated with a major histocompatibility complex (MHC), which is synonymous with the human leukocyte antigen (HLA) system in humans. Such an interaction occurs in a space known as the immune synapse. Class I MHC molecules are expressed on all nucleated cells of human, and present antigens to cytotoxic T cells, on which CD8 stabilizes the MHC/TCR interaction. The activation of cytotoxic T cells then leads to the destruction of target cells. Class II MHCs are found on macrophages, B cells and dendritic cells. These immune cells present antigens to helper T cells with CD4 that stabilizes the MHC/TCR interaction. The interaction between Class II MHC and TCR finally leads to antibody-mediated immune responses. Other costimulatory molecules, such as CD45, CD28 and CD2, contribute to the activation of T cells in the immune synapse and initiate the formation of TCR signalosomes which are macromolecular protein complexes responsible for intracellular signaling.

Several antibodies that bind to human CD3ε have ever been reported, for example, antibody OKT3 (see, e.g., Kung, P. et, al, Science 206 (1979) 347-349; Salmeron, A. et, al, J Immunol 147 (1991) 3047-3052), antibody UCHT1 (see, e.g., Callard, RE et, al, Clin Exp Immunol 43 (1981) 497-505) or antibody SP34 (see, e.g., Pessano, S. et, al, EMBO J 4 (1985) 337-344). Among the currently known antibodies, SP34 is cross-reactive in human and cynomolgus monkey (Conrad M.L. et, al, Cytometry A 71 (2007)925-933). It has been known that proteins directly binding to the CD3ε extracellular domain are antibody molecules, and there has never reported that natural non-antibody proteins (e.g., transmembrane proteins, secretory proteins and other typical ligands) bind to the CD3ε extracellular domain.

Neuropilin-2 (i.e., NRP-2) is a kind of receptor capable of regulating the function of immune cells (Am J Physiol Lung Cell Mol Physiol. 2018), which is reported to regulate the function of antigen-presenting cells and promote the immune evasion of tumors (Sohini Roy et, al, Cancer Res. 2018); NRP2 may affect the migration and phagocytic function of immune cells as well as the contact among immune cells (S Schellenburg et, al, Mol Immunol. 2017). Co-receptors formed from NRP2 and Plexin have negative chemotactic effects on the migration of lymphatic endothelial cells (Liu X et, al, Cell Rep. 2016). NRP2 can also regulate the NFKB signaling in cells, which is seen in the report (Rizzolio, S. et, al. Cancer Research.2017). NRP2 ligands that have been found include Semaphorin family members, but other types of ligands have not been reported.

### SUMMARY OF THE INVENTION

The present disclosure is intended to provide a method of regulating immune responses and suppressing tumors, as well as a use of a regulator of ITPRIPL1 in the preparation of a drug that regulates immune responses or resists tumors.

To achieve the above objectives, the present disclosure provides a use of a regulator of ITPRIPL1 in the preparation of a drug that regulates immune responses or resists tumors, in which the regulator is used to increase or decrease the expression or function of the ITPRIPL1 gene or protein in an organism.

Preferably, the regulator includes any one of the following:
(1) a gene editing system that enables the knockout or mutation of the ITPRIPL1 gene in cells;
(2) an RNA molecule that reduces the expression level of the ITPRIPL1 gene;
(3) a nucleic acid molecule for being introduced into a cell, the nucleic acid molecule encodes ITPRIPL1 and increases the expression level of ITPRIPL1;
(4) an isolated ITPRIPL1 recombinant protein;
(5) an antibody that recognizes and binds to the ITPRIPL1.

Preferably, the gene editing system is a CRISPR/Cas9 gene editing system; a target sequence used in the CRISPR/Cas9 gene editing system is selected from any one sequence as set forth in SEQ ID NOs: 11-13, and an oligomeric DNA sequence for encoding sgRNA is selected from SEQ ID NOs:14-19;

the nucleic acid molecule includes: a sequence as set forth in SEQ ID NO: 8, SEQ ID NO: 9 or SEQ ID NO: 10;

the ITPRIPL1 recombinant protein includes: a functional fragment capable of binding to CD3ε or NRP2 protein in an extracellular domain of the ITPRIPL1 protein;

the antibody that recognizes and binds to ITPRIPL1 is a polyclonal antibody, a monoclonal antibody, a single-chain antibody, an antigen binding domain, a bispecific antibody, a multi-specific antibody, or an antigen binding portion in a chimeric antigen receptor.

Preferably, the sequence of the functional fragment is selected from any one of SEQ ID NO: 1 to SEQ ID NO: 4, or a derivative sequence thereof. The derivative sequence includes DRMDLDTLARSRQLEKRMSEEMRxLEMEFEERxxxAExxQKxENxWxGxTSxDQ ("x" is any amino acid). The derivation method includes: substituting, deleting or inserting more than one amino acid without changing the function of the sequence.

Preferably, the ITPRIPL1 recombinant protein forms a fusion protein (as set forth in SEQ ID NOs: 5-7) with an antibody constant region, or forms a fusion protein with a coagulation factor; alternatively, the ITPRIPL1 recombinant protein is modified by means of: polyethylene glycol modification, glycosylation modification, polysialic acid modification, fatty acid modification, KLH modification, biotin modification.

Preferably, the nucleic acid molecule is introduced into the cell through a drug delivery system which includes recombinant expression vectors, viruses, lipidosome or nanomaterials.

Preferably, the regulation of immune responses includes: regulating the functions of antigen presenting cells and T lymphocytes during the processes of autoimmune responses, transplant rejection-suppressing immune responses, allergies, anti-infection immune responses, and anti-tumor immune responses.

Preferably, the immune responses include: type I diabetes, immunologic infertility, rejection after organ transplantation, allergies, systemic inflammation or cytokine storm, and infection.

Preferably, the tumors are solid tumors or hematological tumors; the solid tumors include: glioma, lung cancer, head and neck cancer, gastric cancer, colorectal cancer, thyroid cancer, esophagus cancer, urothelial carcinoma, testicular cancer, breast cancer, cervical cancer, endometrial cancer, melanoma, pancreatic cancer or liver cancer; the hematological tumors include: leukemia or lymphoma.

The present disclosure further provides a pharmaceutical composition, which includes a regulator used in the above use, and a pharmaceutically acceptable carrier.

The present disclosure further provides an isolated ITPRIPL1 recombinant protein, and the recombinant protein is a functional fragment capable of binding to CD3ε or NRP2 protein in the extracellular domain of the ITPRIPL1 protein.

The present disclosure further provides an antibody that recognizes and binds to the ITPRIPL1, and the antibody recognizes and binds to the extracellular domain of the ITPRIPL1 protein.

The present disclosure further provides an application of the isolated ITPRIPL1 recombinant protein for detecting the presence of its own anti-ITPRIPL1 antibody, wherein the main steps of detection include directly contacting the ITPRIPL1 recombinant protein with the blood sample from a subject, and washing to remove nonspecific binding.

The present disclosure further provides applications of the above antibody for detecting the content of ITPRIPL1 in a sample, and for judging the expression of ITPRIPL1 in cells, tissues, organs or individuals by the antibody that recognizes and binds to the ITPRIPL1, or for judging whether it is suitable to apply the method of the present disclosure to regulate immune responses and suppress tumors by targeting ITPRIPL1. In some embodiments, provided are applications of an antibody that specifically recognizes ITPRIPL1 for marking boundaries between cancer tissues and para-cancerous tissues in primary lesions, boundaries between cancer cells that have metastasized to lymph nodes and normal lymphatic tissues, boundaries between cancer cells with distant metastases and normal tissues of the metastatic organ, as well as marking living cancer tissue cells in other biological samples.

The present disclosure provides a use of an isolated antigenic ITPRIPL1-binding protein in the preparation of a drug that regulates immune responses or resists tumors as well as for detecting the expression of ITPRIPL1 in an individual, where, the isolated antigenic ITPRIPL1-binding protein is capable of binding to an amino acid sequence as set forth in SEQ ID NO: 49 or SEQ ID NO: 1 in the antigenic ITPRIPL1.

In some embodiments, a heavy chain variable region and a light chain variable region are included, where, the heavy chain variable region includes HCDR1, HCDR2 and HCDR3 in the heavy chain variable region VH as set forth in any one of amino acid sequence SEQ ID NO: 24 or SEQ ID NO: 34; the light chain variable region includes LCDR1, LCDR2 and LCDR3 in the light chain variable region VL as set forth in any one of amino acid sequence SEQ ID NO: 25 or SEQ ID NO: 35.

In some embodiments, in the heavy chain variable region VH in the amino acid sequence SEQ ID NO: 24, the amino acid sequence of the HCDR1 is as set forth in SEQ ID NO: 26, the amino acid sequence of the HCDR2 is as set forth in SEQ ID NO: 27, and the amino acid sequence of the HCDR3 is as set forth in SEQ ID NO: 28.

In some embodiments, in the amino acid sequence SEQ ID NO: 34, the amino acid sequence of the HCDR1 is as set forth in SEQ ID NO: 36, the amino acid sequence of the HCDR2 is as set forth in SEQ ID NO: 37, and the amino acid sequence of the HCDR3 is as set forth in SEQ ID NO: 38.

In some embodiments, in the amino acid sequence SEQ ID NO: 25, the amino acid sequence of the LCDR1 is as set forth in SEQ ID NO: 29, the amino acid sequence of the LCDR2 is KV, and the amino acid sequence of the LCDR3 is as set forth in SEQ ID NO: 31, or is more than 80% similar to an amino acid sequence as set forth in SEQ ID NO: 31.

In some embodiments, in the amino acid sequence SEQ ID NO: 35, the amino acid sequence of the LCDR1 is as set forth in SEQ ID NO: 39, the amino acid sequence of the LCDR2 is KV, and the amino acid sequence of the LCDR3 is as set forth in SEQ ID NO: 41.

In some embodiments, the heavy chain variable region includes HCDR1, HCDR2 and HCDR3 in the heavy chain variable region VH as set forth in the amino acid sequence SEQ ID NO: 24; the light chain variable region includes LCDR1, LCDR2 and LCDR3 in the light chain variable region VL as set forth in the amino acid sequence SEQ ID NO: 25.

In some embodiments, the heavy chain variable region includes HCDR1, HCDR2 and HCDR3 in the heavy chain variable region VH as set forth in the amino acid sequence SEQ ID NO: 34; the light chain variable region includes LCDR1, LCDR2 and LCDR3 in the light chain variable region VL as set forth in the amino acid sequence SEQ ID NO: 35.

In some embodiments, an antibody heavy chain constant region is included, and the antibody heavy chain constant region is derived from a human IgG heavy chain constant region.

In some embodiments, an antibody light chain constant region is included, and the antibody light chain constant region includes a human Igκ constant region.

In some embodiments, an antibody heavy chain HC is included, and the HC includes an amino acid sequence as set forth in any one of SEQ ID NO: 22 or 32.

In some embodiments, an antibody light chain LC is included, and the LC includes an amino acid sequence as set forth in any one of SEQ ID NO: 23 or 33.

In some embodiments, an antibody or an antigen binding fragment thereof is included, wherein the antigen binding fragment includes Fab, Fab', F(ab)2, Fv fragment, F(ab')2, scFv and/or di-scFv.

In some embodiments, the ITPRIPL1 protein includes human ITPRIPL1 or the ITPRIPL1 protein of cynomolgus monkey, rat, mouse, gorilla, grivet, golden snub-nosed monkey, black snub-nosed monkey, Amazon squirrel monkey.

In some embodiments, the human ITPRIPL1 protein includes an amino acid sequence as set forth in SEQ ID NO: 20.

In another aspect, the present disclosure further provides a chimeric antigen receptor (CAR), which includes the isolated antigenic ITPRIPL1-binding protein of the present disclosure.

In another aspect, the present disclosure further provides an immunoconjugate, which includes the isolated antigenic ITPRIPL1-binding protein of the present disclosure.

In another aspect, the present disclosure further provides one or more isolated nucleic acid molecules, which encode the isolated antigenic ITPRIPL1-binding protein or the chimeric antigen receptor of the present disclosure.

In another aspect, the present disclosure further provides a vector, which includes the nucleic acid molecules of the present disclosure.

In another aspect, the present disclosure further provides a cell, which includes the nucleic acid molecules or the vector of the present disclosure.

In another aspect, the present disclosure further provides a pharmaceutical composition, which includes the isolated antigenic ITPRIPL1-binding protein, the chimeric antigen receptor, the immunoconjugate of the present disclosure, and optionally a pharmaceutically acceptable adjuvant.

In another aspect, the present disclosure further provides a method for preparing the isolated antigenic ITPRIPL1-binding protein of the present disclosure, which includes culturing the cell of the present disclosure under a condition enabling the expression of the isolated antigenic ITPRIPL1-binding protein of the present disclosure.

In another aspect, the present disclosure further provides uses of the isolated antigenic ITPRIPL1-binding protein, the chimeric antigen receptor, the immunoconjugate, and/or the pharmaceutical composition of the present disclosure in the preparation of a drug which is used for preventing, alleviating and/or treating tumors. In some embodiments, the tumors include solid tumors and lymphoma.

In another aspect, the present disclosure further provides a linear epitope polypeptide that can be used for efficiently screening and preparing an ITPRIPL1 function-regulating antibody, which is characterized in that, the peptide includes: (i) an amino acid sequence of SEQ ID NO: 49, i.e., RLLEMEFEERKRAAE; (ii) or, an amino acid sequence of xxLxxxFxxRxxx (x is any amino acid), in which 1-3 amino acids at both ends can be deleted; (iii) or, an amino acid sequence obtained by substituting, inserting or deleting 1, 2, 3, 4, 5, 6, 7, 8, 9 or 10 amino acids of SEQ ID NO: 49.

The above linear epitope peptide has outstanding advantages in many aspects: it can be synthesized at low cost; the function of the ITPRIPL1 specific antibody can be determined by analyzing the binding ability of the epitope peptide with advantages of simple operation and stable and reliable detection results; the linear epitope peptide can be directly injected to animals as immunogen or be screened, thereby obtaining more effective functional antibodies than those obtained by using full-length proteins, thus improving the discovery efficiency of related drugs.

The present disclosure further provides a method for searching and identifying the function regulator of ITPRIPL1, which is characterized in that, one or more of the following properties of the test molecules are detected: ability of specifically binding to ITPRIPL1 expressed on the cell surface; effect on the binding of ITPRIPL1 to CD3ε; effect on the binding of ITPRIPL1 to NRP2; effect on the binding of ITPRIPL1 to SEMA3G; effect on the binding of ITPRIPL1 to EBI2; and effect on the function of immune cells or tumor cells.

The present disclosure creatively develops a targeting antibody that can bind to ITPRIPL1, which can bind the above target protein with high affinity and neutralize its function, and inhibit its binding to one or more ligands, thereby disabling the immune evasion function of the tumor cells and promoting the killing of tumor cells by immune cells in vitro and in vivo. The antibody provided in the present disclosure can be used as the active ingredient to prepare a drug for treating tumors, providing a new effective solution for the treatment of tumors. At the same time, the present disclosure also provides linear epitopes corresponding to the antibody with excellent neutralizing functions, and biomarkers for administering the antibody.

The conception, specific structure and technical effects produced therefrom will be further illustrated below in conjunction with the accompanying drawings, so as to fully understand the purposes, features and effects of the present disclosure.

Compared with the prior art, the present disclosure has the following beneficial effects:

The present disclosure discloses a method of regulating immune responses and suppressing tumors, which is achieved by regulating the expression or function of the ITPRIPL1 gene. The present disclosure is based on a new scientific discovery that ITPRIPL1 binds to proteins such as CD3ε, so as to regulate the functions of different immune cells, and then participate in the regulation of immune responses and the immune evasion process of tumors. The present disclosure has confirmed that the regulator of ITPRIPL1 can be used to prepare drugs or pharmaceutical compositions, with promising applications in the suppression of diseases such as tumors, autoimmune diseases, transplant rejection, allergies and infections.

### BRIEF DESCRIPTION OF THE DRAWING

FIG. 1 shows the plasmid construction results of Example 1;
FIG. 2 shows the results of the co-immunoprecipitation experiment of Example 1;
FIG. 3 is a diagram showing the colocalization results of exogenously expressed ITPRIPL1 and CD3E in cells according to Example 1;
FIG. 4 shows the construction results of the expression vector expressing the receptor-binding domain of the extracellular domain of ITPRIPL1 according to Example 2;
FIG. 5 shows the results of the co-immunoprecipitation experiment of Example 2;
FIG. 6 shows the experimental results of immunofluorescence and colocalization analysis of Example 2 that the ITPRIPL1 extracellular domain and the CD3 extracellular domain are in trans-binding;
FIG. 7 shows the Coomassie brilliant blue staining results of the ITPRIPL1-RBD recombinant protein (IT1-RBD protein) of Example 3 after gel electrophoresis (Note: ITPRIPL1 = IT1, the same below);
FIG. 8 shows the ELISA experimental results of Example 4;
FIG. 9 and FIG. 10 are diagrams showing the results of the flow cytometry in Example 5, demonstrating that the purified protein fragments from the ITPRIPL1 extracellular domain binds to Jurkat cells that highly express CD3, wherein, FIG. 9 (a) shows the threshold setting for not classified as dead cells, FIG. 9 (b) reflects the binding of Jurkat cells to different concentrations of purified protein fragments from the ITPRIPL1 extracellular domain. FIG. 10 shows specific staining and protein binding profiles under various conditions in FIG. 9 (b).
FIG. 11 and FIG. 12 are diagrams showing the results of the flow cytometry in Example 5, demonstrating that the ITPRIPL1 protein binds to cells overexpressing CD3 with higher efficiency, wherein, FIG. 11 (a) shows the threshold setting for not classified as dead cells, FIG. 11 (b) reflects the binding of cells with different CD3ε expression to different concentrations of ITPRIPL1 recombinant protein. FIG. 12 shows specific staining and protein binding under each condition in FIG. 11 (b).
FIG. 13 and FIG. 14 are diagrams showing the results of the flow cytometry in Example 5, demonstrating that CD3 binds to cells overexpressing ITPRIPL1 with higher efficiency, wherein, (a) of FIG. 13 shows the threshold setting for not classified as dead cells, (b) of FIG. 13 reflects the binding of cells with different expression of ITPRIPL1 to CD3ε protein. FIG. 14 shows specific staining and protein binding under each condition in (b) of FIG. 13, respectively;
FIG. 15 is a diagram showing the results of NFKB signaling changes as a function of the concentration of ITPRIPL1 proteins under the activation of 50 µg/ml ConA in Example 5;
FIG. 16 is a diagram showing the results of NFKB signaling changes as a function of the concentration of microsphere-coated ITPRIPL1 proteins under the activation of 50 µg/ml ConA in Example 5;
FIG. 17 shows the expression of ITPRIPL1 in different types of tumor cells after the alignment of GAPDH internal reference in Western blotting;
FIG. 18 shows the mRNA expression level of ITPRIPL1 in normal tissues and tumor tissues;
FIG. 19 is a diagram showing the results of the enzyme-linked immunosorbent assay in Example 6, demonstrating that the polyclonal antibody can bind to cells expressing ITPRIPL1;
FIG. 20 is a diagram showing the results of the enzyme-linked immunosorbent assay in Example 6, demonstrating that the polyclonal antibody can block the binding of ITPRIPL1 to CD3E;
FIG. 21 and FIG. 22 are diagrams showing the results of the flow cytometry in Example 6, demonstrating that the polyclonal antibody can block the binding of ITPRIPL1 to cells overexpressing CD3ε, wherein, (a) of FIG. 21 shows the threshold setting for not classified as dead cells, (b) of FIG. 21 reflects the changes in the binding of ITPRIPL1 to CD3ε when the concentration of the polyclonal antibody changes. FIG. 22 shows specific staining and protein binding under each condition in (b) of FIG. 21;
FIG. 23 is a diagram showing the results of the luciferin reporter assay in Example 7, demonstrating that HCT116 cells overexpressing ITPRIPL1 can reduce the NFKB proliferation signaling in Jurkat-dual cells more;
FIG. 24 is a diagram showing the results of the luciferin reporter assay in Example 7, demonstrating that CD3E protein can block the inhibition of NFKB proliferation signaling in Jurkat-dual cells by the ITPRIPL1 protein;
FIG. 25 and FIG. 26 are diagrams showing the results of the flow cytometry in Example 8, demonstrating that the ITPRIPL1-RBD recombinant protein can reduce the killing of kidney-derived H3K293 cells by human peripheral blood mononuclear cells (PBMCs), wherein, FIG. 25 (a) and (b) show the classification of 293E cells according to CD45. FIG. 25 (c) shows the relative killing activity of PBMCs calculated based on each group of apoptosis data under the condition of different ITPRIPL1 protein concentrations. FIG. 26 shows specific apoptosis staining under each condition in FIG. 25 (c);

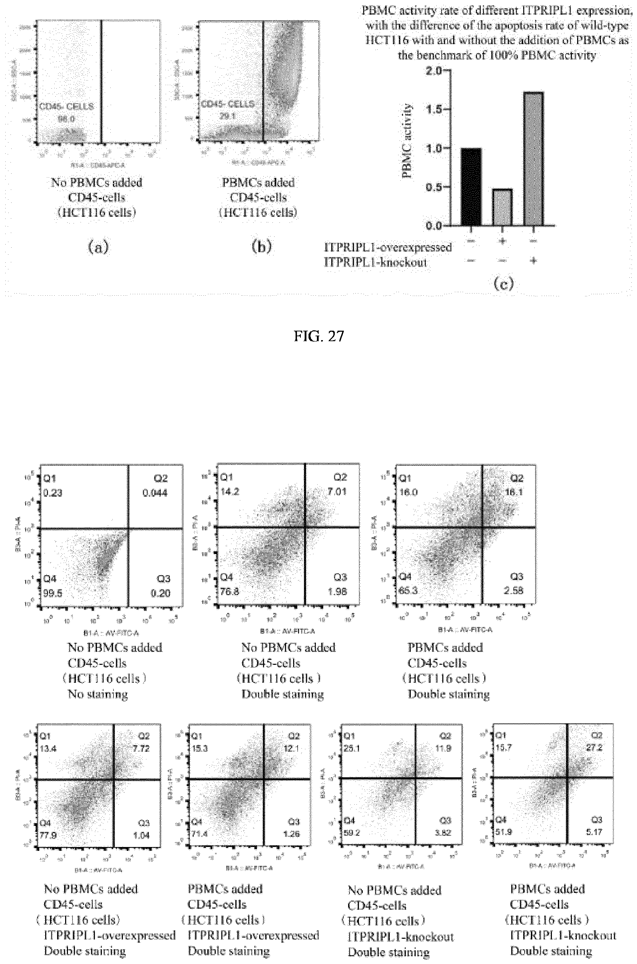

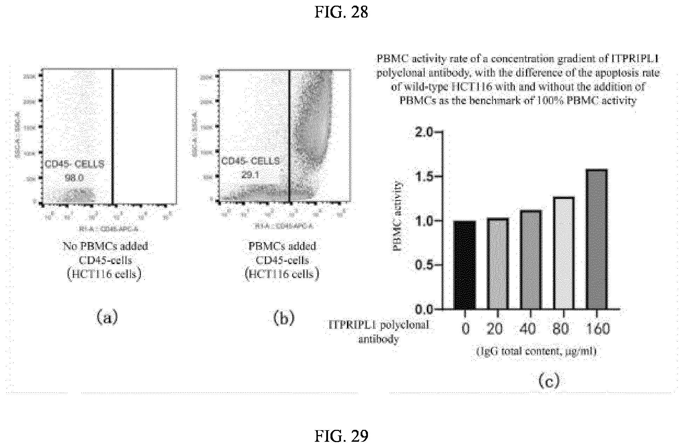

FIG. 27 and FIG. 28 are diagrams showing the results of the flow cytometry in Example 9, demonstrating that the overexpression of ITPRIPL1 can reduce the killing of tumor cells by PBMCs, while the knockout of ITPRIPL1 can promote the killing of tumor cells by PBMCs, wherein, FIG. 27 (a) and (b) indicate that HCT116 cells are divided based on CD45. FIG. 27 (c) shows the relative killing activity of PBMCs calculated based on each group of apoptosis data under the condition of different polyclonal antibody concentrations. FIG. 28 is shows specific apoptosis staining under each condition in FIG. 27 (c);
FIG. 29 and FIG. 30 are diagrams showing the results of the flow cytometry in Example 10, demonstrating that the ITPRIPL1 polyclonal antibody can promote the killing of tumor cells by PBMCs. FIG. 29 (a) and (b) indicate that HCT116 cells are divided based on CD45. FIG. 29 (c) shows the relative killing activity of PBMCs calculated based on each group of apoptosis data under the condition of different polyclonal antibody concentrations. FIG. 30 is shows specific apoptosis staining under each condition in FIG. 29 (c);
FIG. 31 shows the results of the co-immunoprecipitation experiment of Example 11;
FIG. 32 is a diagram showing the results of the enzyme-linked immunosorbent assay in Example 12;
FIG. 33 and FIG. 34 show the experimental results of the flow cytometry in Example 12, demonstrating that NRP2 binds to cells overexpressing ITPRIPL1 with higher efficiency. Wherein, FIG. 33 (a) shows the threshold setting for not classified as dead cells. FIG. 33 (b) reflects the binding of cells with different expression of ITPRIPL1 to NRP2 protein. FIG. 34 shows specific staining and protein binding under each condition in FIG. 33 (b);
FIG. 35 shows the results of the luciferin reporter assay in Example 13;
FIG. 36 shows the results of the luciferin reporter assay in Example 14;
FIG. 37 and FIG. 38 are diagrams showing the results of the flow cytometry in Example 14, demonstrating that the purifiedIT1-RBD1-Fc recombinant protein can reduce the killing of kidney-derived H3K293 cells by human peripheral blood mononuclear cells (PBMCs), wherein, FIG. 37 (a) indicate that 293E cells are divided based on CD45. FIG. 37 (b) shows the relative killing activity of PBMCs calculated based on each group of apoptosis data under the condition of different proteins, and FIG. 38 shows specific apoptosis staining under each condition in FIG. 37 (b);
FIG. 39 shows the experimental results of Western Blot in Example 14;
FIG. 40 shows the interaction of OCTET molecules in Example 5 demonstrating that the ITPRIPL1 protein can bind to CD3E protein directly;
FIG. 41 shows the experimental results of Western Blot in Example 14 that the CD3ε protein block the effect of the ITPRIPL1-RBD-Fc recombinant protein on the phosphorylation pathway;
FIG. 42 shows the experimental results of Western Blot in Example 15 showing the effect of the CD3 mutant of Jurkat on the phosphorylation pathway;
FIG. 43 shows the immunofluorescence experimental results of the ITPRIPL1-RBD-Fc recombinant protein on the Jurkat intracellular calcium ion flux in Example 15;
FIG. 44 shows the immunofluorescence experimental results showing different responses of the CD3 mutant of Jurkat to the effect of intracellular calcium ion of the ITPRIPL1-RBD-Fc recombinant protein in Example 15;
FIG. 45 shows the experimental results of Western Blot in Example 16 that the ITPRIPL1-RBD-Fc recombinant protein increases the binding of CD3 to Nek;
FIG. 46 shows the results of the proximity ligation assay in Example 16 that the ITPRIPL1-RBD-Fc recombinant protein increases the binding of CD3 to Nek;
FIG. 47 is the experimental results of monitoring the tumor volume and measuring the tumor weight in the humanized CD3ε mouse MC38 subcutaneous xenograft tumor model in Example 17;
FIG. 48 shows the experimental results of flow cytometry on PBMCs which are harvested from the humanized CD3ε mouse MC38 subcutaneous xenograft tumor model after sacrifice for analysis of T cell-related immune regulatory points in Example 17;
FIG. 49 shows the immunohistochemical results of tumor tissues from the humanized CD3ε mouse MC38 subcutaneous xenograft tumor model in Example 17;
FIG. 50 shows the experimental results of flow cytometry on PBMCs of ITPRIPL1-knockout and wild-type mice for analysis of T cell-related immune regulatory points in Example 17;
FIG. 51 shows the experimental results of ELISA analysis on the secretory cytokine in PBMCs of ITPRIPL1-knockout and wild-type mice in Example 17;
FIG. 52 shows the immumohistochemical staining results of testis tissue T cells of ITPRIPL1-knockout and wild-type mice in Example 17, as well as the analysis results of sperm morphology and motility;
FIG. 53 shows the analysis results of the expression of ITPRIPL1 in tumor and normal tissues in Example 18;
FIG. 54 shows the ITPRIPL1 sequence and function annotation as described in Example 14, wherein the bar chart shows the binding of different species;

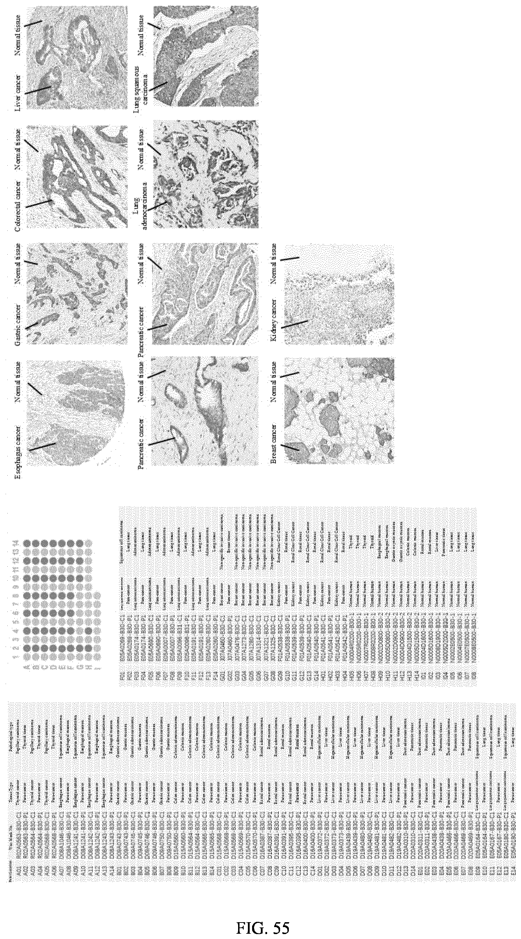

FIG. 55 shows the marking and differentiation roles of the ITPRIPL1 antibody on tumor tissues and normal tissues;
FIG. 56 shows the specific marking and differentiation roles of the ITPRIPL1 antibody to tumor cells with distant metastases;
FIG. 57 is a diagram showing the binding results of the mouse hybridoma antibody to ITPRIPL1 in the present disclosure, wherein, FIG. 57A is a diagram showing the ELISA results in which 100 hybridoma antibodies of 1 µg/ml react with ITPRIPL1 of 1 µg/ml, FIG. 57B is a diagram showing the ELISA results in which 9 sorted hybridoma antibodies of 1 µg/ml react with ITPRIPL1 of 1 µg/ml. FIG. 57C is a binding curve of 13B7 antibody to ITPRIPL1;
FIG. 58 is a diagram showing the results of the flow cytometry in the present disclosure that detects the binding of each mouse hybridoma antibody to Jurkat cells with high endogenous expression of ITPRIPL1, wherein, FIG. 58A shows the gate setting of Jurkat cells, FIG. 58B shows the statistical results of the binding rate of each hybridoma antibody, FIG. 58C-58L shows the results of flow cytometry on the binding of different hybridoma antibodies including 2E7, 5E5, 13B7, 13F7, 15C9, 16E1, 18B12, 18G5, 19B11 and 20E3 to Jurkat cells;

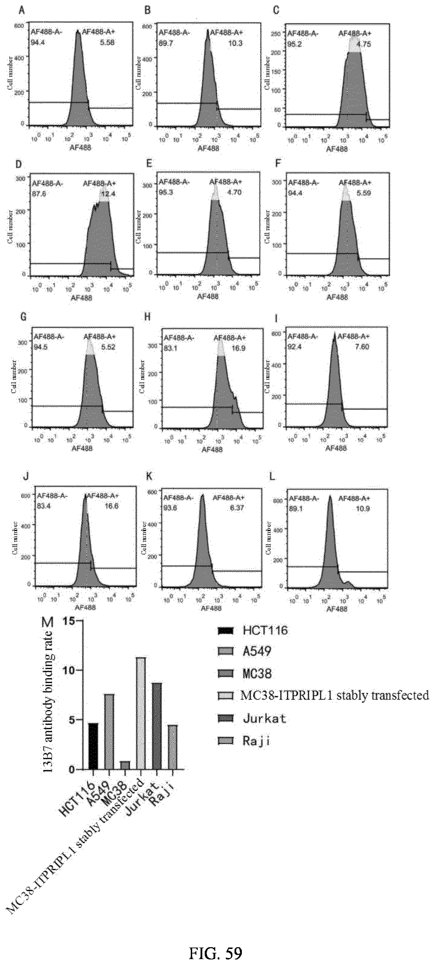

FIG. 59 is a diagram showing the results of the flow cytometry in the present disclosure that detects the binding of mouse hybridoma 13B7 antibody to various tumor cells expressing ITPRIPL1, wherein, FIG. 59A represents a group of HCT116 without antibody control, FIG. 59B represents a group of HCT116 with antibody, FIG. 59C represents a group of A549 without antibody control, FIG. 59D represents a group of A549 with antibody, FIG. 59E represents a group of MC38 without antibody control, FIG. 59F represents a group of MC38 with antibody, FIG. 59G represents a group of MC38-ITPRIPL1 stably transfected cell strains without antibody control, FIG. 59H represents a group of MC38-ITPRIPL1 stably transfected cell strains with antibody, FIG. 59I represents a group of Jurkat without antibody control, FIG. 59J represents a group of Jurkat with antibody, FIG. 59K represents a group of Raji without antibody control, FIG. 59L represents a group of Raji with antibody, FIG. 59M shows the statistical results of the binding rate of 13B7 antibody to different tumor cells expressing ITPRIPL1;
FIG. 60 a diagram showing the results of the flow cytometry in the present disclosure that detects the binding of different concentrations of mouse hybridoma 13B7 antibodies to Jurkat cells with high endogenous expression of ITPRIPL1, wherein, FIG. 60A shows the gate setting, FIG. 60B shows the negative control, FIG. 60C-FIG. 60H show the binding rates of 0.0625/0.125/0.25/0.5/1/2 µg/ml of 13B7 antibody during binding, respectively, and FIG. 601 shows the data statistical results of each group of binding rate;
FIG. 61 is a diagram showing the results of Western Blot in the present disclosure analyzing the binding of 13B7 antibody to ITPRIPL1, wherein, the Western Blot experiment is conducted with Jurkat cells with high endogenous expression of ITPRIPL1, HCT116 cells with endogenous expression of ITPRIPL1 and MC38 cells without the expression of ITPRIPL1, and the 13B7 antibody is used for incubation;
FIG. 62 shows the results of different mouse hybridoma antibody blocking the binding of ITPRIPL1 to different proteins in the present disclosure, wherein, FIG. 62A shows the results of different mouse hybridoma antibody blocking the binding of ITPRIPL1 to CD3E, and FIG. 62B shows the results of different mouse hybridoma antibody blocking the binding of ITPRIPL1 to SEMA3G;
FIG. 63 shows the ELISA results of the binding of mouse hybridoma monoclonal antibody to ITPRIPL1 in the present disclosure;
FIG. 64 is a diagram showing the results of the flow cytometry in the present disclosure that detects the binding of different mouse hybridoma monoclonal antibodies to Jurkat cells with high endogenous expression of ITPRIPL1;

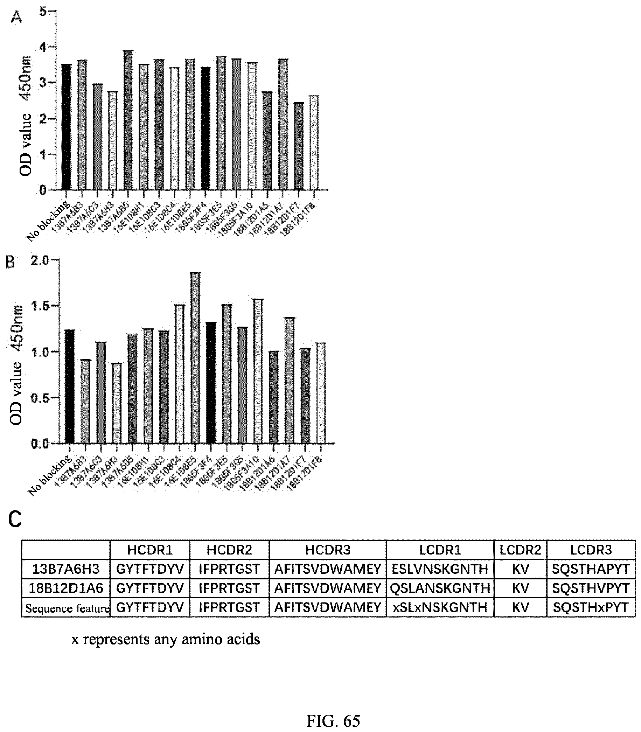

FIG. 65 is a diagram showing the statistical results in the present disclosure that different mouse hybridoma monoclonal antibodies block the binding of ITPRIPL1 to different proteins as well as the sequence comparison between two antibodies, wherein, FIG. 65A is a diagram showing the results that different mouse hybridoma monoclonal antibodies block the binding of ITPRIPL1 to CD3E, FIG. 65B is a statistical diagram showing that different mouse hybridoma antibodies block the binding of ITPRIPL1 to SEMA3G, and FIG. 65C shows the comparison and analysis between the sequences of the two antibodies;
FIG. 66 is a diagram showing the identification results of ITPRIPL1 antigen binding regions in the present disclosure. Wherein, FIG. 66A-66M are diagrams showing the statistical results of the binding of antibodies 18B12, 18B12D1A6, 13B7, 13B7A6H3, 16E1, 18G5, 20E3, 16E1D8H1, 5E5, 2E7, 19B7, 13F7, 18G5F3F4 to different peptide segments from the ITPRIPL1 protein, in turn;

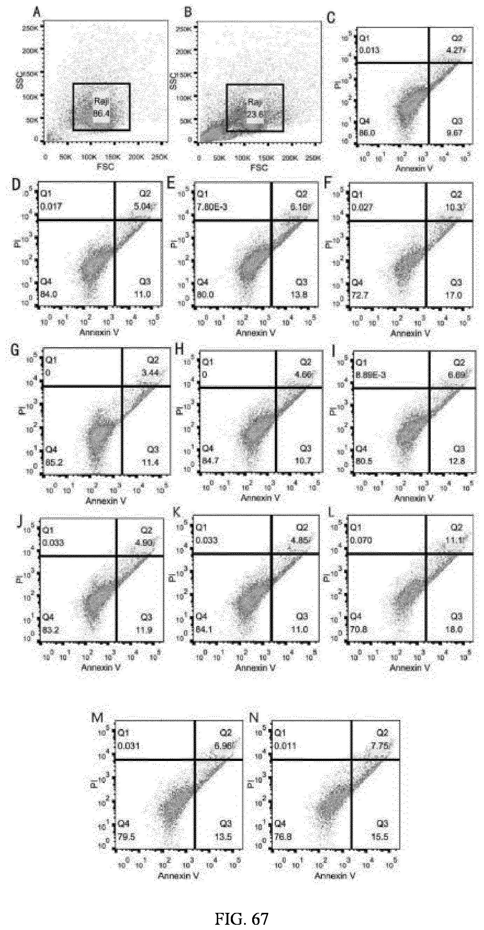

FIG. 67 is a diagram showing the detection results of flow cytometry in the present disclosure that different mouse hybridoma monoclonal antibodies promote the killing of Raji cells with high endogenous expression of ITPRIPL1 by PBMCs, wherein, FIG. 67A-B show the gate setting, FIG. 67C shows the autogenic apoptosis control of Raji cells, FIG. 67D shows the addition of PBMCs and the killing of negative serum, FIG. 67E-FIG. 67N respectively show the detection results of adding 0.5 µg/ml of 13B7A6H3 monoclonal antibody, 2 µg/ml of 13B7A6H3 monoclonal antibody, 0.5 µg/ml of 16E1D8C4 monoclonal antibody, 2 µg/ml of 16E1D8C4 monoclonal antibody, 0.5 µg/ml of 18G5F3E5 monoclonal antibody, 2 µg/ml of 18G5F3E5 monoclonal antibody, 0.5 µg/ml of 18B12D1 monoclonal antibody, 2 µg/ml of 18B12D1 monoclonal antibody, 0.5 µg/ml of 18B12D1A6 monoclonal antibody, 2 µg/ml of 18B12D1A6 monoclonal antibody while adding PBMCs;
FIG. 68 a statistical diagram showing the detection results of flow cytometry in the present disclosure that different mouse hybridoma monoclonal antibodies promote the killing of Raji cells with high endogenous expression of ITPRIPL1 by PBMCs;
FIG. 69 shows the ELISA experimental results of the binding of P8 polypeptide segments to the 13B7A6H3 monoclonal antibody after different point mutations according to the present disclosure;
FIG. 70 shows the experimental results and the corresponding antibody grouping analysis by using epitope mapping in the present disclosure;
FIG. 71 shows the experimental results of the changes in tumor volume and mass of the mouse MC38-ITPRIPL1-overexpressed subcutaneous xenograft tumor model treated with a monoclonal antibody that binds to ITPRIPL1-RBD;
FIG. 72 shows the experimental results of the flow cytometry on peripheral blood PBMCs from the mouse MC38-ITPRIPL1-overexpressed subcutaneous xenograft tumor model treated with a monoclonal antibody that binds to ITPRIPL1-RBD;
FIG. 73 shows the immumohistochemical staining results of tumor tissues from the mouse MC38-ITPRIPL1-overexpressed subcutaneous xenograft tumor model treated with a monoclonal antibody that binds to ITPRIPL1-RBD;
FIG. 74 shows the ELISA experimental results of the humanized antibody binding to the P8 polypeptide;
FIG. 75 shows the ELISA experimental results of the corresponding polypeptide of cynomolgus monkey ITPRIPL1-P8 binding to the 13B7A6H3 monoclonal antibody.

### DETAILED DESCRIPTION

Although the present invention can be implemented in many different forms, disclosed herein are specific illustrative examples thereof that verify the principles of the invention. It should be emphasized that the present invention is not limited to the specific embodiments illustrated by the examples given herein. In addition, any section headings used herein are only for organizational purposes and are not to be construed as limiting the subject matter described. Experimental methods for which the specific conditions are not indicated in the following examples are usually conducted according to conventional conditions, for example, the conditions as described in (Sambrook and Russell et, al, Molecular Cloning-A Laboratory Manual (Third Edition) (2001) CSHL Press), or the conditions as recommended by the manufacturers. Unless otherwise indicated, percentages and parts are by weight. Unless otherwise indicated, materials and reagents used in the examples of the present invention are all commercial products.

Unless otherwise defined below, all technical and scientific terms used in the detailed description of the present invention are intended to have the same meaning as commonly understood by those skilled in the art. While the following terms are believed to be easily understood by those skilled in the art, the following definitions are set forth to better explain the present invention.

The term "include", "comprise", "have", "contain" or "involve" is inclusive or open-ended, and does not exclude other unenumerated elements or process steps. The term "composed of ..." is considered as the preferable implementation of the term "comprise". If one group is defined below as comprising at least a certain number of examples, this should also be understood as disclosing a group which preferably consists only of these examples.

The indefinite or definite article used when referring to a noun in the singular form, such as "a" or "a", "the", it also includes the plural form of the noun.

Furthermore, the terms first, second, third, (a), (b), (c) and the like in the specification and claims are used to distinguish similar elements and are not necessary for the descriptive order or the chronological order. It should be understood that the terms so applied are interchangeable in appropriate circumstances, and that the examples described herein can be implemented in other sequences different from those described or exemplified herein.

The term "and/or" is considered to be a specific disclosure of each of the two specified features or components with or without the other. Therefore, the term "and/or" used in the phrase, e.g., "A and/or B" as used herein is intended to include A and B; A or B; A (alone); and B (alone). Likewise, the term "and/or" used in the phrase, e.g., "A, B and/or C" is intended to cover each of the followings: A, B and C; A, B or C; A or C; A or B; B or C; A and C; A and B; B and C; A (alone); B (alone); and C (alone).

The term "e.g." and "i.e." are only used as examples, with no intention of limiting, and should not be interpreted as only relating to items explicitly enumerated in the description.

The terms "or more", "at least", "more than" and the like, e.g., "at least one" should be understood to include, but not limited to, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or those more than the value. Any larger numbers or fractions therebetween are also included.

On the contrary, the term "not more than" includes each value less than that value. For example, "not more than 100 nucleotides" include 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1 and 0 nucleotides. Any smaller numbers or fractions therebetween are also included.

The term "multiple", "at least two", "two or more", "at least a second", and the like should be understood to include, but not limited to, at least 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or more. Any larger numbers or fractions therebetween are also included.

The term "approximately" or "substantially" indicates a range of accuracy that can be understood by those skilled in the art and can still ensure the technical effect of the feature in question. The term generally indicates a deviation of ±10%, preferably ±5%, from the indicated value.

The term "derivation" or "mutation" refers to the formation of a new sequence through substitution, deletion, insertion, or other changes of a nucleic acid or amino acid sequence, the amino acid sequence in the group which is composed of the new sequence may be at least 70%, 80%, 90%, 95% or 99% identical to the sequence in the group;
As used herein, unless otherwise specified, any concentration range, percentage range, ratio range or integer range should be understood to include any integer value within the indicated range, and, if appropriate, include fractions thereof (e.g., one-tenth and one-hundredth of the integer).

In order to make the purposes, technical solutions, and advantages of the present disclosure clearer, some of the terms involved in the present invention will be explained below:
The term "immune response" refers to the body's defensive response to a foreign component or a variant of its own component;
The term "tumor" refers to a neoplasm or solid lesion formed from the growth of abnormal cells;

The term "gene editing system" refers to the editing of target genes, specifically obtaining gene sequences by means of knockout, insertion, mutation, etc. of specific DNA fragments.

The term "sgRNA" is a small guide RNA that directs the insertion or deletion of uridine residues into the kinetoplasts during the process of RNA editing and is a kind of small non-coding RNA;
The term "antibody constant region" refers to the relatively stable region of amino acids at the C-terminus of an antibody molecule, which has many important biological functions;
The term "coagulation factor" refers to various protein components involved in the process of blood coagulation;
The term "antigen presenting cell" is a cell in the body that takes up, processes and transmits antigenic information and induces immune responses from T and B cells, mainly including macrophages, dendritic cells as well as B cells;
The term "autoimmune disease" refers to a disease in which the body develops an immune response to its own antigens, resulting in a damage to its own tissues;
The term "transplant rejection" refers to the immunological response of the recipient to a foreign tissue or organ graft after an allogeneic tissue or organ transplantation, in which the foreign tissue or organ is recognized by the recipient's immune system as a "foreign component" and the latter initiates an attack, destruction and removal against the graft;
The term "allergies" refers to a tissue damage or dysfunction that occurs when an organism that has developed immunity is re-stimulated by the same antigen;
The term "infection" refers to local tissue and systemic inflammatory responses caused by bacteria, viruses, fungi, parasites and other pathogens invading the human body;
The term "neutralizing antibody" refers to the corresponding antibody that is produced when pathogenic microorganisms invade the body. When the pathogenic microorganisms invade cells, they need to rely on specific molecules expressed by the pathogen itself to bind to receptors on the cells so as to infect the cells and further amplify. The neutralizing antibodies are certain antibodies produced by B lymphocytes, which can bind to antigens on the surface of pathogenic microorganisms, thereby preventing the pathogenic microorganisms from adhering to target cell receptors and preventing them from invading the cells;
The term "blocking antibody" binds to the molecule-acting site on the cell surface, which mainly acts to block the binding of receptors to ligands;
The term "enzyme-linked immunosorbent assay" refers to the detection of test specimens by making use of the specific bonding between antigens and antibodies; since antigens or antibodies bound to a solid support can still be immunologically active, the bonding mechanism is designed to indicate the presence of specific antigens or antibodies when combined with the coloring reaction of an enzyme, and the shade of the coloring can be used for quantitative analysis;
The term "flow cytometry" is used for the counting and sorting of tiny particles suspended in a fluid. Such a technique can be used to perform continuous multiparameter analysis of individual cells flowing through an optical or electronic detector;
The term "signaling pathway" refers to a phenomenon that when a certain response is to occur in a cell, a signal transmits a message from outside the cell to the inside of the cell, and the cell will response according to this message;

The term "immune evasion" refers to the antagonism, blockage and suppression of the body's immune responses by immunosuppressive pathogens through their structural and nonstructural products.

The term "modification" refers to the linking of a polypeptide or protein with other compounds or functional groups by means of chemical linkage, for example, antibody constant regions (Fc), polyethylene glycol modification, glycosylation modification, polysialic acid modification, fatty acid modification, KLH modification, biotin modification, etc.

In the present disclosure, the term "isolated" generally refers to artificially obtained from the natural state or synthesized artificially. If a certain "isolated" substance or component occurs in nature, it may be due to a change in its natural environment, or the substance may be isolated from its natural environment, or both. For example, a certain non-isolated polynucleotide or polypeptide naturally exists in a living animal, and the same polynucleotide or polypeptide with a high purity isolated from this natural state is called isolated. The term "isolated" does not exclude the mixing of artificial or synthetic substances, nor does it exclude the presence of other impure substances that do not affect the activity of the substance.

As used herein, the term "antibody" (Ab) includes, but not limited to, glycoprotein immunoglobulin that specifically binds to an antigen. In general, an antibody can include at least two heavy (H) chains and two light (L) chains which are connected to each other through disulfide bonds, or antigen binding molecules thereof. Each H chain includes a heavy chain variable region (abbreviated as VH herein) and a heavy chain constant region. The heavy chain constant region includes three constant domains: CH1, CH2 and CH3. Each light chain includes a light chain variable region (abbreviated as VL herein) and a light chain constant region. The light chain constant region includes one constant domain, CL. VH and VL regions can be further subdivided into hypervariable regions called complementarity determining regions (CDR) interspersed with more conservative regions called framework regions (FR). Each of VH and VL includes three CDRs and four FRs, arranged in the following order from the amino terminus to the carboxyl terminus: FR1, CDR1, FR2, CDR2, FR3, CDR3 and FR4. The variable regions of heavy chain and light chain contain binding domains interacting with antigens. The constant region of Ab can mediate the binding of immunoglobulins to host tissues or factors, including various cells of the immune system (e.g., effector cells) and the first component of the classical complement system (C1q).

The light chain variable region and the heavy chain variable region include a "framework" region interspersed with three hypervariable regions (also known as "complementarity determining region" or "CDR"), respectively. The "complementarity determining region" or "CDR region" or "CDR" or "hypervariable region" (that can be used interchangeably with hypervariable region "HVR" herein) is a region in the variable domain of an antibody, which is hypervariable in sequence and forms a structurally defined loop ("a hypervariable loop") and/or contains antigen-contacting residues ("antigen contact points"). CDRs are mainly responsible for binding to antigenic epitopes. The CDRs of the heavy chain and the light chain are generally referred to as CDR1, CDR2 and CDR3, which are numbered sequentially from the N-terminus. The CDRs within the heavy chain variable domain of an antibody are referred to as HCDR1, HCDR2 and HCDR3, and the CDRs within the light chain variable domain of an antibody are referred to as LCDR1, LCDR2 and LCDR3. In the amino acid sequence of a given light chain variable region or heavy chain variable region, the precise amino acid sequence boundary of each CDR can be determined by any one of many well-known antibody CDR assignment systems or a combination thereof, which include, for example: Chothia based on the three-dimensional structure of an antibody and the topology of CDR loops (Chothia et, al. (1989) Nature 342:877-883, Al-Lazikani et, al, "Standard conformations for the canonical structures of immunoglobulins", Journal of Molecular Biology, 273, 927-948 (1997)), Kabat based on the variability of antibody sequence (Kabat et, al, Sequences of Protein of Immunological Interest, the 4th Edition, U.S. Department of Health and Human Services, National Institutes of Health (1987)), AbM (University of Bath), Contact (University College London), International ImMunoGeneTics database (IMGT), as well as North CDR Definition based on the affinity propagation clustering using a large number of crystal structures.

However, it should be noted that, the CDR boundaries of the variable region of the same antibody obtained based on different assignment systems might differ. That is, the CDR sequences of the variable regions of the same antibody as defined by different assignment systems are different. For example, the residue ranges defined by different assignment systems for CDR regions using Kabat and Chothia numbering are shown in Table A below.

**Table A. CDR residue ranges under the definition of different assignment systems**

| Loops | Kabat CDR | AbM | Chothia | Contact | IMGT |
|---|---|---|---|---|---|
| L1 | L24-L34 | L24-L34 | L24-L34 | L30-L36 | L27-L32 |
| L2 | L50-L56 | L50-L56 | L50-L56 | L46-L55 | L50-L52 |
| L3 | L89-L97 | L89-L97 | L89-L97 | L89-L96 | L89-L96 |
| H1 | H31-H35b | H26-H35b | H26-H32...34 | H30-H35b | H26-H35b |
| | Kabat numbering | | | | |
| H1 | H31-H35 | H26-H35 | H26-H32 | H30-H35 | H26-H35 |
| | Chothia numbering | | | | |
| H2 | H50-H65 | H50-H58 | H52-H56 | H47-H58 | H51-H57 |
| H3 | H95-H102 | H95-H102 | H95-H102 | H93-H101 | H93-H102 |

Therefore, when referring to instances where an antibody is defined by specific CDR sequences as defined in the present invention, the scope of the antibody also encompasses antibodies whose variable region sequences include the specific CDR sequences, but due to the application of different schemes (for example, different assignment system rules or combination thereof), the claimed CDR boundaries may be different from the specific CDR boundaries as defined in the present invention.

The CDRs of the antibodies of the present invention can be evaluated manually to determine the boundaries according to any protocols in the art or a combination thereof. Unless otherwise stated, in the present invention, the term "CDR" or "CDR sequence" encompasses CDR sequences determined in any one of the above ways.

Antibodies can include, for example, a monoclonal antibody, a recombinantly produced antibody, a monospecific antibody, a multi-specific antibody (including a bispecific antibody ), a human antibody, an engineered antibody, a humanized antibody, a chimeric antibody, immunoglobulin, a synthetic antibody, a tetrameric antibody comprising two heavy chain and two light chain molecules, an antibody light-chain monomer, an antibody heavy-chain monomer, an antibody light-chain dimer, an antibody heavy-chain dimer, an antibody light chain-antibody heavy chain pair, an intracellular antibody, an antibody fusion (herein sometimes referred to as "antibody conjugate"), a heteroconjugate antibody, a single-domain antibody, a monovalent antibody, a single-chain antibody or a single-chain Fv (scFv), a camelid antibody, an affibody, a Fab fragment, a F(ab')2 fragment, Fv(sdFv) linked through a disulfide bond, an anti-idiotype (anti-Id) antibody (including, for example, anti-anti-Id antibody), a minibody, a domain antibody, a synthetic antibody (herein sometimes referred to as "antibody mimic")and antigen binding fragments of any of the above.

The term "humanized antibody" is intended to refer to an antibody obtained by grafting a CDR sequence derived from the germline of another mammalian species, such as mouse, onto the human framework sequence. Other framework region modifications can be made in human framework sequences.

As used herein, "antigen binding molecules", "antigen binding fragments" or "antibody fragments" refer to any molecules including antigen binding fragments (for example, CDR) of an antibody from which the molecules are derived. The antigen binding molecules may include antigen complementarity determining regions (CDRs). Examples of antibody fragments include, but not limited to, Fab, Fab', F(ab')2 and Fv fragments formed from antigen binding molecules, dAb, linear antibodies, scFv antibodies and multi-specific antibodies. In some embodiments, antigen binding molecules bind to ITPRIPL1 protein. In some embodiments, antigen binding molecules have neutralizing activities so that they can inhibit the binding of ITPRIPL1 to the receptor CD3E or EBI2.

The term "chimeric antigen receptors" (i.e., CARs) includes extracellular domains, transmembrane domains and possible intracellular domains, the extracellular domains being composed of protein domains that recognize and bind to specific antigens. The chimeric antigen receptors can be expressed in immune cells and regulate their ability to interact with target cells.

The term "immunoconjugate" may include antibody immunoconjugate (that is, antibody-drug conjugate, ADC), in which biologically active small-molecule drugs are linked to antibodies through chemical linkage. Similarly, the immunoconjugate also includes protein-drug conjugates, nucleic acid-drug conjugates.

As used herein, the term "antigen" refers to any molecules that induce immune responses or can be bound by antibody or antigen binding molecules. Immune responses may involve the production of antibodies or the activation of specific immunocompetent cells or both. Those skilled in the art will readily understand that any macromolecules (including almost all the proteins or peptides) can serve as antigens. Antigens can be expressed endogenously, i.e., they can be expressed by genomic DNA or can be expressed recombinantly. Antigens may be specific to certain tissues, e.g., cancer cells, or they may be expressed widely. Furthermore, fragments of larger molecules can serve as antigens. In some embodiments, the antigens are ITPRIPL1 protein antigens.

As used herein, in some embodiments, antigen binding molecules, scFv, antibodies or fragments thereof block the binding sites on the ligands directly or change the binding ability of the ligands indirectly (for example, by changing the structure or energy of the ligands). In some embodiments, antigen binding molecules, scFv, antibodies or fragments thereof prevent the proteins to which they bind from performing their biological functions.

As used herein, the terms "peptide", "polypeptide" and "protein" can be used interchangeably and refer to compounds comprising amino acids residues covalently linked through peptide bonds. Proteins or peptides contain at least two amino acids and there is no limitation to the maximum number of amino acids that can include the sequence of the protein or peptide. Polypeptides include any peptides or proteins that comprise two or more amino acids linked to each other via peptide bonds. As used herein, this term refers to both short chains (in the art, they are also generally referred to as, for example, peptides, oligopeptides and oligomers) and longer chains (in the art, they are generally referred to as proteins, of many types). "Polypeptides" include, for example, biologically active fragments, substantially homologous polypeptides, oligopeptides, homologous dimers, heterologous dimers, variants of the polypeptides, modified polypeptides, derivatives, analogues, fusion proteins, etc. Polypeptides include native peptides, recombinant peptides, synthetic peptides or a combination thereof.

As used herein, the term "specific binding" or "specifically binding to" refers to a non-random binding reaction between two molecules, for example, between an antibody and an antigen.

As used herein, the ability of "inhibiting the binding", "blocking the binding" or "competing for the same epitope" refers to the ability of an antibody to inhibit the binding of two molecules to any detectable degree. In some embodiments, the antibody blocking the binding of two molecules inhibits the binding interaction between the two molecules by at least 50%. In some embodiments, the inhibition may be greater than 20%, 30%, greater than 40%, greater than 50%, greater than 60%, greater than 70%, greater than 80% or greater than 90%.

As used herein, the term "Ka" is intended to indicate the association rate of the specific antibody-antigen interaction, while the term "Kd" used herein is intended to indicate the dissociation rate of the specific antibody-antigen interaction. As used herein, the term "KD" or "KD value" is intended to indicate the dissociation constant of the specific antibody-antigen interaction, which is obtained from the ratio of Kd to Ka (i.e., Kd/Ka) and represented as molar concentration (M). The KD value of an antibody can be determined using a well-established method in the art.

As used herein, the term an antibody of "high affinity" refers to an antibody with a KD value of 1×10⁻⁷ M or lower, more preferably 5×10⁻⁸ M or lower, even more preferably 1×10⁻⁸ M or lower, even more preferably 5×10⁻⁹ M or lower, and even more preferably 1×10⁻⁹ M or lower, against the target antigen.

As used herein, the term "epitope" refers to the portion of an antigen to which an immunoglobulin or antibody specifically binds. The "epitope" is also referred to as "antigenic determinant". The epitope or antigenic determinant is usually composed of chemically active surface groups on the side chain of molecules such as amino acids, carbohydrates, or sugar, and usually has a specific three-dimensional structure and specific charge features. For example, an epitope usually includes at least 3, 4, 5, 6, 7, 8, 9, 10, 11, 12, 13, 14 or 15 continuous or discontinuous amino acids in a unique stereoscopic conformation, which can be a "linear epitope" or "conformational epitope". See, for example, Epitope Mapping Protocols in Methods in Molecular Biology, Vol. 66, G. E. Morris, Ed. (1996). In the linear epitope, all interaction sites between a protein and an interacting molecule (e.g., an antibody) are arranged linearly along the primary amino acid sequence of the protein. In the conformational epitope, interaction sites span amino acid residues in the protein that are separated from each other. Depending on the competition of binding identical epitopes as detected by conventional techniques known to those skilled in the art, the antibodies can be screened. For example, competition or cross-competition studies can be conducted to obtain antibodies that compete or cross-compete with each other to bind antigens (e.g., CLDN18.2). International Patent Application WO 03/048731 describes a high-throughput method for obtaining antibodies that bind identical epitopes, which is based on their cross-competition.

The term "nucleic acid" or "nucleic acid sequence" in the present invention refers to any molecules, preferably polymeric molecules, comprising units of ribonucleic acid, deoxyribonucleic acid or analogues thereof. The nucleic acid may be single-stranded or double-stranded. The single-stranded nucleic acid may be the nucleic acid of one strand of denatured double-stranded DNA. Alternatively, the single-stranded nucleic acid may be a single-stranded nucleic acid not deriving from any double-stranded DNA.

The term "complementary" as used herein relates to the hydrogen-bonded base pairing between nucleotide bases G, A, T, C and U, such that when two given polynucleotides or polynucleotide sequences anneal to each other, A pairs with T and G pairs with C in DNA, and G pairs with C and A pairs with U in RNA.

As used herein, the term "cancer" refers to a large group of various diseases characterized by the uncontrolled growth of abnormal cells in the body. Unregulated cell division and growth result in the formation of malignant tumors that invade adjacent tissues and can also metastasize to distal parts of the body through the lymphatic system or bloodstream. "Cancers" or "cancerous tissues" can include tumors, such as: bone cancer, pancreatic cancer, skin cancer, head or neck cancer, malignant melanoma of the skin or eye, uterine cancer, ovarian cancer, rectal cancer, anal cancer, gastrointestinal cancer, testicular cancer, uterine cancer, fallopian tube cancer, endometrial cancer, cervical cancer, vaginal cancer, vulva cancer, Hodgkin's disease, non-Hodgkin's lymphoma, esophageal cancer, small intestine cancer, cancer of the endocrine system, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma, bladder cancer, renal or ureteral cancer, renal pelvis cancer, neoplasms/tumors of central nervous system (CNS), primary CNS lymphoma, tumor angiogenesis, spinal axis tumors, brainstem gliomas, pituitary adenomas, Kaposi's sarcoma, epidermoid carcinoma, squamous cell carcinoma, T-cell lymphoma, environmentally induced cancers (including those induced by asbestos), and combinations of these cancers.

As used herein, the term "ITPRIPL1-associated cancers" refers to any cancers caused by, exacerbated by, or otherwise associated with, increased or decreased expression or activity of ITPRIPL1. In some embodiments, the method disclosed herein can be used in the treatment of a cancer selected from colorectal cancer, lung cancer, breast cancer, melanoma, lymphoma, liver cancer, head and neck cancer, gastric cancer, kidney cancer, bladder cancer, prostatic cancer, testicular cancer, endometrial cancer, breast cancer, and ovarian cancer.

As used herein, an "effective dose", "effective amount" or "therapeutically effective dose" is any amount that, when used alone or in combination with another therapeutic agent, protects the subject from the onset of a disease or promotes the disease regression. Evidences of the disease regression include a decrease in the severity of disease symptoms, an increase in the frequency and duration of asymptomatic periods of the disease or prevention of injury or disability due to disease affliction. The ability of a therapeutic agent to promote disease regression can be assessed using a variety of methods known to the skilled practitioner, such as by assessing in human subjects during clinical trials, by assessing in animal model systems used to predict efficacy in humans, or by determining the activity of the reagent in an in vitro assay.

As used herein, an "individual" or "subject" is a mammal. Mammals include primates (for example, human and non-human primates, such as monkey) and rodents (for example, mice and rats). In some embodiments, an individual or subject is human. The "subject" can be a "patient"-the patient is a human subject in need of treatment, which may be an individual with ITPRIPL1-associated cancer such as breast cancer, or a subject with the risk of having an ITPRIPL1-associated cancer such as breast cancer.

As used herein, the term "in vitro cells" refers to any cells cultured ex vivo. Particularly, in vitro cells may include T cells.

As used herein, the term "pharmaceutically acceptable" refers to that the vector, diluent, excipient and/or salts thereof are chemically and/or physically compatible with other components in the preparation, and physiologically compatible with the recipient.

As used herein, the term "pharmaceutically acceptable carrier and/or excipient" refers to the carrier and/or excipient that are pharmacologically and/or physiologically compatible with the subject and the activating agent, which are well known in the art (see, e.g., Remington's Pharmaceutical Sciences. Edited by Gennaro AR, 19th ed. Pennsylvania: Mack Publishing Company, 1995), and include, but not limited to, a pH regulator, a surfactant, an adjuvant and an ionic strength enhancer. For example, the pH regulator includes, but not limited to, phosphate buffer; the surfactant includes, but not limited to, cationic, anionic or nonionic surfactant, for example, Tween-80; and the ionic strength enhancer includes, but not limited to, sodium chloride.

As used herein, the term "modulate" or "regulate" generally includes the meaning of upward or downward regulation in two different directions, which in some cases can be understood as inhibition or enhancement, in some cases can be understood as reduction or improvement, in some cases can be understood as decreasing or increasing, etc. Its specific interpretation is not limited, and it should be understood and interpreted according to the actual application context. Exemplarily, in some embodiments, "modulating" the tumor cell growth can be understood as inhibiting or enhancing the tumor cell growth.

As used herein, the term "decreasing" and "reduction" are used interchangeably and indicate any changes that are less than the original. "Decreasing" and "reduction" are relative terms, and it requires a comparison before and after measurement. "Decreasing" and "reduction" include complete consumption; likewise, the terms "increasing" and "improvement" are interpreted on the contrary.

The "treatment" or "treating" of a subject means any type of intervention or process performed on the subject, or the administration of an activating agent to the subject, for the purpose of reversing, alleviating, ameliorating, suppressing, slowing, or preventing the onset, progression, development, severity, or recurrence of a symptom, complication, or condition or biochemical indicators associated with a disease. In some embodiments, "treatment" or "treating" includes partial remission. In another embodiment, "treatment" or "treating" includes complete remission.

The term "the expression of ITPRIPL1 in an individual" refers to the expression level of the protein or mRNA of ITPRIPL1 in normal human and patients, including the contents in diseased tissues such as tumor tissues or autoimmune disease tissues, blood samples, urine, feces and other biological samples. This expression level can be used as a diagnostic biomarker for a disease, or as a companion diagnostic marker for a related drug.

The technical solutions of the present disclosure will be further illustrated below in conjunction with the attached drawings and examples.

The present disclosure firstly discloses that, the ITPRIPL1 extracellular domain binds to CD3ε extracellular domain and NRP2 extracellular domain, and also discloses the regulation roles of ITPRIPL1 on T cells and antigen presenting cells respectively after binding to CD3ε and NRP2. Based on new scientific findings, the present disclosure discloses a method for regulating immune responses and suppressing tumors by targeting ITPRIPL1 and discloses a use of a regulator of ITPRIPL1 in the preparation of a drug that regulates immune responses or resists tumors. Specifically, the present disclosure provides a method for editing the ITPRIPL1 gene, and a method for introducing genetic materials into cells to regulate the expression of ITPRIPL1. The present disclosure further discloses the receptor-binding domain (RBD) of ITPRIPL1 and provides a protocol for preparing ITPRIPL1-RBD isolated protein, and demonstrates a method for binding ITPRIPL1-RBD to CD3ε and NRP2 so as to regulate the function of immune cells. Furthermore, the present disclosure exemplifies a method for preparing an ITPRIPL1 antibody, and demonstrates that the antibody can enhance the anti-tumor effect of immune cells. The method for regulating immune responses and suppressing tumors by targeting ITPRIPL1 as disclosed in the present disclosure shows the value of ITPRIPL1 as a target in the treatment of tumors, autoimmune diseases, transplant rejection, allergies, infections and other diseases.

There are no previous reports on the function of ITPRIPL1 gene. The present disclosure discloses ITPRIPL1 as a newly identified ligand for CD3ε (Example 1) and elucidates the receptor-binding domain (RBD) of the ITPRIPL1 extracellular domain as amino acids 25-103 (Example 2), on the basis of which isolated recombinant proteins with the function of regulating CD3ε and T cells are prepared (Example 3). The present disclosure also discloses the preparation, use and administration method of the above isolated proteins.

Further, the present disclosure discloses the ability of the isolated ITPRIPL1-RBD protein to bind CD3ε (Example 4), and the ability of the ITPRIPL1-RBD isolated protein to bind to CD3ε extracellular domain has been demonstrated by using enzyme-linked immunosorbent assay (ELISA), flow cytometry or any other experimental methods, respectively.

Since ITPRIPL1-RBD (seen SEQ ID NO: 1 for its sequence) is the key region for binding CD3ε, the isolated ITPRIPL1-RBD protein can be prepared by means of recombinant expression and can be used to bind the CD3ε protein on the cell surface and transmit inhibitory signals into T cells (Example 5), so the isolated ITPRIPL1-RBD protein can be used to regulate the function of T cells. Although the present disclosure has provided the roles of the isolated protein of sequence SEQ ID NO: 1 in binding to CD3ε, regulating T cells and preparing neutralizing antibodies, other protein sequences with similar functions obtained by non-creative derivation methods (such as, truncation, insertion, mutation or fusion, etc.) that are easy to implement by those skilled in the art also fall within the scope as claimed in the present disclosure.

The present disclosure discloses an application of the isolated ITPRIPL1-RBD as immunogen in the preparation of neutralizing antibodies (Example 6). In the case that ITPRIPL1 has not been disclosed as a ligand for CD3ε, those skilled in the art cannot purposefully prepare antibodies for blocking the binding of ITPRIPL1 to CD3. The present disclosure discloses the existence of the pair of receptor ligands CD3ε and ITPRIPL1, defines the key structure for the binding of ITPRIPL1 to CD3, and provides a preparation method of the ITPRIPL1-RBD isolated protein, making the preparation of antibodies for blocking the binding of ITPRIPL1 to CD3 become an easily achievable goal by those of skills in the art through conventional techniques (e.g., hybridoma, phage display, etc.). Therefore, the blocking antibodies prepared by using the fragment from ITPRIPL1 extracellular domain as the immunogen also fall within the scope of the claims of the present disclosure.

The present disclosure also discloses that, the activation of proliferation signaling pathway of T cell-derived cell lines is regulated by regulating the binding of ITPRIPL1-RBD to the CD3 extracellular domain (Example 7).

The present disclosure discloses that, ITPRIPL1-RBD recombinant protein can reduce the killing of kidney-derived H3K293 cells by human peripheral blood mononuclear cells (PBMCs) (Example 8), on the basis of which the application of the targeting ITPRIPL1 in inhibiting autoimmunity, as well as its application value in the preparation of pharmaceutical compositions for treating autoimmune diseases, transplant rejection, allergies, infections and other diseases are proposed.

The present disclosure discloses that, the ITPRIPL1 that reduces the expression of tumor cells can significantly increase the killing of tumor cells by human peripheral blood mononuclear cells (Example 9), supporting the role of ITPRIPL1 in the immune evasion of tumors, on the basis of which the important value of ITPRIPL1 as the target of the immunotherapy of tumors is proposed.

The present disclosure also discloses that, the antibodies prepared by using the ITPRIPL1-RBD protein as the immunogen effectively promote the killing of tumor cells by immune cells (Example 10). This verifies the important value of ITPRIPL1 as the target for immunotherapy of tumors, and establishes a method for blocking the binding of ITPRIPL1 to CD3 with the antibodies. The antibodies represent a class of antibodies with completely new functions, i.e., antibodies capable of recognizing ITPRIPL1 and blocking its binding to CD3ε, thereby regulating the function of T cells.

Furthermore, by comparing the fragments from the ITPRIPL1 extracellular domain of different length, the present disclosure demonstrates that the ability of the ITPRIPL1-RBD2 sequence to bind CD3ε is slightly reduced, while the ability of the ITPRIPL1-RBD3 to bind CD3 is significantly reduced, thereby disclosing the positive correlation between the length of the ITPRIPL1 extracellular domain and the ability to bind CD3ε and characterizing the essential properties of a functional ITPRIPL1 extracellular domain sequence (Example 11). The present disclosure discloses that the ITPRIPL1-RBD protein has the ability of binding NRP2 (Example 12). And the isolated ITPRIPL1-RBD protein has the ability of transmitting inhibitory signals to differentiated THP1 macrophages that express the NRP2 protein (Example 13).

Modification methods are often used in the development of drugs to improve their pharmacokinetic properties, in which polypeptides or proteins can be linked to other compounds or functional groups, for example, antibody constant regions (Fc), polyethylene glycol modification, glycosylation modification, polysialic acid modification, fatty acid modification, KLH modification, biotin modification, etc. The present disclosure exemplifies the effect of modification on the function of the ITPRIPL1 recombinant protein by means of Fc modification. The ITPRIPL1-RBD-Fc modified protein obtained after purification has the functions of inhibiting T cell pathway signaling and killing (Example 14). Furthermore, the ITPRIPL1-RBD-Fc protein can inhibit the phosphorylation pathway of T cells, and can be blocked by CD3ε (Example 14).

The present disclosure prepares Jurkat CD3 mutants and further discloses the related mechanism of affecting the phosphorylation pathway (Example 15). At the same time, the effect of ITPRIPL1-Fc on Jurkat cells with different CD3 expression is explored by detecting the intracellular calcium ion flux (Example 15), and find that ITPRIPL1-Fc regulates the pathway by increasing the binding of CD3 to Nek (Example 16).

Furthermore, the present disclosure is applied to an in vivo animal model to construct a MC38 subcutaneous xenograft tumor model in a humanized CD3ε mouse, so as to detect the tumor growth, the function of T cells in PBMCs, and the infiltration of T cells in tumor cells (Example 17). At the same time, an ITPRIPL1 knockout mouse model is established to detect the function of T cells in PBMCs, the changes of cytokine, and the infiltration of testicular T cells, thereby verifying that the present disclosure can be applied in vivo (Example 17).

The present disclosure discloses the expression of ITPRIPL1 in common multi-organ cancers and compares it with the corresponding para-cancerous tissues, confirming that ITPRIPL1 is increased in carcinomas (Example 18).

### Example 1: ITPRIPL1 as a newly identified ligand for CD3ε

### 1. Construction of expression plasmid

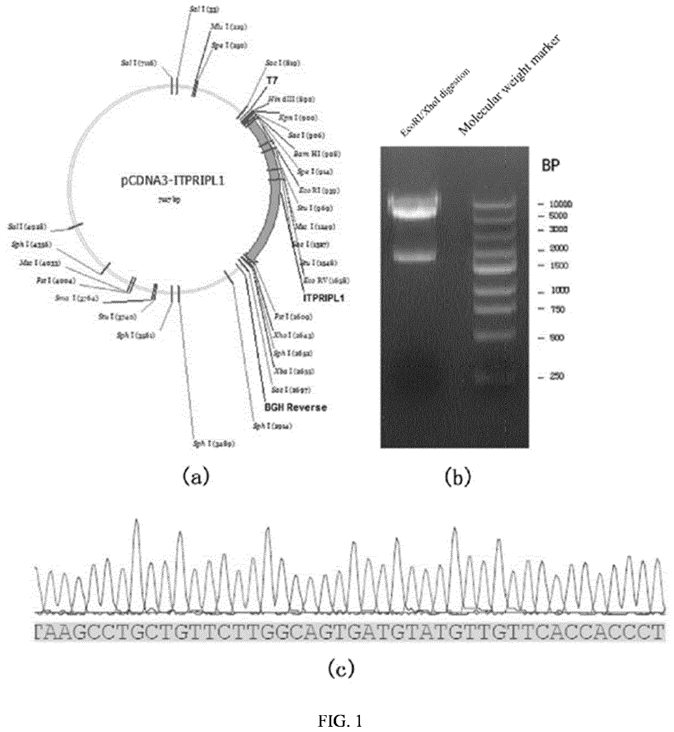

Based on the published sequence (NCBI reference sequence NM_001008949.3), a Flag-tagged ITPRIPL1 plasmid was produced by synthesizing the cDNA of a full-length human ITPRIPL1 with pcDNA3.1 as the vector, in which the C terminus was fused to the Flag tag. The results of plasmid construction were shown in FIG. 1. FIG. 1 (a) is a schematic diagram of the vector cloning structure, FIG. 1 (b) is a DNA gel electrophoretogram of the gene expression vector plasmid after being digested by EcoRI/XhoI, and the size of the resulting fragments was as expected, and FIG. 1 (c) shows a partial interception of the vector sequencing verification results.

2. The analysis results of co-immunoprecipitation and Western Blot demonstrate that ITPRIPL1 binds to CD3.

The pcDNA3.1 plasmids containing Flag-tagged ITPRIPL1 (or empty) and HA-tagged CD3ε were co-transfected into HCT1 16 cells (ATCC, VA, USA), and cultured in a 6-well plate (Corning, NY, USA) for 48-72 hours until the protein was fully expressed, and then lysed with a hybrid lysate of immunoprecipitation lysate (Thermo Fisher, MA, USA) mixed with a triple of protease-phosphatase-PMSF (Consun, Shanghai, China) at 1:100, and the cells were scraped. A portion of the cell samples were centrifuged, mixed with loading buffer (Beyotime, Shanghai, China) and denatured in a metal bath at 100°C to obtain an input level of protein samples; the remaining cell samples were immunoprecipitated with Flag-tagged specific mouse antibodies (CST, MA, USA), washed with PBS, mixed with the loading buffer (Beyotime, Shanghai, China) and denatured in a metal bath at 100°C to obtain immunoprecipitated protein samples. And then, 12.5% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of the ITPRIPL1-Flag and CD3ε-HA protein. After blocking with rapid blocking buffer (Epizyme, Shanghai, China) for 10 minutes, the corresponding ITPRIPL1-Flag and CD3ε-HA strips were respectively incubated with Flag-tagged specific rabbit antibodies (Abeam, MA, USA) and HA-tagged specific rabbit antibodies (CST, MA, USA) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

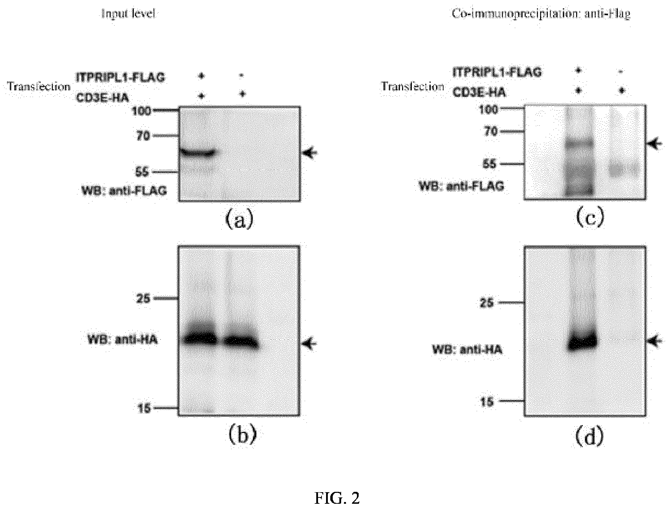

The results of the co-immunoprecipitation experiment were shown in FIG. 2. FIG. 2 (a) and (b) show the contents of ITPRIPL1 and CD3ε in the input protein for co-immunoprecipitation, respectively, that is, the input levels; FIG. 2 (c) shows the directly precipitated ITPRIPL1, and FIG. 2 (d) shows the indirectly precipitated CD3ε that binds to ITPRIPL1. The results of the co-immunoprecipitation experiment demonstrated the binding of ITPRIPL1 to CD3.

3. Immunofluorescence and colocalization analysis demonstrates that ITPRIPL1 has significant colocalization with CD3.

The pcDNA3.1 plasmid containing Flag-tagged ITPRIPL1 (or empty) and HA-tagged CD3ε was co-transfected into HCT116 cells (ATCC, VA, USA), cultured in an 8-well glass slide (Thermo Fisher, MA, USA) for 30 hours until the protein was fully expressed, and then the culture medium was discarded. After washing with PBS, it was fixed with 4% paraformaldehyde for 20 minutes, washed with PBS again and blocked with a membrane-permeable blocking buffer for 1 hour, and then Flag-tagged specific mouse antibodies (CST, MA, USA) and HA-tagged specific rabbit antibodies (CST, MA, USA) which had been diluted with the membrane-permeable blocking buffer were added and incubated at 4°C overnight. After washing the 8-well glass slides with PBS overnight, Alexa Fluor 488 fluorescence specific anti-mouse antibodies (Invitrogen, CA, USA) and Alexa Fluor 594 fluorescence specific anti-rabbit antibodies (Invitrogen, CA, USA) which had been diluted with PBS were added and incubated at room temperature for 20 minutes. After washing with PBS, the slides were mounted with DAPI, and observed under a fluorescence microscope after the mounting medium was dried.

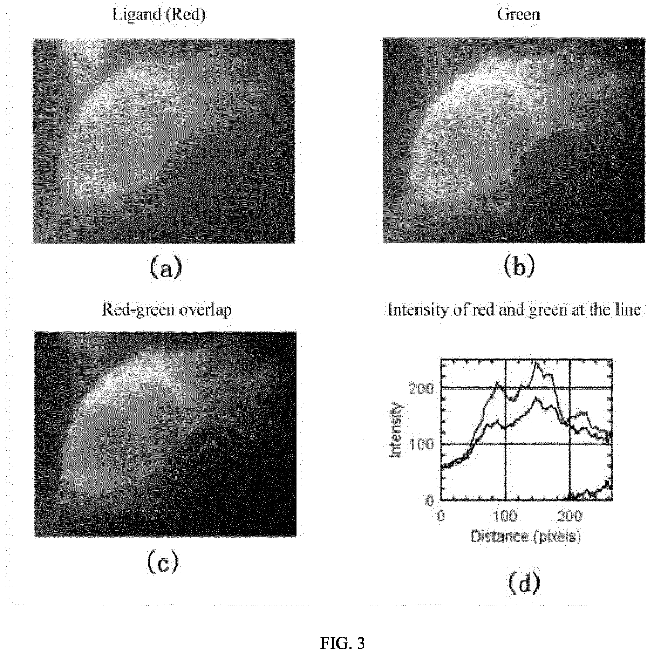

FIG. 3 shows the significant colocalization between the exogenously expressed ITPRIPL1 and CD3ε in the cells. FIG. 3 (a) shows the localization pattern of ITPRIPL1; (b) shows the localization pattern of CD3ε protein; (c) shows the superposition of the protein localization patterns of two colors, wherein the fluorescence intensity of ITPRIPL1 and CD3ε on the white straight path was shown in FIG. 3 (d). It can be observed from the above results that the localizations of the two proteins have significantly correlation, supporting the mutual binding of two proteins.

### Example 2: Functions of ITPRIPL1 extracellular domain receptor-binding domains (RBDs) and derivative sequences thereof

### 1. Construction of expression plasmid

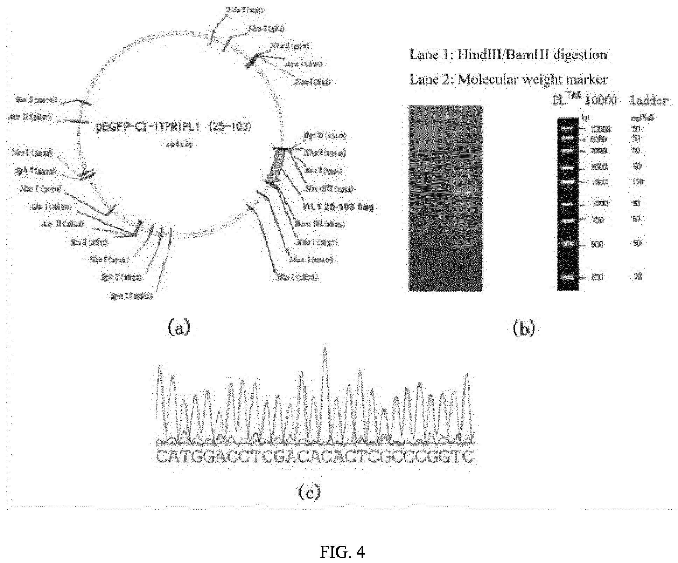

Based on the published sequence (NCBI reference sequence NM_001008949.3), from which an extracellular domain (25-103 amino acids), an extracellular domain-and-transmembrane domain (25-124 amino acids), a transmembrane domain-and-intracellular domain (104-555 amino acids) were respectively selected as three different target sequences to synthesize a full-length human ITPRIPL1 with pcDNA3.1 as the vector, wherein the C termini of the extracellular domain and the extracellular domain-and-transmembrane domain were fused to Flag tags and green fluorescent proteins (GFPs), the N terminus of the transmembrane domain-and-intracellular domain was fuse to Flag tags and green fluorescent proteins (GFPs), thereby producing ITPRIPL1 extracellular domain, extracellular domain-and-transmembrane domain, transmembrane domain-and-intracellular domain plasmids containing Flag tags and green fluorescent proteins (GFPs). Wherein, the construction results of the expression vector of ITPRIPL1 (25-103), i.e., the CD3 binding domain, were shown in FIG. 4. FIG. 4 (a) shows the construction map of the vector plasmid. The cDNA encoding the amino acid fragment of ITPRIPL1 (25-103) was linked to the cDNA encoding the Flag tag at the end, and inserted into pEGFP-C1 (Shanghai Generay Biotech Co., Ltd). The vector expression product carries GFP fluorescent markers, but its main function product is ITPRIPL1 (25-103), i.e., the CD3 binding fragment. FIG. 4 (b) is a DNA gel electrophoretogram of the gene expression vector plasmid after being digested by EcoRI/XhoI, and the size of the resulting fragments was as expected, and FIG. 4 (c) shows a screenshot of the peak plot of the sequencing results.

2. The results of the co-immunoprecipitation experiment demonstrate that the ITPRIPL1 extracellular domain (rather than the intracellular domain or the transmembrane domain) binds to CD3ε.

The pcDNA3.1 plasmids containing the Flag-tagged ITPRIPL1 extracellular domain, the extracellular domain-and-transmembrane domain, the intracellular domain-and-transmembrane domain and HA-tagged CD3ε were respectively co-transfected into HCT116 cells (ATCC, VA, USA), and cultured in a 6-well plate (Corning, NY, USA) for 48-72 hours until the protein was fully expressed, and then lysed with a hybrid lysate of immunoprecipitation lysate (Thermo Fisher, MA, USA) mixed with a triple of protease-phosphatase-PMSF (Consun, Shanghai, China) at 1:100, and the cells were scraped. A portion of the cell samples were centrifuged, mixed with loading buffer (Beyotime, Shanghai, China) and denatured in a metal bath at 100°C to obtain an input level of protein samples; the remaining cell samples were immunoprecipitated with Flag-tagged specific mouse antibodies (CST, MA, USA), washed with PBS, mixed with the loading buffer (Beyotime, Shanghai, China) and denatured in a metal bath at 100°C to obtain immunoprecipitated protein samples. And then, 12.5% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of the ITPRIPL1 extracellular domain, the extracellular domain-and-transmembrane domain, the intracellular domain-and-transmembrane domain-Flag and the CD3ε-HA protein. After blocking with rapid blocking buffer (Epizyme, Shanghai, China) for 10 minutes, the corresponding ITPRIPL1-Flag and CD3ε-HA strips were respectively incubated with Flag-tagged specific rabbit antibodies (Abeam, MA, USA) and HA-tagged specific rabbit antibodies (CST, MA, USA) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

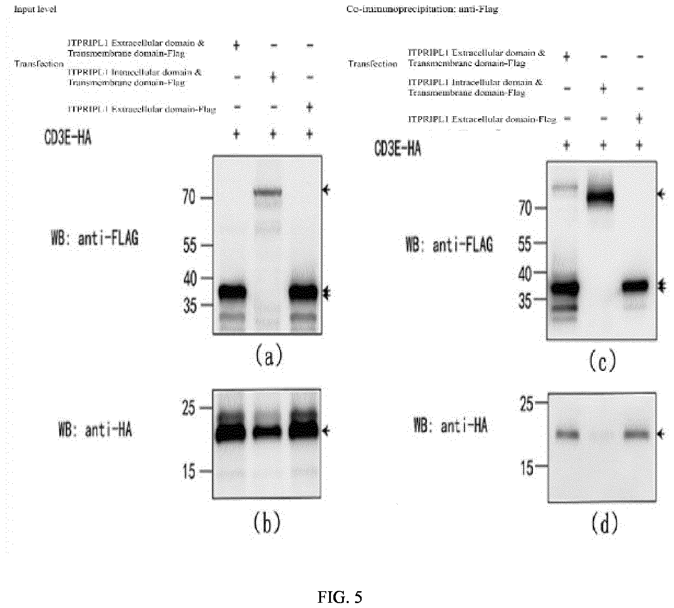

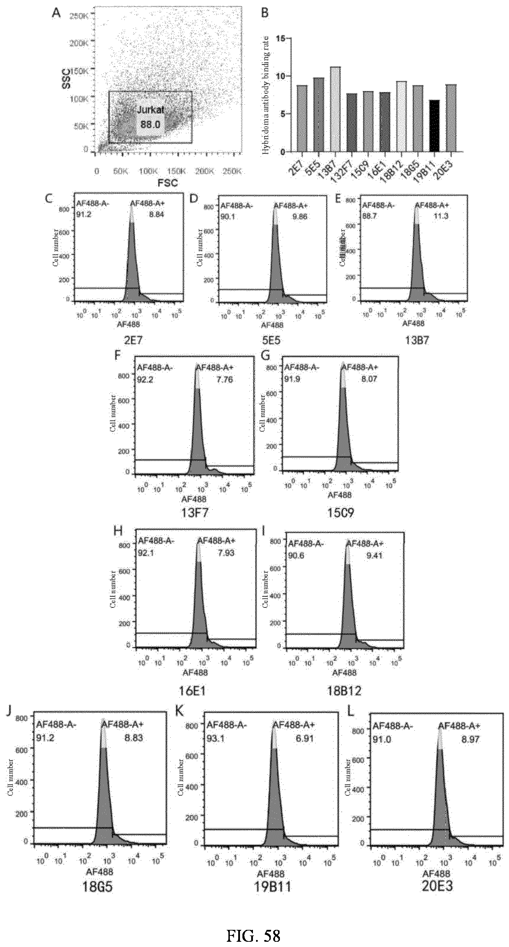

FIG. 5 shows the results of the co-immunoprecipitation experiment. As shown in FIG. 5, FIG. 5 (a) shows the content of ITPRIPL1 in the input protein as detected by Flag antibodies, while FIG. 5 (b) shows the content of CD3ε in the input protein. FIG. 5 (c) shows different mutants of ITPRIPL1 that were precipitated directly, while FIG. 5 (d) shows the CD3ε that were correspondingly precipitated indirectly (because of binding to the ITPRIPL1 mutants). The above results show that, in the presence of ITPRIPL1 extracellular domain (rather than the intracellular domain or the transmembrane domain), CD3ε and ITPRIPL1 remain the status of binding. Therefore, the ITPRIPL1 extracellular domain binds to CD3ε, which is consistent with the conclusion that the ligand ITPRIPL1 of CD3ε has an extracellular domain receptor-binding domain (RBD) of amino acids 25-103.

3. Immunofluorescence and colocalization analysis demonstrates that the ITPRIPL1 extracellular domain and the CD3 extracellular domain are in trans-binding.

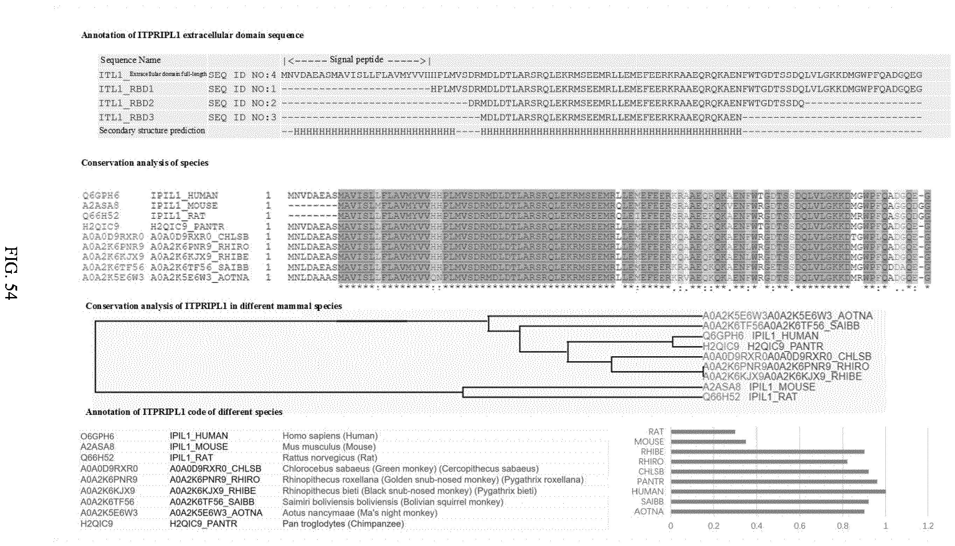

HA-tagged CD3ε was transfected into HCT116 cells; additionally, the pcDNA3.1 plasmids of Flag-tagged ITPRIPL1 (respectively the ITPRIPL1 extracellular domains of human, mouse, gorilla, grivet, golden snub-nosed monkey and Amazon squirrel monkey, and the ITPRIPL1 transmembrane domain of human)-green fluorescent protein were separately transfected into another batch of HCT116; finally, the two separately transfected cells were co-cultured and the localization patterns of the two proteins were determined by an immunofluorescence double staining process. Wherein, the sequences of the ITPRIPL1 extracellular domains of rat, mouse, gorilla, grivet, golden snub-nosed monkey, black snub-nosed monkey, and Amazon squirrel monkey were seen in FIG. 54.

The specific steps were as below: the pcDNA3.1 plasmids containing HA-tagged CD3ε were transfected into one batch of HCT116 cells (ATCC, VA, USA); additionally, the pcDNA3.1 plasmids containing Flag-tagged ITPRIPL1 (the extracellular domain-and-transmembrane domain)-green fluorescent protein were separately transfected into another batch of HCT116 cells (ATCC, VA, USA); 20 hours after transfection, the cells were individually digested with trypsin, mixed and resuspended well, and spread onto 8-well glass slides (Thermo Fisher, MA, USA) and continued to culture for 10 hours. The culture medium was discarded. After washing with PBS, the cells were fixed with 4% paraformaldehyde for 20 minutes, washed with PBS again and blocked with a membrane-permeable blocking buffer for 1 hour, and then HA-tagged specific rabbit antibodies (CST, MA, USA) which had been diluted with the membrane-permeable blocking buffer were added and incubated at 4°C overnight. After washing the 8-well glass slides with PBS overnight, Alexa Fluor 594 fluorescence specific anti-rabbit antibodies (Invitrogen, CA, USA) which had been diluted with PBS were added and incubated at room temperature for 20 minutes. After washing with PBS, the slides were mounted with DAPI, and observed under a fluorescence microscope after the mounting medium was dried. The localization patterns of the two proteins were determined by an immunofluorescence double staining process.

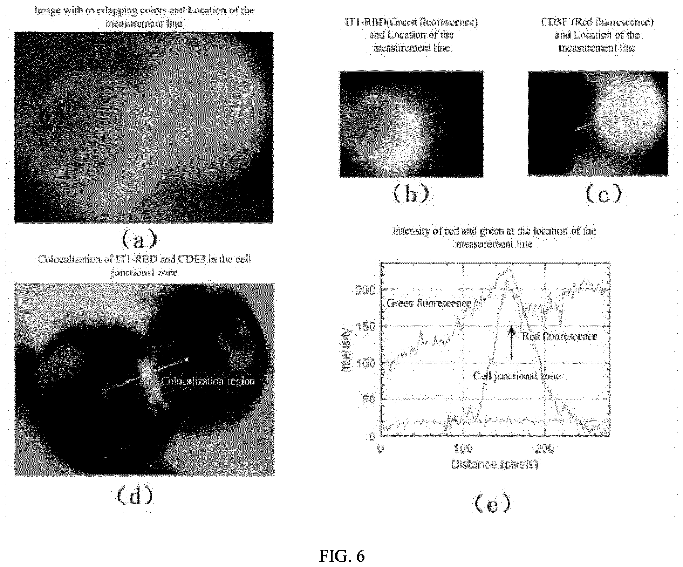

As shown in FIG. 6, FIG. 6 (a) shows the overlapping image of ITPRIPL1 and CD3ε staining, FIG. 6 (b) and (c) shows the staining results of ITPRIPL1 receptor-binding domain (ITPRIPL1-RBD) and CD3ε, respectively. FIG. 6 (d) shows the co-localized regions of the two proteins obtained by analyzing with the co-localization analysis module of the ImageJ software package. Wherein, the fluorescence intensity of ITPRIPL1 and CD3ε on the white straight path was shown in FIG. 6 (e). It can be seen that the signals of ITP-RBD (green fluorescence) and CD3ε (red fluorescence) are obviously concentrated and enhanced at the junction of the two cells, supporting the trans-binding of the extracellular domains of the two proteins on the cell surface. The experimental results also demonstrated that, the ITPRIPL1 extracellular domains of mouse, gorilla, grivet, golden snub-nosed monkey, and Amazon squirrel monkey bind to the CD3E extracellular domain of human.

### Example 3: Preparation of ITPRIPL1-RBD isolated recombinant protein with the function of regulating CD3ε and T cells

Based on the published sequence (NCBI reference sequence NP_001008949.1), human ITPRIPL1 extracellular domain (25-103 amino acids) recombinant protein was synthesized by Cusabio (Wuhan, China), expressed and purified by yeast, wherein the C terminus was fused to 6×-His and Myc tags, so as to produce ITPRIPL1-RBD recombinant proteins containing 6×-His and Myc-tags.

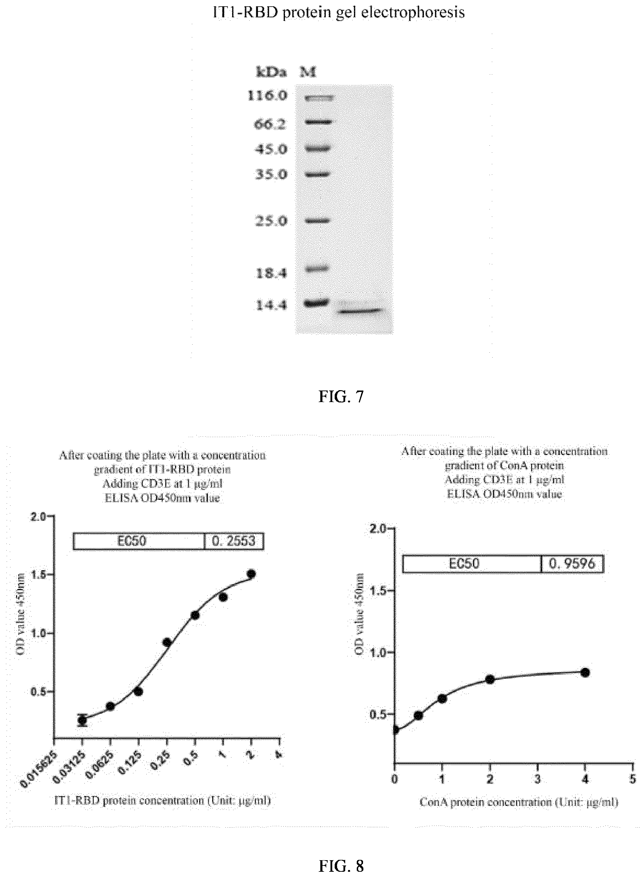

As shown in FIG. 7, it shows the Coomassie brilliant blue staining results of the ITPRIPL1-RBD recombinant protein after gel electrophoresis, indicating that its molecular weight and purity were as expected.

### Example 4: Concanavalin A (ConA) and the isolated ITPRIPL1-RBD protein have the ability to bind CD3ε, respectively

Enzyme-linked immunosorbent assay (ELISA) shows that there is direct concentration-dependent binding of Concanavalin A (ConA) and the isolated fragments from the ITPRIPL1 extracellular domain to the CD3ε extracellular domain, respectively. An ELISA special plate (costar, ME, USA) was used. Firstly, the plate was coated with 0.5/1/2/4 µg/ml of ConA or 0.03125/0.0625/0.125/0.25/0.5/1/2 µg/ml of ITPRIPL1-RBD recombinant protein each dissolved in 100 µl of ELISA coating buffer (Solarbio, Beijing, China), while for the negative control, the plate was coated with 100 µl of coating buffer free of proteins. The plate was coated at 4°C overnight. After washing with PBST, they were blocked with 100 µl of 5% BSA dissolved in PBS (VWR, PA, USA) in an incubator at 37°C for 90 minutes. After washing with PBST, they were incubated with 1 µg/ml of the protein fragments from the CD3ε extracellular domain (Sino Biological Inc., Beijing, China) in an incubator at 37°C for 60 minutes for binding, in which the CD3ε (Met1-Asp126) proteins contained hFc tags. After washing with PBST, they were incubated with PBS-diluted specific anti-human Fc segment antibodies (Abeam, MA, USA) in an incubator at 37°C for 30 minutes for binding. After washing with PBST, a color developing solution (Sangon, Shanghai, China) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 8 shows the ELISA experimental results. The experiment shows that there is direct concentration-dependent binding of Concanavalin A (ConA) and the isolated purified protein from the ITPRIPL1 extracellular domain to the purified protein from the CD3ε extracellular domain, respectively, and the binding intensity of IT1-RBD was greater than that of ConA. The above results show that Concanavalin A (ConA) and the isolated purified protein from the ITPRIPL1 extracellular domain (IT1-RBD) directly bind to the purified protein from the CD3ε extracellular domain, respectively, and the binding intensity of IT1-RBD is greater than that of ConA.

### Example 5: The isolated ITPRIPL1-RBD protein can be used to bind the CD3ε protein on the cell surface, and transmit inhibitory signals into T cells

### 1. Construction of HCT116-ITPRIPL1 and HCT116-CD3ε stably transfected cell lines

The constructed full-length ITPRIPL1-Flag plasmid and CD3ε-HA plasmid as well as empty pcDNA3.1 plasmid was respectively transfected into HCT116 cells (ATCC, VA, USA), cultured in an incubator for 24-48 hours, and screened by adding 1000 µg/ml of Geneticin (G418) (Gibco, CA, USA). 10-14 days later, after the empty pcDNA3.1 plasmid-transfected group of cells all died, HCT116-ITPRIPL1 and HCT116-CD3ε stably transfected cell lines were obtained.

2. Flow cytometry demonstrates that the purified protein fragments from the ITPRIPL1 extracellular domain bind to Jurkat cells with high expression of CD3.

The cultured Jurkat cells (ATCC, VA, USA) were counted, and the cell number was adjusted to 2×10⁵/ml. 200 µl of them was respectively added into six EP tubes of 1.5 ml (Axygen, CA, USA). Into four of the EP tubes were respectively added 0.1 µg, 0.2 µg, 0.4 µg, 0.8 µg ITPRIPL1-RBD-6_{X}-His recombinant protein (IT1-6×-His protein) so that the concentrations were 0.5 µg/ml, 1 µg/ml, 2 µg/ml, 4 µg/ml, respectively. All the EP tubes were placed in a cell incubator, standing for 30 minutes. After then, the EP tubes were taken out and centrifuged at 400 rcf for 5 minutes, and the supernatants were then discarded. The cells were resuspended and washed with 500 µl of cell staining buffer (Invitrogen, CA, USA), centrifuged at 400 ref for 5 minutes, the supernatants were then discarded again and the washing was repeated. The 6×-His-FITC antibodies (Abcam, MA, USA) were diluted with the cell staining buffer at 1:500. In addition to the negative control, 200 µl of antibody diluent was added into each EP tube and incubated at room temperature for 30 minutes at 40 rpm on a shaker. After then, the EP tubes were taken out and centrifuged at 400 ref for 5 minutes, and the supernatants were then discarded. The cells were resuspended and washed with 1000 µl of the cell staining buffer and centrifuged at 400 ref for 5 minutes, the supernatants were then discarded again and the washing was repeated. After the completion of washing, 300 µl of the cell staining buffer was added into each EP tube for resuspension, and transferred into flow tubes (Falcon, NY, USA) for on-board analysis (Miltenyi Biotec, Cologne, Germany).

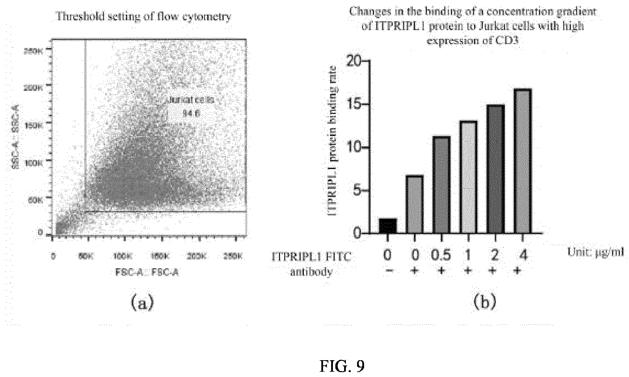

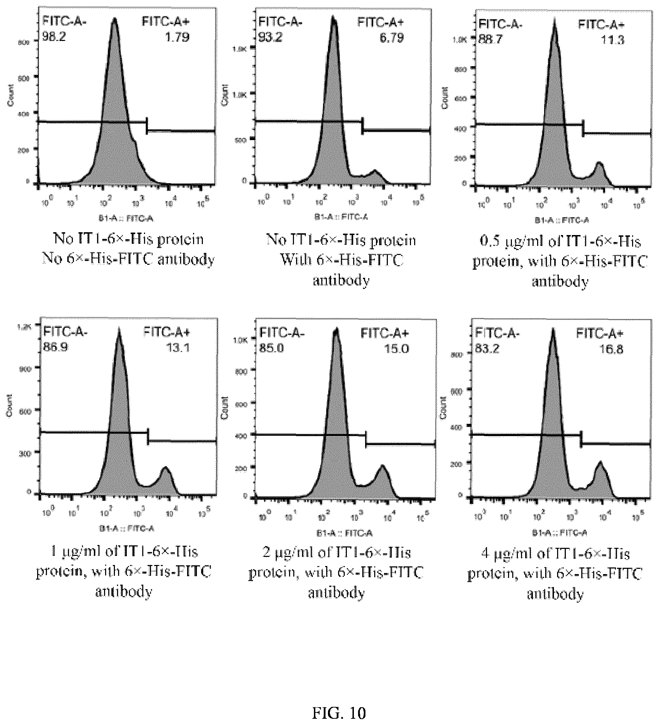

FIG. 9 (a) shows the threshold setting for not classified as dead cells. FIG. 9 (b) reflects the binding of Jurkat cells to different concentrations of the purified protein fragments from the ITPRIPL1 extracellular domain. FIG. 10 shows specific staining and protein binding under each condition in FIG. 9 (b). The above results show that the purified protein fragments from the ITPRIPL1 extracellular domain can bind to Jurkat cells with high expression of CD3.

3. Flow cytometry demonstrates that ITPRIPL1 protein binds to cells overexpressing CD3 with higher efficiency

The cultured HCT116 wild-type cells (ATCC, VA, USA) and HCT116-CD3ε stably transfected cell lines were digested and then counted, and the cell number was respectively adjusted to 2×10⁵/ml. 200 µl of them were respectively added into five and three EP tubes of 1.5 ml (Axygen, CA, USA). Into three EP tubes of HCT116 wild-type cells and three EP tubes of HCT116-CD3ε stably transfected cell lines were respectively added 0.2 µg, 0.4 µg, 0.8 µg ITPRIPL1-RBD-6×-His recombinant protein so that the concentrations were 1 µg/ml, 2 µg/ml, 4 µg/ml, respectively. All the EP tubes were placed in a cell incubator, standing for 30 minutes. After then, the EP tubes were taken out and centrifuged at 400 rcf for 5 minutes and the supernatants were then discarded. The cells were resuspended and washed with 500 µl of cell staining buffer (Invitrogen, CA, USA), centrifuged at 400 ref for 5 minutes, the supernatants were then discarded again and the washing was repeated. The 6×-His-FITC antibodies (Abeam, MA, USA) were diluted with the cell staining buffer at 1:500. In addition to the negative control, 200 µl of antibody diluent was added into each EP tube and incubated at room temperature for 30 minutes at 40 rpm on a shaker. After then, the EP tubes were taken out and centrifuged at 400 ref for 5 minutes, and the supernatants were then discarded. The cells were resuspended and washed with 1000 µl of the cell staining buffer and centrifuged at 400 ref for 5 minutes, the supernatants were then discarded again and the washing was repeated. After the completion of washing, 300 µl of the cell staining buffer was added into each EP tube for resuspension, and transferred into flow tubes (Falcon, NY, USA) for on-board analysis (Miltenyi Biotec, Cologne, Germany).

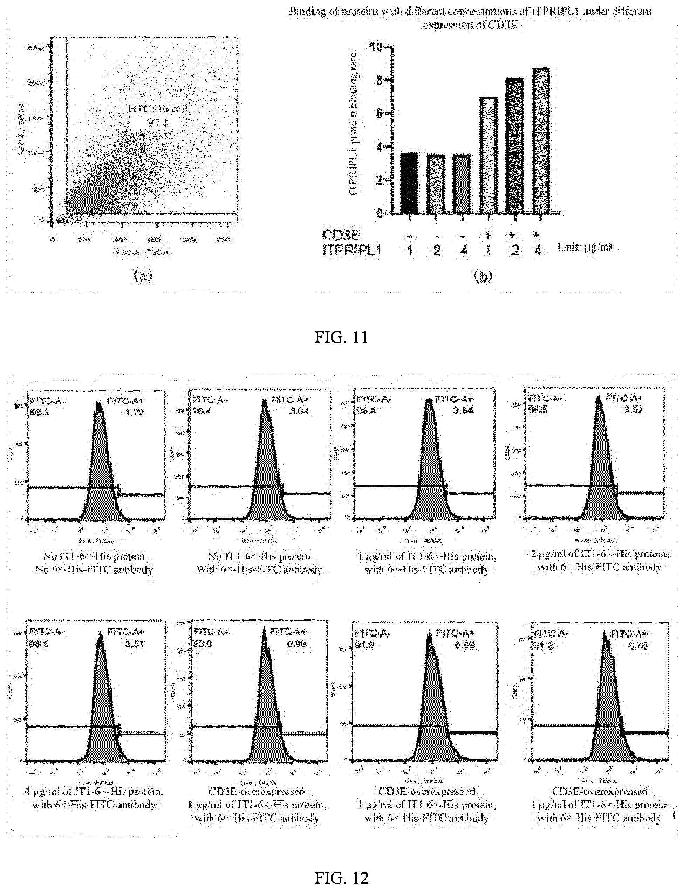

FIG. 11 (a) shows the threshold setting for not classified as dead cells. FIG. 11 (b) reflects the binding of cells with different expression of CD3ε to different concentrations of ITPRIPL1 recombinant proteins. FIG. 12 shows specific staining and protein binding under each condition in FIG. 11 (b). The above results show that the purified protein fragments from the ITPRIPL1 extracellular domain can bind to HCT116 cells overexpressing CD3, but do not bind to HCT116 cells not expressing CD3.

4. Flow cytometry demonstrates that CD3 binds to cells overexpressing ITPRIPL1 with higher efficiency.

The cultured HCT116 wild-type cells (ATCC, VA, USA) and HCT116-ITPRIPL1 stably transfected cell lines were digested and then counted, and the cell number was respectively adjusted to 2×10⁵/ml. 200 µl of them were respectively added into three and one EP tubes of 1.5 ml (Axygen, CA, USA). Into one EP tube of HCT116 wild-type cells and the EP tube of HCT116-ITPRIPL1 stably transfected cell lines were respectively added 0.4 µg CD3E-human Fc protein (Sino Biological Inc., Beijing, China) so that the concentration was 2 µg/ml. All the EP tubes were placed in a cell incubator, standing for 30 minutes. After then, the EP tubes were taken out and centrifuged at 400 ref for 5 minutes and the supernatants were then discarded. The cells were resuspended and washed with 500 µl of cell staining buffer (Invitrogen, CA, USA), centrifuged at 400 ref for 5 minutes, the supernatants were then discarded again and the washing was repeated. The anti-human IgG Alexa Fluor 647 antibodies (Invitrogen, CA, USA) were diluted with the cell staining buffer at 1:1000. In addition to the negative control, 200 µl of antibody diluent was added into each EP tube and incubated at room temperature for 30 minutes at 40 rpm on a shaker. After then, the EP tubes were taken out and centrifuged at 400 ref for 5 minutes, and the supernatants were then discarded. The cells were resuspended and washed with 1000 µl of the cell staining buffer and centrifuged at 400 ref for 5 minutes, the supernatants were then discarded again and the washing was repeated. After the completion of washing, 300 µl of the cell staining buffer was added into each EP tube for resuspension, and transferred into flow tubes (Falcon, NY, USA) for on-board analysis (Miltenyi Biotec, Cologne, Germany).

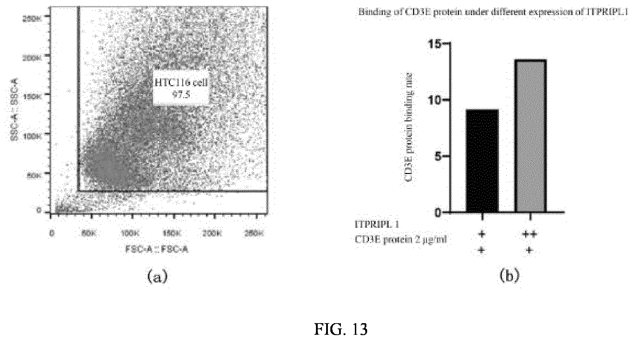

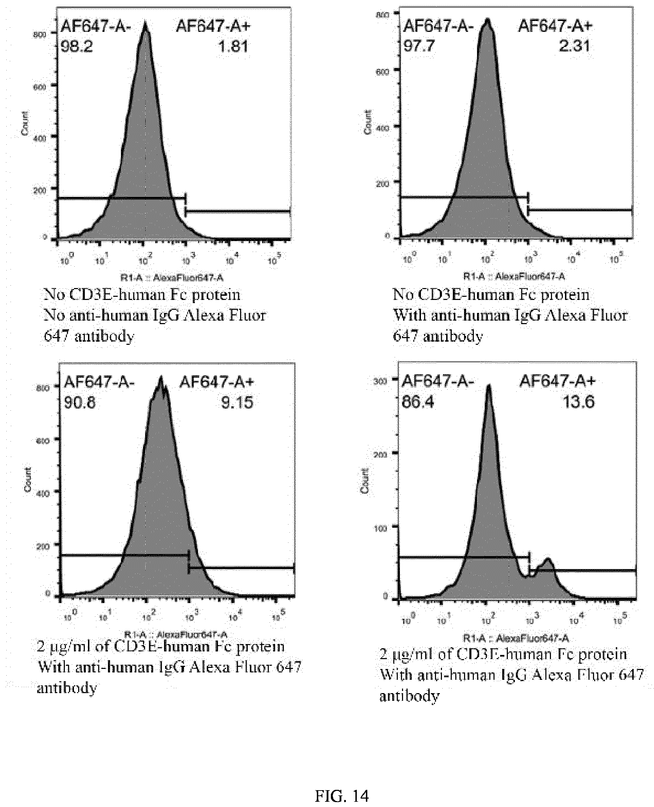

FIG. 13 (a) shows the threshold setting for not classified as dead cells. FIG. 13 (b) reflects the binding of cells with different expression of ITPRIPL1 to CD3ε proteins. FIG. 14 shows specific staining and protein binding under each condition in FIG. 13 (b), respectively. The above results show that CD3ε can bind to HCT116 expressing ITPRIPL1, and can bind to HCT116 cells overexpressing ITPRIPL1 more strongly.

5. Luciferin reporter assay demonstrates that the purified protein fragments from the ITPRIPL1 extracellular domain inhibit the ConA-activated NFKB proliferation signaling in Jurkat-dual cells.

The cultured Jurkat-dual cells (Invivogen, CA, USA) were counted, centrifuged at 1000 rpm for 5 minutes, and then resuspended with antibiotic-free IMDM medium (Gibco, CA, USA) to adjust the cell number to 2×10⁶/ml. 200 µl of the cells were added into each well of a transparent 96-well plate (Thermo Fisher, MA, USA). Into each well was respectively added 0.2 µg, 0.4 µg, 0.8 µg ITPRIPL1-6×-His recombinant protein so that the concentrations were 1 µg/ml, 2 µg/ml, 4 µg/ml, respectively. After reaction for two hours in a cell incubator, 10 µg/ml of Concanavalin A (ConA) (Aladdin, Shanghai, China) was added into each well and reacted in the cell incubator for 18-24 hours. After the completion of reaction, a non-transparent 96-well plate (costar, ME, USA) was taken, into each well of which were added 50 µl of Quanti-luc reagent (Invivogen, CA, USA) and 20 µl of the reaction mixture and mixed well, and then the signals were detected immediately with a multimode microplate reader (Thermo Fisher, MA, USA).

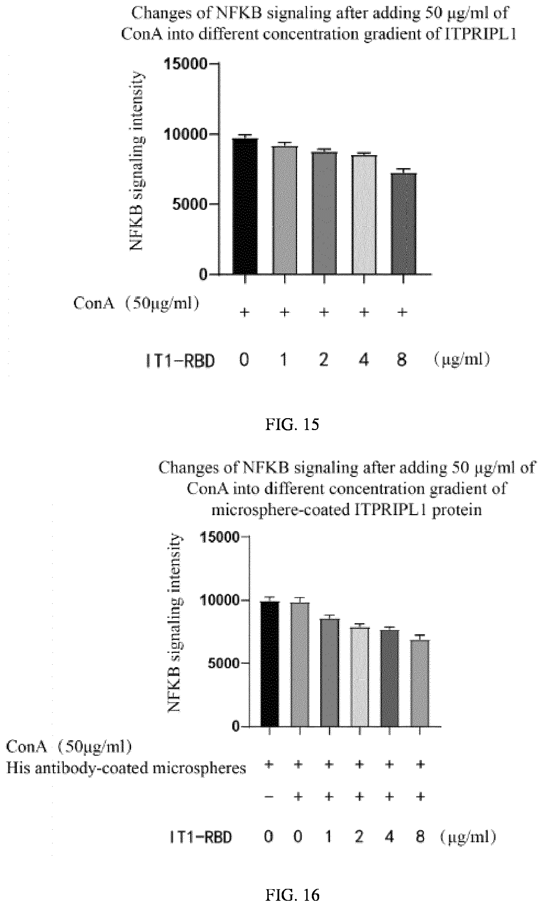

As shown in FIG. 15, the NFKB signaling changed as a function of the concentration of ITPRIPL1 protein under the activation of 10 µg/ml of ConA. The above results show that the ITPRIPL1-RBD purified recombinant protein can inhibit the ConA-activated NFKB proliferation signaling in Jurkat-dual cells in a concentration-dependent manner.

6. Luciferin reporter assay demonstrates that the purified protein fragments from the ITPRIPL1 extracellular domain immobilized on the surface of microspheres inhibit the ConA-activated NFKB proliferation signaling in Jurkat-dual cells.

Protein G magnetic microspheres (Thermo Fisher, MA, USA) and His antibodies (Abeam, MA, USA) were placed in four EP tubes (Axygen, CA, USA), and revolved in a DNA mixer (Scientz, Ningbo, China) at the lowest speed at room temperature for 1 hour. Into three of the EP tubes were added 0.2 µg, 0.4 µg, 0.8 µg ITPRIPL1-6×-His recombinant protein, respectively, and revolved at the lowest speed at room temperature for another 1 hour. The cultured Jurkat-dual cells (Invivogen, CA, USA) were counted, centrifuged at 1000 rpm for 5 minutes and then resuspended with antibiotic-free IMDM medium (Gibco, CA, USA) to adjust the cell number to 2×10⁶/ml. 200 µl of the cells were added into each well of a transparent 96-well plate (Thermo Fisher, MA, USA). Into each well was added the content of each EP tube respectively so that the concentrations of the coated proteins were 1 µg/ml, 2 µg/ml, 4 µg/ml, respectively. After reaction for two hours in a cell incubator, 50 µg/ml Concanavalin A (ConA) (Aladdin, Shanghai, China) was added into each well and reacted in the cell incubator for 18-24 hours. After the completion of reaction, a non-transparent 96-well plate (costar, ME, USA) was taken, into each well of which were added 50 µl of Quanti-luc reagent (Invivogen, CA, USA) and 20 µl of the reaction mixture and mixed well, and then the signals were detected immediately with a multimode microplate reader (Thermo Fisher, MA, USA).

As shown in FIG. 16, the NFKB signaling changed as a function of the concentration of microsphere-coated ITPRIPL1 proteins under the activation of 50 µg/ml of ConA. The above results show that the ITPRIPL1-RBD purified recombinant proteins immobilized on the surface of microspheres can inhibit the ConA-activated NFKB proliferation signaling in Jurkat-dual cells in a concentration-dependent manner.

### 7. Detection of the expression level of ITPRIPL1 in different types of tumor cells

Since the ITPRIPL1 extracellular domain has the function of inhibiting immune cells (for example, T cells), tumor cells may express ITPRIPL1 to evade surveillance and killing by the immune system. If a variety of tumor cells abnormally express ITPRIPL1, it suggests that ITPRIPL1 may contribute to tumorigenesis and its progression, including immune evasion. The present invention reveals that ITPRIPL1 is abnormally expressed in cell lines from different types of tumors. The specific experimental procedures were: the cultured cell lines were counted, from which 2×10⁶ cells were taken into a centrifuged tube of 15 ml and centrifuged at 800 rpm for 4 minutes, and the supernatants were then discarded. The cells were resuspended and washed with PBS, centrifuged at 800 rpm for 4 minutes, and resuspended and washed with PBS again. After the completion of centrifugation, the supernatants were discarded. The RIPA lysate and a triple of protease-phosphatase-PMSF were formulated at a ratio of 1:100. 120 µl of the hybrid lysate was added into each tube of cells, and transferred into EP tubes. Each EP tube was frozen and thawed on liquid nitrogen-ice for three cycles, and centrifuged at 12000 rpm at 4°C for 15 minutes after the last thawing. After the completion of centrifugation, the supernatants were taken and formulated into a variety of cell samples at a ratio of 4:1 of the supernatant to 5×loading buffer, and denatured in a metal bath at 100°C for 10 minutes. After the completion of denaturation, the endogenous expression of ITPRIPL1 in the variety of tumor cell lines was determined through gel electrophoresis and Western Blot assay, with GAPDH as the internal reference.

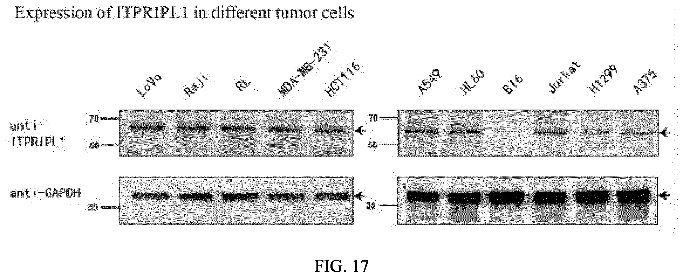

FIG. 17 shows the expression of ITPRIPL1 after the alignment of GAPDH internal reference in Western blotting. As shown in FIG. 17, the expression level of ITPRIPL1 protein in tumor cell lines was detected through Western Blot experiment, wherein there was high level of expression in cells of LoVo colorectal cancer, Raji lymphoma, RL lymphoma, MDA-MB-231 breast cancer, HCT116 colorectal cancer, A549 lung cancer, HL60, Jurkat lymphoma, H1299 lung cancer, and A375 melanoma. Although the inventors did not detect the expression of ITPRIPL1 in all types of tumor cells, it can be judged from the proportion of ITPRIPL1 expressed in tumor cells that have been detected (10/11, more than 90%) that, ITPRIPL1 may be expressed in multiple types and quite widely in malignant tumors.

Correspondingly, Protein Atlas database indicated, based on the results extracted by analyzing the high-throughput mRNA expression profile, that ITPRIPL1 may have significantly elevated expression in a variety of tumors. There have not been any reports of ITPRIPL1 function in the past. Therefore, before the disclosure of the contents of the present invention, those skilled in the art could not have predicted any functions of ITPRIPL1 in tumorigenesis and its progression as well as immune evasion. The present invention has disclosed the abnormal elevated expression level of ITPRIPL1 tumor cell proteins, the ITPRIPL1 extracellular domain binding to CD3ε and causing inhibition of T cell, as well as other experimental data, which firstly disclosed the important value of ITPRIPL1 as the target in tumor immunotherapy.

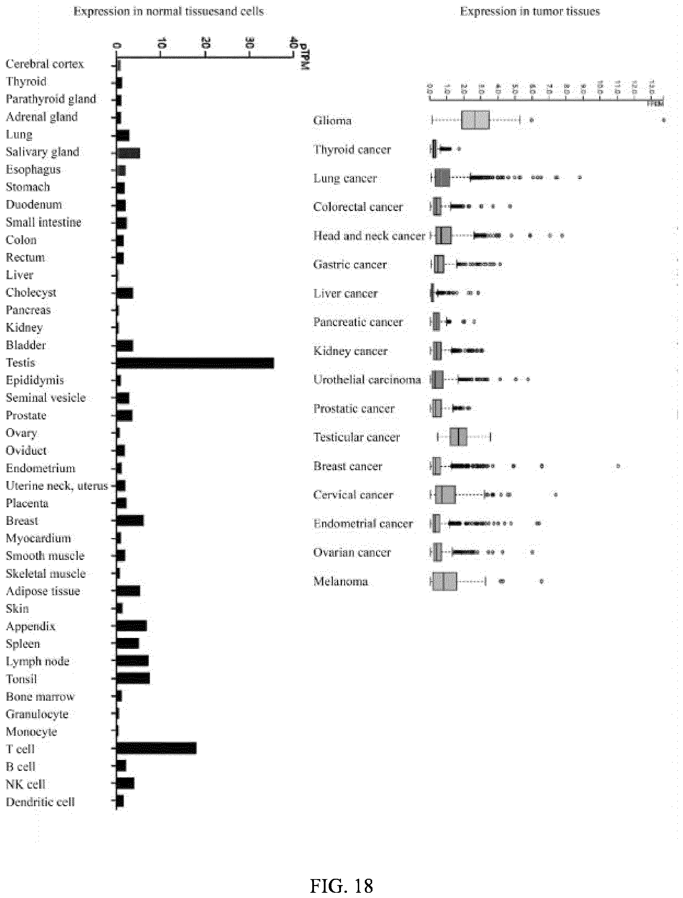

As shown in FIG. 18, according to the mRNA expression profile data collected from the Protein Atlas website, ITPRIPL1 man be mainly in testis, T cells in normal tissues or cells. It should be noted that the expression of mRNA does not directly represent the expression level of protein, and in general, the reanalysis of expression profiling data also requires validation by low-throughput biological experiments (e.g., protein gel electrophoresis-Western Blot assay) before reliable conclusions can be drawn.

8. OCTET molecular interaction experiments demonstrate that the purified protein fragments from the ITPRIPL1 extracellular domain can directly bind to CD3ε protein.

The OCTET instrument was initiated. 100 µg of CD3E-Fc protein (Sino Biological Inc., Beijing, China) was adsorbed to saturation by Fc probes, and then 50 µg of recombinant protein from the ITPRIPL1 extracellular domain was correspondingly bound at concentrations of 800nM/1600nM/3200nM, thereby plotting the binding curves and calculating the dissociation constants.

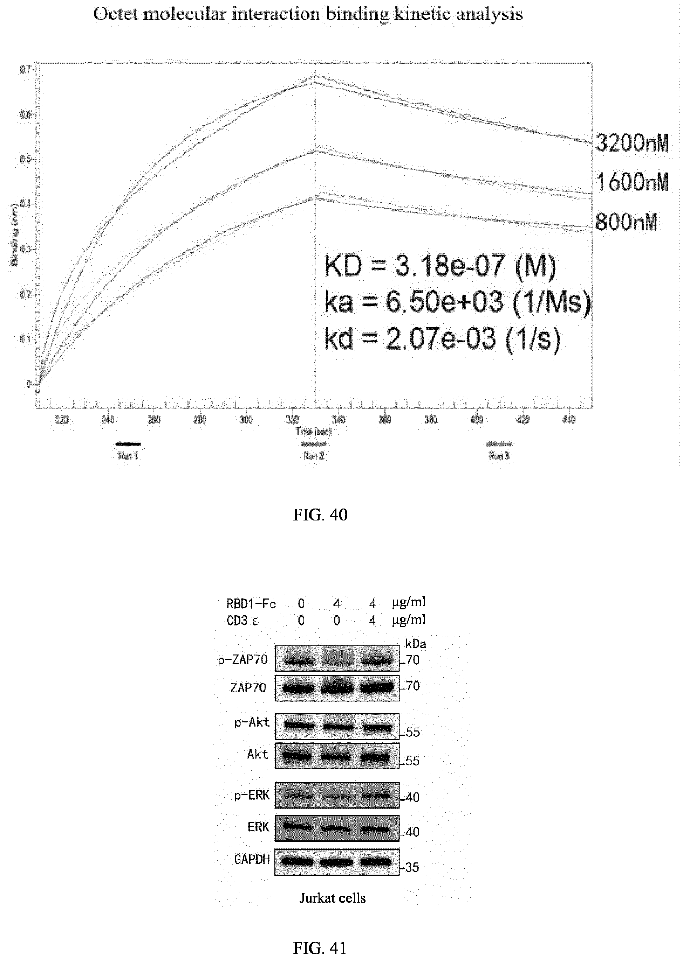

As shown in FIG. 40, the whole adsorption-dissociation process was shown in this diagram and the relevant constants were calculated. The above results show that the purified protein fragments from the ITPRIPL1 extracellular domain can directly bind to CD3E protein.

### Example 6: Isolated ITPRIPL1-RBD as an immunogen to prepare antibodies for inhibiting the in vivo tumor growth

### 1. Preparation of mouse polyclonal antibodies and polyclonal antibodies with ITPRIPL1-RBD as an immunogen

By using the human ITPRIPL1-RBD recombinant protein as the immunogen, and after verifying the purity and biological activity of the samples in the above examples, C57BL/6 mice were subjected to multiple immunizations to enhance the effect: (1) primary immunization, with the antigen at 50 µg per mouse, subcutaneous multi-point injection with Freund's complete adjuvant, at an interval of 3 weeks; (2) secondary immunization, with the dose and route the same as above but using Freund's incomplete adjuvant, at an interval of 3 weeks; (3) third immunization, with the dose the same as above, no addition of adjuvant, intraperitoneal injection at an interval of 3 weeks; (4) booster immunization, at a dose of 50 µg, intraperitoneal injection. Three days after the last injection, blood was taken and tested for its potency. After the immunization effect was detected to meet the requirements, the blood was taken and the polyclonal antibodies were isolated in a cumulative manner for multiple times, and the polyclonal antibodies were purified, for which the specific experimental procedures included: (1) Preparation of a protein G sepharose CL-4B affinity column. 10 mL of protein G sepharose CL-4B packings were prepared, and equal volumes of the packings and TBS buffer solution were mixed in a vacuum flask and stirred. Evacuation was performed for 15 minutes to remove air bubbles in the packings. The protein G sepharose CL-4B packings were slowly added into a glass column while controlling the filling rate at 1 mL/min-2 mL/min with a pump. To avoid column dryness, the column was equilibrated with a pre-cooled TBS buffer solution that was 10 times the bed volume. (2) Preparation of polyclonal antibodies. The polyclonal antibodies were slowly thawed in ice water or in a 4°C refrigerator to avoid the aggregation of protein. The aggregation that occurs during the protein thawing can be dissolved by preheating at 37°C. Solid sodium azide was added to a concentration of 0.05%, and centrifuged at 15,000 × g for 5 min at 4°C. The clarified polyclonal antibodies were removed out and filtered through a filter to remove excess lipid. (3) Affinity chromatography. The antibodies were diluted with TBS buffer solution at a ratio of 1:5, and filtered through a filter. The polyclonal antibodies were loaded onto the column at a rate of 0.5 mL/min. To ensure the binding of the polyclonal antibodies to the packings, the column should be loaded twice in succession and the loading effluent should be retained. After washing the column with TBS buffer solution until Aλ, 280 nm was < 0.008, an elution buffer solution at Ph 2.7 was added to elute at a rate of 0.5 mL/min until all protein flew down. The eluent was collected into EP tubes of 1.5 mlwhich had been added with 100 µL of neutralizing buffer solution, mixed well and detected with pH test paper for the pH of the eluent. If the pH was lower than 7, it could be adjusted to about pH 7.4 with a neutralizing buffer so as to avoid the denaturation of the antibodies. Into the column was added 10 mL of elution buffer solution at pH 1.9 to collect the eluent according to the above method until Aλ, 280 nm was < 0.008. The protein content in each tube was determined by a spectrophotometer.

2. Enzyme-linked immunosorbent assay (ELISA) demonstrates that polyclonal antibodies with ITPRIPL1-RBD as the immunogen can bind to cells expressing ITPRIPL1

An ELISA special plate (costar, ME, USA) was used. Firstly, B16 cells not expressing ITPRIPL1 (ATCC, VA, USA), LoVo cells moderately expressing ITPRIPL1 (ATCC, VA, USA) and HCT116-ITPRIPL1 stably transfected cell lines were digested with trypsin and counted, and the cell number was adjusted to 2×10⁶/ml. Each well was plated with 100 µl of cells and coated at 4°C overnight. After washing with PBST, they were blocked with 100 µl of 5% skim milk powder dissolved in PBS (Sangon, Shanghai, China) in an incubator at 37°C for 90 minutes. After washing with PBST, the polyclonal antibodies at a total IgG concentration of 10 mg/ml were then diluted at a gradient of 1:1000/1:500/1:250/1:125, incubated with plated cells in an incubator at 37°C for 60 minutes for binding. After washing with PBST, the PBS-diluted specific anti-mouse Fc segment antibodies (Consun, Shanghai, China) were incubated in an incubator at 37°C for 30 minutes for binding. After washing with PBST, a color developing solution (Sangon, Shanghai, China) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

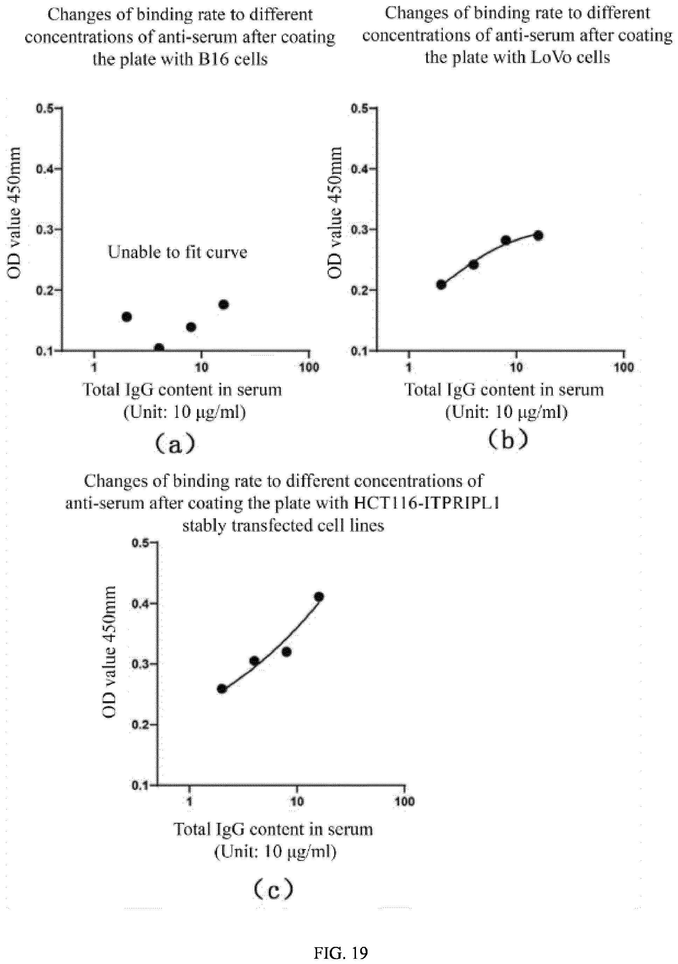

FIG. 19 (a) shows the changes in the binding rate of B16 cells to different concentrations of polyclonal antibodies. FIG. 19 (b) shows the changes in the binding rate of LoVo cells to different concentrations of polyclonal antibodies. FIG. 19 (c) shows the changes in the binding rate of HCT116-ITPRIPL1 stably transfected cell lines to different concentrations of polyclonal antibodies. This experiment demonstrated that, with the increase of the IgG concentration in the polyclonal antibodies, there was no obvious change trend in the binding rate of B16 cells to polyclonal antibodies, the binding rate of LoVo cells to polyclonal antibodies showed some degree of concentration-dependent increase, and the binding rate of HCT116-ITPRIPL1 stably transfected cell lines to polyclonal antibodies show obvious concentration-dependent increase. The above results show that the polyclonal antibodies can bind to cells expressing ITPRIPL1.

3. Enzyme-linked immunosorbent assay (ELISA) demonstrates that polyclonal antibodies can block the binding of ITPRIPL1 to CD3ε.

An ELISA special plate (costar, ME, USA) was used. Firstly, the plate was coated with 0.1 µg of CD3ε protein fragments (Sino Biological Inc., Beijing, China) dissolved in 100 µl of ELISA coating buffer (Solarbio, Beijing, China), in which the CD3ε (Met 1-Asp117) proteins contained hFc tags, while for the negative control, the plate was coated with 100 µl of coating buffer free of proteins. The plate was coated at 4°C overnight. After washing with PBST, they were blocked with 100 µl of 5% skim milk powder dissolved in PBS (Sangon, Shanghai, China) in an incubator at 37°C for 90 minutes. After washing with PBST, 2 µg/ml of ITPRIPL1-RBD-6×-His protein was mixed with polyclonal antibodies at a total IgG concentration of 10 mg/ml at ratios of 1:1000/1:500/1:250/1:125, respectively, and then co-incubated in an incubator at 37°C for 60 minutes for binding. After washing with PBST, they were incubated with PBS-diluted specific anti-6×-His-tagged horseradish peroxidase antibodies (Abeam, MA, USA) in an incubator at 37°C for 30 minutes for binding. After washing with PBST, a color developing solution (Sangon, Shanghai, China) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

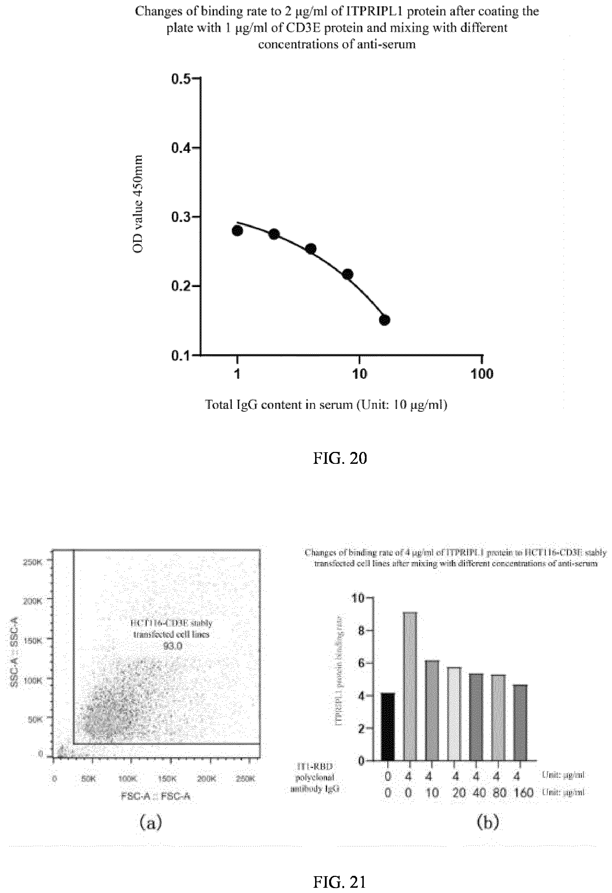

As shown in FIG. 20, this experiment demonstrated that, with the increase of the IgG concentration in the polyclonal antibodies, the binding rate of CD3ε to ITPRIPL1-RBD protein gradually decreased. The above results show that polyclonal antibodies can block the binding of ITPRIPL1 to CD3ε.

4. Flow cytometry demonstrates that polyclonal antibodies can block the binding of ITPRIPL1 to cells overexpressing CD3ε.

HCT116-CD3ε stably transfected cell lines were digested and then counted, and the cell number was respectively adjusted to 2×10⁵/ml. 200 µl of them were respectively added into eight EP tubes of 1.5 ml (Axygen, CA, USA). Into each EP tube was added 0.8 µg IT1-RBD protein so that the concentrations were 4 µg/ml respectively. Into five EP tubes of them were respectively added polyclonal antibodies at a total IgG concentration of 10 mg/ml which had been diluted at 1:1000/1:500/1:250/1:125/1:67.5, and left in a cell incubator for 30 minutes. After then, the EP tubes were taken out and centrifuged at 400 ref for 5 minutes and the supernatants were then discarded. The cells were resuspended and washed with 500 µl of cell staining buffer (Invitrogen, CA, USA), centrifuged at 400 rcf for 5 minutes, the supernatants were then discarded again and the washing was repeated. The 6×-His FITC antibodies (Abeam, MA, USA) were diluted with the cell staining buffer at 1:500. In addition to the negative control, 200 µl of antibody diluent was added into each EP tube and incubated at room temperature for 30 minutes at 40 rpm on a shaker. The EP tubes were taken out and centrifuged at 400 rcf for 5 minutes, and the supernatants were then discarded. The cells were resuspended and washed with 1000 µl of the cell staining buffer and centrifuged at 400 rcf for 5 minutes, the supernatants were then discarded again and the washing was repeated. After the completion of washing, 300 µl of the cell staining buffer was added into each EP tube for resuspension, and transferred into flow tubes (Falcon, NY, USA) for on-board analysis (Miltenyi Biotec, Cologne, Germany).

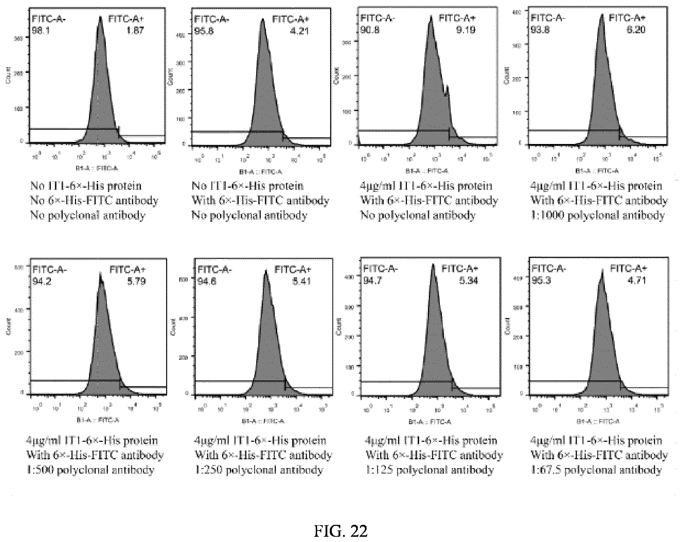

FIG. 21 (a) shows the threshold setting for not classified as dead cells. FIG. 21 (b) reflects the binding profile of ITPRIPL1 to CD3ε when the concentration of polyclonal antibodies changes. FIG. 22 shows specific staining and protein binding under each condition in FIG. 21 (b). The above results show that polyclonal antibodies can block the binding of ITPRIPL1 to cells overexpressing CD3ε.

5. Preparing monoclonal antibodies on the basis of polyclonal antibodies, and further verifying the ability of the antibodies to inhibit the tumor growth.

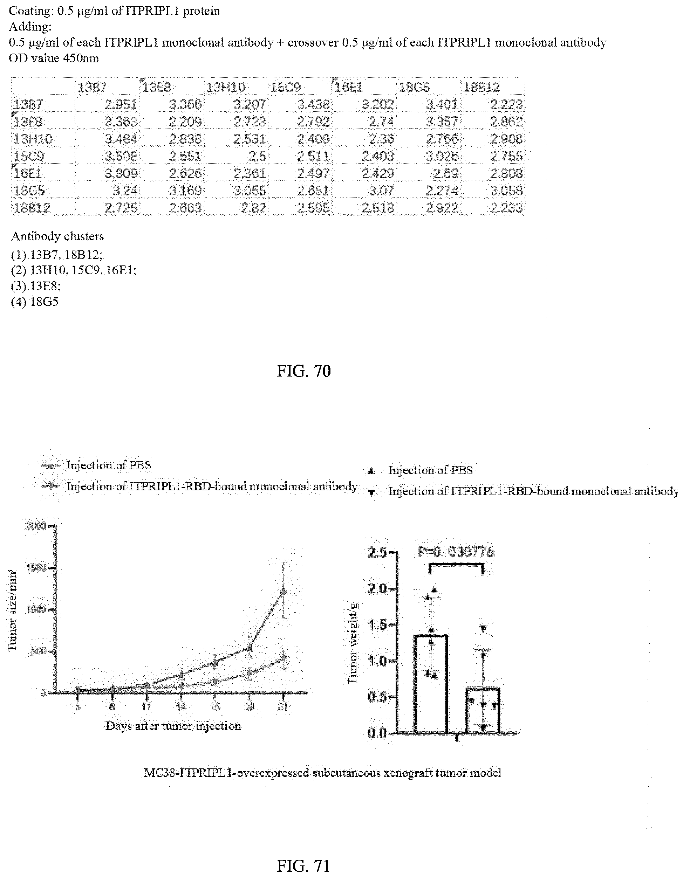

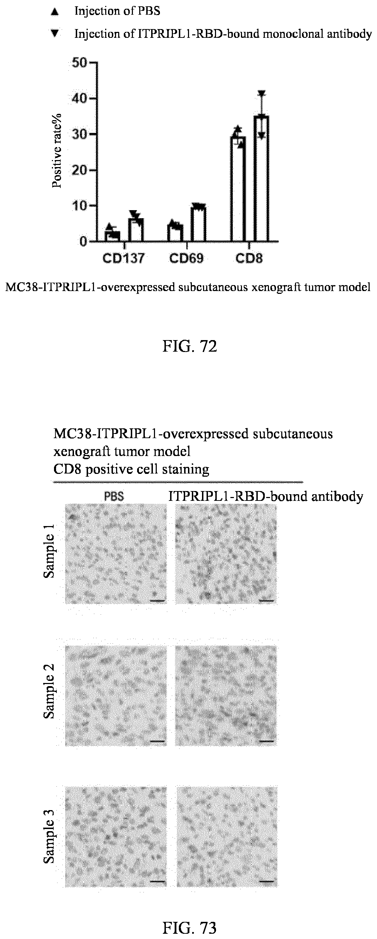

Since the expression of ITRPRIPL1 is up-regulated in a variety of tumors, the antibody that specifically binds to ITPRIPL1 can recognize tumor cells in the body, and kill tumor cells by exerting ADCC, ADCP and CDC effects through the Fc segment of the constant region of the antibody. ADCC, i.e., Antibody-Dependent Cell-mediated Cytotoxicity, refers to that the Fab segment of an antibody binds to the antigenic epitopes of virus-infected cells or tumor cells, and its Fc segment binds to the FcR on the surface of killer cells (NK cells, macrophages, neutrophils, etc.), which mediate the direct killing of target cells by killer cells, and this is an important mechanism for the action of anti-tumor therapeutic antibody drugs. ADCP, i.e., Antibody-Dependent Cellular Phagocytosis, is also an important mechanism for recognizing and mediating the effect of therapeutic antibodies on tumor cells. CDC, i.e., Complement Dependent Cytotoxicity, refers to complement-involved cytotoxicity, which means that through the binding of specific antibodies to the corresponding antigen on the surface of the cell membrane, a complex is formed to activate the classical pathway of complement, and the resulting membrane-attacking complex has a lytic effect on target cells. This example will confirm the inhibition of in vivo tumor growth by an antibody that specifically recognizes the ITPRIPL1 extracellular domain. The specific implementation steps are as below:
a) The hybridoma cells obtained from fusion were diluted into a 96-well plate (the estimated density was 0.5 cells per well), and further cultured to the formation of clones. The culture supernatant derived from monoclonal hybridoma was taken for ELISA test to determine the degree of antigen binding to 0.2 µg/ml of ITPRIPL1 (RBD1 protein)-coated plate, which was ranked according to the OD450 adsorption value. And the monoclonal hybridoma cells corresponding to the most strongly bound antibody were taken, expanded and cultured, and the ascites antibodies (hereafter referred to as: RBD1-conjugated antibodies) were prepared for in vivo functional studies in animals.
b) The constructed full-length ITPRIPL1-Flag plasmids and empty pcDNA3.1 plasmids were respectively transfected into MC38 cells (Kerafast, MA, USA), and cultured in an incubator for 24-48 hours, into which was then added 200 µg/ml of Geneticin (G418) (Gibco, CA, USA) for screening. 10-14 days later, after the empty pcDNA3.1 plasmid-transfected group of cells all died, MC38-ITPRIPL1 stably transfected cell lines were obtained. 6-8-week-old humanized CD3ε mice (Model Organisms Center, Shanghai, China) were selected and randomly grouped according to the weight, with 6 mice in each group. MC38 wild-type and MC38-ITPRIPL1 stably transfected cell lines were counted, and resuspended with PBS to a cell density of 1.5×10⁷/ml. The mice were shaved, and 1.5 × 10⁶ ITPRIPL1-overexpressed MC38 cells were subcutaneously inoculated into the right armpit to construct in vivo models of humanized CD3ε mouse MC38 ITPRIPL1-overexpressed subcutaneous xenograft tumor. From the fifth day of tumor inoculation, each group of MC38 ITPRIPL1-overexpressed transplanted tumor-bearing mice were injected intraperitoneally with 100 µg of RBD1-conjugated antibody or an equivalent amount of PBS every three days, and then injected every three days for a total of four treatments. The tumor size was measured with a vernier caliper. The long diameter and short diameter of the tumor were measured each time, and the tumor size was calculated following the formula of 1/2*A*a*a. Measurement was conducted every three days, and the tumor sizes were recorded. On day 23 after inoculation, all mice were sacrificed. After stripping the tumors, the tumor weight was weighed and statistically analyzed. As shown in FIG. 71, both the tumor size and the tumor weight show that the tumor growth is significantly inhibited after treating with RBD1-conjugated antibodies, confirming that the ITPRIPL1 monoclonal antibodies have in vivo functions.
c) Flow cytometry demonstrated that ITPRIPL1 monoclonal antibodies can significantly increase the activity of T cells in ITPRIPL1-overexpressed tumors. At least 1 ml of peripheral blood was obtained from mice by means of cardiac blood sampling. Mouse PBMC cells were obtained by using a mouse peripheral blood PBMC separation kit (Solarbio, Beijing, China) according to the corresponding instruction of the kit. The mouse PBMCs were placed into EP tubes (Axygen, CA, USA), centrifuged at 400 ref for 5 minutes and the supernatants were then discarded. The cells were resuspended and washed with 500 µl of cell staining buffer (Invitrogen, CA, USA), and centrifuged and washed again. Mouse CD8-APC antibodies (Biolegend, CA, USA), mouse CD69-APC antibodies (Biolegend, CA, USA), and mouse CD137-APC antibodies (Biolegend, CA, USA) were diluted with the cell staining buffer at 1:20. 200 µl of the mixture solution was added into each EP tube for resuspension, and incubated at room temperature while shaking slowly for 30 minutes. After centrifugation at 400 rcf for 5 minutes, the supernatant was discarded, and the cells were resuspended and washed with 500 µl of the cell staining buffer; and centrifuged and washed again. 200 µl of the cell staining buffer was respectively added, and transferred into flow tubes (Falcon, NY, USA) for loading (Miltenyi Biotec, Cologne, Germany). As shown in FIG. 72, according to the fluorescence positive rate of cells, RBD1-bound antibody can significantly increase the expression of CD8, CD25, CD139. The experimental results show that RBD1-bound antibody can relieve the inhibition of ITPRIPL1 on the activity of T cells in mice.
d) Immunohistochemical staining demonstrated that RBD1-bound antibody can significantly increase the infiltration of immune cells in ITPRIPL1-overexpressing tumors. MC38-ITPRIPL1-overexpressed tumor tissues were stripped for sectioning and paraffin embedding treatment (Biossci, Wuhan, China). The resulting paraffin sections were subjected to dewaxing, hydration, antigen retrieval or other treatment, and then incubated with mouse CD8 antibodies (CST, MA, USA) in a wet box overnight. The next day, after rewarming, secondary antibodies were incubated according to the reagent instructions, stained with DAB (Solarbio, Beijing, China) and hematoxylin (Solarbio, Beijing, China), and then air-dried in a reverse alcohol concentration gradient and mounted. After the mounting was completed, the slide was observed under a fluorescence microscope and photographed under natural light. As shown in FIG. 73, the positive rate of CD8 in tumor tissues can be significantly increased after using the RBD1-bound antibody. The experimental results show that RBD1-bound antibody can increase the infiltration of immune cells in MC38 ITPRIPL1-overexpressing tumor tissues.

The above results show that, antibodies that specifically recognize the ITPRIPL1 extracellular domain, i.e., RBD1, can significantly inhibit the tumor cell growth in vivo. Since ITPRIPL1 is expressed in primary cancer, lymph metastatic carcinoma, distant metastatic carcinoma simultaneously, this allows the antibody drug to act on tumors at different sites and stages of development, exert a more sustained and widespread anti-tumor effect through ADCC, ADCP and CDC effects, and activate the local immune response in the tumor. At the same time, since ITPRIPL1 is expressed very low in the control normal tissues of the above tumors, the toxic side effects that may be produced from such an antibody against ITPRIPL1 on normal tissues and cells are mor limited. The above properties highlight the outstanding advantages and application prospects of the ITPRIPL1 specific antibody as a new anti-cancer therapy.

### Example 7: Regulation of the activation of proliferation signaling pathway of T cell-derived cell lines by regulating the binding of ITPRIPL1-RBD to CD3 extracellular domain

Since previous studies have shown that, CD3ε binding may cause alterations in T cell proliferation and function, and since T cells are primary cells that are not conducive to intervention and detection, the most widely used model for such studies is Jurkat cells, that is, a cell line derived from T cells, and NF-KB signaling is a widely used assay that reflects the degree of activation of Jurkat or T cells. Therefore, we constructed a Jurkat-NFKB reporter cell line (by transducing firefly luciferase downstream of the NF-KB promoter into Jurkat cells by lentivirus, which was tested to be activated with Concanavalin A), in order to test the effect of ITPRIPL1 on the functional status of co-cultured T cells in the presence of tumor cell expression.

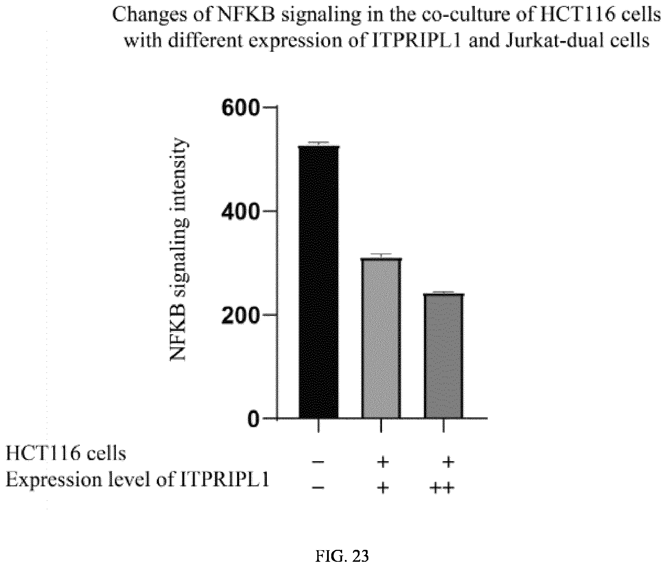

Firstly, the luciferin reporter assay demonstrates that HCT116 cells overexpressing ITPRIPL1 can reduce the NFKB proliferation signaling in Jurkat-dual cells more, as shown in FIG. 23. The cultured Jurkat-dual cells were counted, centrifuged at 1000 rpm for 5 minutes, and then resuspended with antibiotic-free IMDM medium to adjust the cell number to 2×10⁶/ml. 200 µl of the cells were added into each well of a transparent 96-well plate. HCT116 cells and HCT116-ITPRIPL1 stably transfected cell lines were digested with trypsin and counted, and the cell number was adjusted to 2×10⁶/ml. 20 µl of them were respectively added and mixed with the corresponding Jurkat-dual cells. They were co-cultured in a cell incubator for 18-24 hours. After the completion of reaction, a non-transparent 96-well plate was taken, into each well of which were added 50 µl of Quanti-luc reagent and 20 µl of the reaction mixture and mixed well, and then the signals were detected immediately with a multimode microplate reader. The above figure reflects that HCT116 cells expressing ITPRIPL1 can inhibit the NFKB proliferation signaling in Jurkat-dual cells, while HCT116 cells overexpressing ITPRIPL1 can further reduce the NFKB proliferation signaling. The above results show that HCT116 cells overexpressing ITPRIPL1 can reduce the NFKB proliferation signaling in Jurkat-dual cells more.

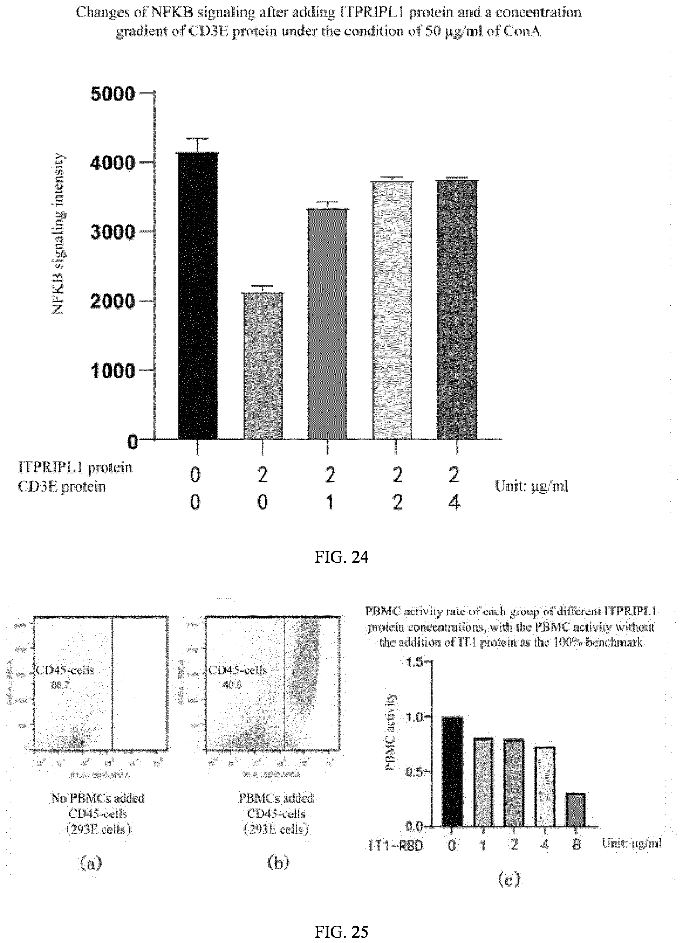

Furthermore, the luciferin reporter assay demonstrates that CD3ε protein can block the inhibition of NFKB proliferation signaling in Jurkat-dual cells by the ITPRIPL1 protein, as shown in FIG. 24. The cultured Jurkat-dual cells were counted, centrifuged at 1000 rpm for 5 minutes, and then resuspended with antibiotic-free IMDM medium to adjust the cell number to 2×10⁶/ml. 200 µl of the cells were added into each well of a transparent 96-well plate. Into each well were respectively added 2 µg/ml of ITPRIPL1 protein and 0 µg/ml, 1 µg/ml, 2 µg/ml, 4 µg/ml of CD3ε protein mixture. After reaction for two hours in a cell incubator, 50 µg/ml of Concanavalin A (ConA) was added into each well and reacted in the cell incubator for 18-24 hours. After the completion of reaction, a non-transparent 96-well plate was taken, into each well of which were added 50 µl of Quanti-luc reagent and 20 µl of the reaction mixture and mixed well, and then the signals were detected immediately with a multimode microplate reader. The above figure showed the variation in the NFKB signals of Jurkat-dual cells under the condition of adding 2 µg/ml of ITPRIPL1 protein and different concentrations of CD3ε protein, under the activation of 50 µg/ml of ConA. The above results show that the inhibition on the ConA-activated NFKB proliferation signaling in Jurkat-dual cells by the ITPRIPL1 protein can be blocked by CD3ε protein in a concentration-dependent manner, thus indicating that the inhibitory effect of ITPRIPL1 is produced from CD3ε.

### Example 8: ITPRIPL1-RBD recombinant protein can reduce the killing of kidney-derived H3K293 cells by human peripheral blood mononuclear cells (PBMCs)

It was demonstrated by using flow cytometry that ITPRIPL1-RBD recombinant protein can reduce the killing of kidney-derived H3K293 cells by human peripheral blood mononuclear cells (PBMCs). CD3 and CD28 antibodies (Invitrogen, CA, USA) were diluted by mixing with PBMCs (ATCC, VA, USA) to a final concentration of 1 µg/ml so as to activate T cells, and then cultured overnight. The next day, the PBMCs and 293E (ATCC, VA, USA) cells were counted, and the cell number was respectively adjusted to 1×10⁶/ml. Into the control group were added 100 µl of 293E cells and 100 µl of the culture medium. Each 100 µl of the two cells in the experimental groups were added into a 96-well plate (Thermo Fisher, MA, USA). Into 4 of the experimental groups was respectively added 1, 2, 4, 8 µg/ml of the ITPRIPL1-RBD recombinant protein, and incubated in an incubator for 6 hours. The 96-well plate was taken out. Each well of cells were placed in an EP tube (Axygen, CA, USA), centrifuged at 400 rcf for 5 minutes and the supernatants were then discarded. The cells were resuspended and washed with 500 µl of cell staining buffer (Invitrogen, CA, USA), and centrifuged and washed again. The CD45-APC antibodies (Invitrogen, CA, USA) were diluted with the cell staining buffer at 1:20. 200 µl of the mixture solution was added into each EP tube for resuspension, and incubated at room temperature while shaking slowly for 30 minutes. After centrifugation at 400 rcf for 5 minutes, the supernatant was discarded, and the cells were resuspended and washed with 1 ml of binding buffer (Meilun Biotech, Shanghai, China); and centrifuged and washed again. An unstained group without the addition of PBMCs, a double-stained group without the addition of PBMCs, an unstained group with the addition of PBMCs, a single-stained Annexin V-FITC group with the addition of PBMCs, a single-stained PI group with the addition of PBMCs, a double-stained group with the addition of PBMCs, and a double-stained group each with the addition of PBMCs were set, into which were respectively added 100 µl of the binding buffer, 5 µl of Annexin V-FITC (Meilun Biotech, Shanghai, China) and 10 µl of PI (Meilun Biotech, Shanghai, China) according to the conditions, and incubated at room temperature while shaking slowly for 15 minutes. 400 µl of the binding buffer was respectively further added, and transferred into flow tubes (Falcon, NY, USA) for loading (Miltenyi Biotec, Cologne, Germany).

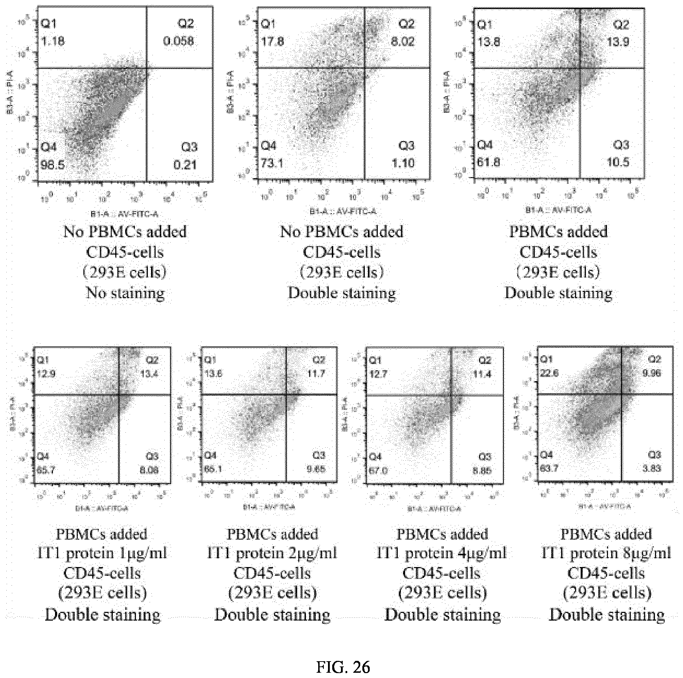

FIG. 25 (a) and (b) show the classification of 293E cells according to CD45. FIG. 25 (c) shows the relative killing activity of PBMCs calculated based on each group of apoptosis data under the condition of different ITPRIPL1 protein concentrations. FIG. 26 shows specific apoptosis staining under each condition in FIG. 25 (c). The above results show that the ITPRIPL1-RBD recombinant protein can reduce the killing of kidney-derived H3K293 cells by human peripheral blood mononuclear cells (PBMCs).

### Example 9: Knockout of ITPRIPL1 expressed in tumor cells by gene editing can significantly increase the killing of tumor cells by human peripheral blood mononuclear cells

1. Construction of CRISPR/Cas9 gene editing system, i.e., a lentivirus containing a puromycin-resistant sgRNA for specific cleavage of ITPRIPL1.

General experimental procedures: Firstly synthesizing single-stranded DNA oligo of the gRNA sequence, then annealing and pairing to produce double-stranded DNA oligo, and then linking it directly into the enzymatically cleaved CRISPR/Cas9 vector through the enzymatic cleavage sites contained in its both ends; transferring the ligation product into the prepared bacterial competent cells, and sending the grown monoclonal colonies to a sequencing company for sequencing identification, wherein the clones that were correct through comparison were successfully constructed CRISPR/Cas9 vectors. With regard to the target gene sequence of the target gene, multiple target site sequences were designed using the design principles of gRNA sequences as provided in the public website, which were shown in SEQ ID NOs:11-13. 3 pairs of gRNA oligomeric single-stranded DNA were respectively designed and synthesized according to the gene sequence, with the oligo sequences shown in SEQ ID NOs: 14-19, wherein SEQ ID NOs: 14, 15 are gRNA oligomeric single-stranded DNA sequences corresponding to the target sequence SEQ ID NO: 11; SEQ ID NOs: 16, 17 are gRNA oligomeric single-stranded DNA sequences corresponding to the target sequence SEQ ID NO: 12; and SEQ ID NOs: 18, 19 are gRNA oligomeric single-stranded DNA sequences corresponding to the target sequence SEQ ID NO: 13. The oligomeric single-stranded DNA was annealed to double-stranded, and the double-stranded gRNA oligo was respectively inserted into CRISPR/Cas9 vectors to construct CRISPR/Cas9 recombinant plasmids, which were transformed to competent cells Stbl3. The construction of vectors includes the following specific steps:
1) Annealing of gRNA: the primers were diluted with a sterile TE buffer to a final concentration of 100 µMol. 10 µl of upstream and downstream primers were separately pipetted and mixed, then blown evenly into a PCR tube for annealing. After completion, the tube was placed on ice for a few minutes, and then the primer mixture can be used for direct ligation or cryopreserved at -20°C.
2) Enzyme digestion and recovery of CRISPR/Cas9 vector: the enzyme digestion system was as below: CRISPR/Cas9 vector 5 µg; 10*Buffer 5 µl; BsmBI 4 µl; ddH2O complement to 50 µl. The enzyme digestion was conducted at 37°C for about 30 min. During this time, 0.8% of agarose gel can be formulated to be used for nucleic acid electrophoresis at the end of enzyme digestion. After electrophoresis, the strip containing the target fragment was cut off. The total weight was weighed with a balance, from which the weight of an empty tube was subtracted to calculate the gel weight, and the gel volume can be calculated by taking 100 mg as approximately 100 µl. 3 times the gel volume of QG buffer was added into the gel and the tube were placed in a water bath at 50°C to completely melt the gel, during which the EP tube was shaken appropriately to accelerate the dissolution of the gel. After the gel was completely melted, an equal volume of isopropanol with the gel was added and mixed uniformly. The above liquid was all transferred into a filter column and centrifuged at 13000 rpm for 30 seconds. The liquid in the tube was discarded, and 750 µl of PE buffer was added into the column and centrifuged for 1 minute. The liquid in the tube was discarded, and the empty tube was centrifuged for another 1 minute. A new EP tube of 1.5 ml was changed, 50 µl of EB buffer was added into the column and centrifuged for 1 min, the content in the centrifugal tube was the recovered vector.
3) Ligation of CRISPR/Cas9 vector and primer. The ligation system is as below: the recovered vector 3 µl (50 ng); Oligo primer 1 µl (0.5 µM); T4 DNA ligase buffer 1.5 µl; T4 DNA ligase 1 µl; ddH2O complement to 15 µl. Incubation in a water bath at 25°C for 30 min.
4) Transformation: the competent cells were placed on ice for natural thawing, after then the ligation products were all added into the competent cells, and placed on ice for 20 minutes, and then heat-shocked in a water bath at 42°C for 90 s. They were then immediately placed on ice for 2-3 minutes. 1000 µl of antibiotic-free LB medium was added and cultured at 37°C while shaking at 150 rpm for 45 minutes, and centrifuged at 3000 rpm for 2 min. About 850µl of supernatant was discarded. The bacterial solution at the bottom of the tube was blown to disperse, added to a petri dish containing the corresponding resistance, and coated evenly with a sterilized coater. The petri dish was inverted in a constant temperature incubator at 37°C for overnight culture.
5) Preparation of recombinant plasmid: a few single colonies were picked and subjected to a small amount of shake culture.
6) Identification of positive clones by sequencing: it was showed upon comparison that the sequence of the fragment inserted in the recombinant clone was completely consistent with the designed oligo sequence, so the vector was successfully constructed.

2. Gene editing with CRISPR/Cas9 gene editing system to construct HCT116-ITPRIPL1-knockout cell lines.

HCT116 cells (ATCC, VA, USA) were plated in a 24-well plate (Corning, NY, USA) at an appropriate density (growing to a density of about 30-40% the next day), and digested and counted the next day. The cells were first infected with a quantitative gradient of GFP control lentivirus (Genomeditech, Shanghai, China). After 48 hours, the medium was changed. 72 hours later, the GFP fluorescence was observed under a microscope to determine the optimal virus-to-cell infection ratio so as to explore the MOI value. After the MOI value was determined, the plate was re-plated to infect HCT116 cells with Cas9 system lentivirus (Genomeditech, Shanghai, China) containing blasticidin-resistance at the MOI value, and the medium was changed after 48 hours. 72 hours later, blasticidin (Invivogen, CA, USA) was added at a concentration gradient for screening for 10-14 days, and the cell lines obtained from the final screening were HCT116 cells containing the Cas9 system. After then, the HCT116 cells containing the Cas9 system were plated in a 24-well plate and counted, and infected with the lentivirus containing puromycin-resistant sgRNA that specifically cleaves ITPRIPL1 at the MOI value, and the medium was changed after 48 hours. 72 hours later, puromycin (Invivogen, CA, USA) was added at a concentration gradient for screening for 10-14 days, resulting in the HCT116-ITPRIPL1-knockout cell lines.

3. Flow cytometry demonstrates that the knockout of ITPRIPL1 expressed in tumor cells can significantly increase the killing of tumor cells by human peripheral blood mononuclear cells.

CD3 and CD28 antibodies (Invitrogen, CA, USA) were diluted by mixing with PBMCs (ATCC, VA, USA) to a final concentration of 1 µg/ml so as to activate the T cells, and then cultured overnight. The next day, the PBMCs and HCT116 wild-type (ATCC, VA, USA)/ITPRIPL1-overexpressed/ITPRIPL1-knockout cells were counted, and the cell number was respectively adjusted to 1×10⁶/ml. Into the control group were added 100 µl of tumor cells and 100 µl of the culture medium. Each 100 µl of the tumor cells and PBMCs in the experimental groups were added into a 96-well plate (Thermo Fisher, MA, USA), and incubated in an incubator for 6 hours. The 96-well plate was taken out and rinsed with PBS (Meilun Biotech, Dalian, China), and then the cells were digested with EDTA-free trypsin (Beyotime, Shanghai, China), placed in EP tubes (Axygen, CA, USA), and centrifuged at 400 rcf for 5 minutes, and the supernatants were then discarded. The cells were resuspended and washed with 500 µl of cell staining buffer (Invitrogen, CA, USA), and centrifuged and washed again. The CD45-APC antibodies (Invitrogen, CA, USA) were diluted with the cell staining buffer at 1:20. 200 µl of the mixture solution was added into each EP tube for resuspension, and incubated at room temperature while shaking slowly for 30 minutes. After centrifugation at 400 ref for 5 minutes, the supernatant was discarded, and the cells were resuspended and washed with 1 ml of binding buffer (Meilun Biotech, Dalian, China); and centrifuged and washed again. An unstained group without the addition of PBMCs, a double-stained group without the addition of PBMCs, an unstained group with the addition of PBMCs, a single-stained Annexin V-FITC group with the addition of PBMCs, a single-stained PI group with the addition of PBMCs, a double-stained group with the addition of PBMCs, and a double-stained group each with the addition of IT1 protein were set, into which were respectively added 100 µl of the binding buffer, 5 µl of Annexin V-FITC (Meilun Biotech, Dalian, China) and 10 µl of PI (Meilun Biotech, Dalian, China) according to the conditions, and incubated at room temperature while shaking slowly for 15 minutes. 400 µl of the binding buffer was respectively further added, and transferred into flow tubes (Falcon, NY, USA) for loading (Miltenyi Biotec, Cologne, Germany).

FIG. 27 (a) and (b) show the classification of tumor cells according to CD45. FIG. 27 (c) shows the relative killing activity of PBMCs calculated based on each group of apoptosis data under different ITPRIPL1 expression conditions. FIG. 28 shows specific apoptosis staining under each condition in FIG. 27 (c). The above results show that the overexpression of ITPRIPL1 can reduce the killing of tumor cells by PBMCs, while the knockout of ITPRIPL1 can promote the killing of tumor cells by PBMCs.

### Example 10: Antibodies prepared with ITPRIPL1-RBD protein as the immunogen can effectively promote the killing of tumor cells by immune cells

CD3 and CD28 antibodies (Invitrogen, CA, USA) were diluted by mixing with PBMCs (ATCC, VA, USA) to a final concentration of 1 µg/ml so as to activate T cells, and then cultured overnight. The next day, the PBMCs and HCT116 cells (ATCC, VA, USA) were counted, and the cell number was respectively adjusted to 1×10⁶/ml. Into the control group were added 100 µl of HCT116 cells and 100 µl of the culture medium. Each 100 µl of the two cells of the experimental groups were added into a 96-well plate (Thermo Fisher, MA, USA). Into 4 of the experimental groups were respectively added ITPRIPL1 polyclonal antibodies of 1:500/250/125/62.5, and incubated in an incubator for 6 hours. The 96-well plate was taken out and rinsed with PBS (Meilun Biotech, Dalian, China), and then the cells were digested with EDTA-free trypsin (Beyotime, Shanghai, China), placed in EP tubes (Axygen, CA, USA), and centrifuged at 400 ref for 5 minutes, and the supernatants were then discarded. The cells were resuspended and washed with 500 µl of cell staining buffer (Invitrogen, CA, USA), and centrifuged and washed again. The CD45-APC antibodies (Invitrogen, CA, USA) were diluted with the cell staining buffer at 1:20. 200 µl of the mixture solution was added into each EP tube for resuspension, and incubated at room temperature while shaking slowly for 30 minutes. After centrifugation at 400 rcf for 5 minutes, the supernatant was discarded, and the cells were resuspended and washed with 1 ml of binding buffer (Meilun Biotech, Shanghai, China); and centrifuged and washed again. An unstained group without the addition of PBMCs, a double-stained group without the addition of PBMCs, an unstained group with the addition of PBMCs, a single-stained Annexin V-FITC group with the addition of PBMCs, a single-stained PI group with the addition of PBMCs, a double-stained group with the addition of PBMCs, and a double-stained group each with the addition of ITPRIPL1 protein were set, into which were respectively added 100 µl of the binding buffer, 5 µl of Annexin V-FITC (Meilun Biotech, Shanghai, China) and 10 µl of PI (Meilun Biotech, Shanghai, China) according to the conditions, and incubated at room temperature while shaking slowly for 15 minutes. 400 µl of the binding buffer was respectively further added, and transferred into flow tubes (Falcon, NY, USA) for loading (Miltenyi Biotec, Cologne, Germany).

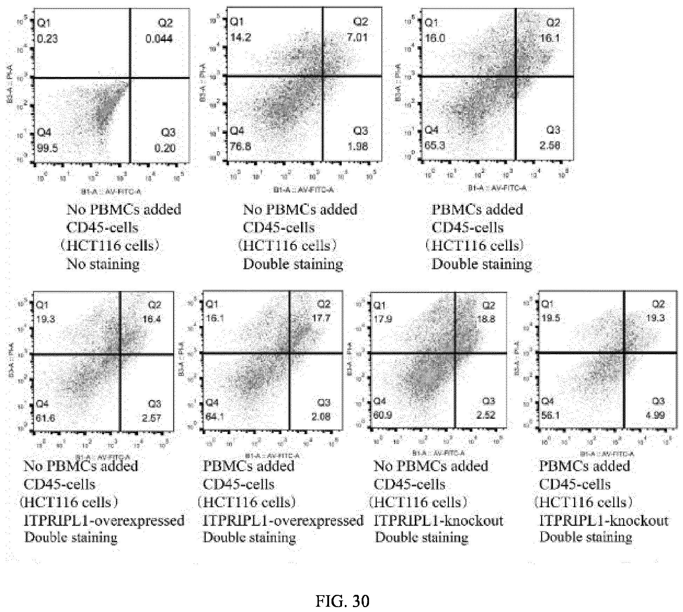

FIG. 29 (a) and (b) show the classification of HCT116 cells according to CD45. FIG. 29 (c) shows the relative killing activity of PBMCs calculated based on each group of apoptosis data under the condition of different polyclonal antibody concentrations. FIG. 30 shows specific apoptosis staining under each condition in FIG. 29 (c). The above results show that the ITPRIPL1 polyclonal antibodies can promote the killing of tumor cells by PBMCs.

### Example 11: The binding ability of ITPRIPL1-RBD2 sequence mutants to CD3E is reduced to some extent, and the binding ability of ITPRIPL1-RBD3 to CD3_{E} is significantly reduced

### 1. Construction of HCT116-RBD2 and RBD3 sequence mutants

Based on the published sequence (NCBI reference sequence NM_001008949.3), from the extracellular domain (25-103 amino acids) of which DRMDLDTLARSRQLEKRMSEEMRLLEMEFEERKRAAEQRQKAENFWTGDTSSDQ (ITPRIPL1-RBD2, i.e., SEQ ID NO: 2) and MDLDTLARSRQLEKRMSEEMRLLEMEFEERKRAAEQRQKAEN (ITPRIPL1-RBD3, i.e., SEQ ID NO: 3) were respectively selected as two different target sequences to synthesize a specific ITPRIPL1 sequence mutant with pcDNA3.1 as the vector, wherein the C termini of the extracellular domain and the extracellular domain-and-transmembrane domain were fused to Flag tags and green fluorescent proteins (GFPs), thereby producing ITPRIPL1 extracellular domain, ITPRIPL1-RBD2, ITPRIPL1-RBD3 plasmids containing Flag tags and green fluorescent proteins (GFPs).

2. It is analyzed by co-immunoprecipitation experiment that ITPRIPL1-RBD2 is the shortest sequence that binds to CD3E.

The pcDNA3.1 plasmids respectively containing Flag-tagged ITPRIPL1 extracellular domain, ITPRIPL1-RBD2, ITPRIPL1-RBD3 and HA-tagged CD3E were co-transfected into HCT116 cells (ATCC, VA, USA), cultured in a 6-well plate (Corning, NY, USA) for 48-72 hours until the protein was fully expressed, and then lysed with a hybrid lysate of immunoprecipitation lysate (Thermo Fisher, MA, USA) mixed with a triple of protease-phosphatase-PMSF (Consun, Shanghai, China) at 1:100, and the cells were scraped. A portion of the cell samples were centrifuged, mixed with loading buffer (Beyotime, Shanghai, China) and denatured in a metal bath at 100°C to obtain an input level of protein samples; the remaining cell samples were immunoprecipitated with HA-tagged specific mouse antibodies (Biolegend, CA, USA) or Flag-tagged specific mouse antibodies (CST, MA, USA), washed with PBS, mixed with the loading buffer (Beyotime, Shanghai, China) and denatured in a metal bath at 100°C to obtain immunoprecipitated protein samples. And then, 12.5% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of the ITPRIPL1 extracellular domain, ITPRIPL1-RBD2, ITPRIPL1-RBD3 and CD3E-HA protein. After blocking with rapid blocking buffer (Epizyme, Shanghai, China) for 10 minutes, the corresponding ITPRIPL1 extracellular domain, ITPRIPL1-RBD2, ITPRIPL1-RBD3 and CD3E-HA strips were respectively incubated with Flag-tagged specific rabbit antibodies (Abeam, MA, USA) and HA-tagged specific rabbit antibodies (CST, MA, USA) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

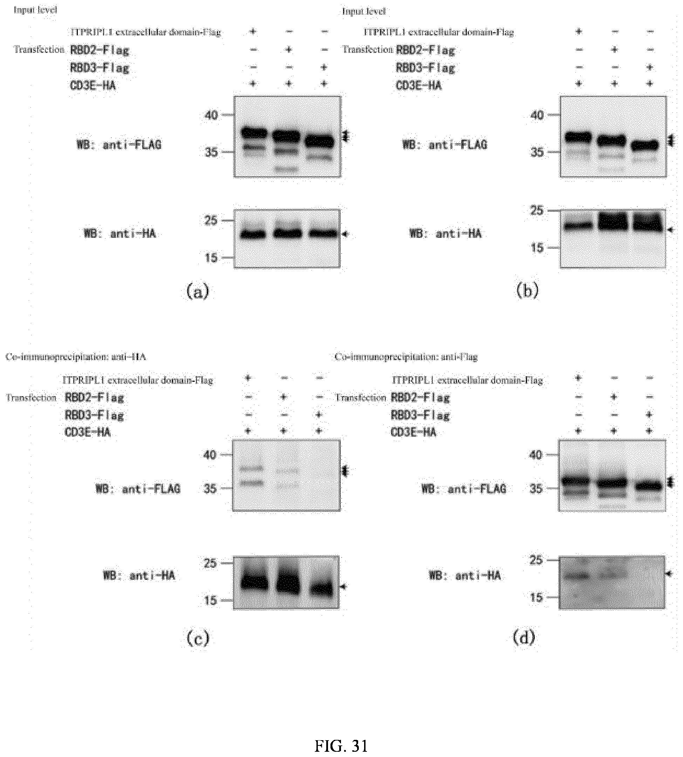

FIG. 31 (a) shows the contents of mutants of different ITPRIPL1 sequences and CD3ε in the input protein, and FIG. 31 (b) shows the mutants of different ITPRIPL1 sequences that are indirectly precipitated because of binding to CD3ε, and the directly precipitated CD3ε. FIG. 31 (c) shows the contents of mutants of different ITPRIPL1 sequences and CD3ε in the input protein, and FIG. 31 (d) shows the CD3E that are indirectly precipitated because of binding to mutants of different ITPRIPL1 sequences, and the directly precipitated mutants of different ITPRIPL1 sequences. The above results show that, in the presence of ITPRIPL1 extracellular domain and RBD2 sequence mutants, CD3E and ITPRIPL1 remain the status of binding; while no significant binding to RBD3 sequences is detected. It can be concluded from the above that there is a positive correlation between the length or completeness of the ITPRIPL1 extracellular domain and the ability to bind CD3E.

### Example 12: Isolated ITPRIPL1-RBD protein having the ability to bind NRP2

1. Enzyme-linked immunosorbent assay (ELISA) shows that there is direct concentration-dependent binding of the isolated fragments from the ITPRIPL1 (IT1) extracellular domain to the NRP2 extracellular domain.

An ELISA special plate (costar, ME, USA) was used. Firstly, the plate was coated with 0.03125/0.0625/0.125/0.25/0.5/1/2 µg/ml of ITPRIPL1-RBD recombinant protein each dissolved in 100 µl of ELISA coating buffer (Solarbio, Beijing, China), while for the negative control, the plate was coated with 100 µl of coating buffer free of proteins. The plate was coated at 4°C overnight. After washing with PBST, they were blocked with 100 µl of 5% BSA dissolved in PBS (VWR, PA, USA) in an incubator at 37°C for 90 minutes. After washing with PBST, they were incubated with 1 µg/ml of protein fragments from the NRP2 extracellular domain (Sino Biological Inc., Beijing, China) in an incubator at 37°C for 60 minutes for binding, in which the NRP2 proteins contained hFc (N2-Fc). After washing with PBST, they were incubated with PBS-diluted specific anti-human Fc segment antibodies (Abeam, MA, USA) in an incubator at 37°C for 30 minutes for binding. After washing with PBST, a color developing solution (Sangon, Shanghai, China) was added at 100 µl per well for color development, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

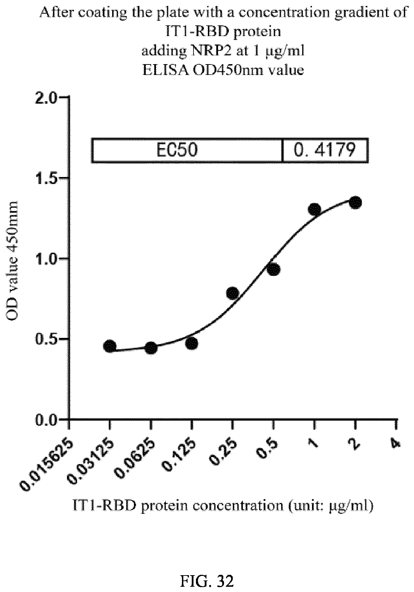

As shown in FIG. 32, this experiment shows that there is direct concentration-dependent binding of the isolated fragments from the ITPRIPL1 extracellular domain to the NRP2 extracellular domain. The above results demonstrate that the isolated purified protein from the ITPRIPL1 extracellular domain (IT1-RBD) directly binds to the purified protein from the NRP2 extracellular domain in a concentration-dependent manner.

2. Flow cytometry demonstrates that NRP2 binds to cells overexpressing ITPRIPL1 with higher efficiency.

It was found after testing that, HEK293 cells endogenously expressed a certain level of ITPRIPL1, and the expression level was further increased after stable transfection. The cultured 293E wild-type cells (ATCC, VA, USA) and 293E-ITPRIPL1 stably transfected cell lines were digested and then counted, and the cell number was respectively adjusted to 2×10⁶/ml. 200 µl of them were respectively added into the wells of a 96-well plate (costar, ME, USA). Into the wells of each experimental group were respectively added 0/0.5/1/2/4 µg/ml of NRP2 protein (Sino Biological Inc., Beijing, China). The 96-well plate was placed in a cell incubator, standing for 30 minutes, and then taken out and transferred into EP tubes (Axygen, CA, USA), centrifuged at 400 ref for 5 minutes and the supernatants were then discarded. The cells were resuspended and washed with 500 µl of cell staining buffer (Invitrogen, CA, USA) and centrifuged at 400 rcf for 5 minutes, the supernatants were then discarded again and the washing was repeated. Anti-human IgG Alexa Fluor 647 antibodies (Invitrogen, CA, USA) were diluted with the cell staining buffer at 1:1000. In addition to the negative control, 200 µl of antibody diluent was added into each EP tube and incubated at room temperature for 30 minutes at 40 rpm on a shaker. After then, the EP tubes were taken out and centrifuged at 400 rcf for 5 minutes and the supernatants were then discarded. The cells were resuspended and washed with 1000 µl of the cell staining buffer and centrifuged at 400 ref for 5 minutes, the supernatants were then discarded again and the washing was repeated. After the completion of washing, 300 µl of the cell staining buffer was added into each EP tube for resuspension, and transferred into flow tubes (Falcon, NY, USA) for on-board analysis (Miltenyi Biotec, Cologne, Germany).

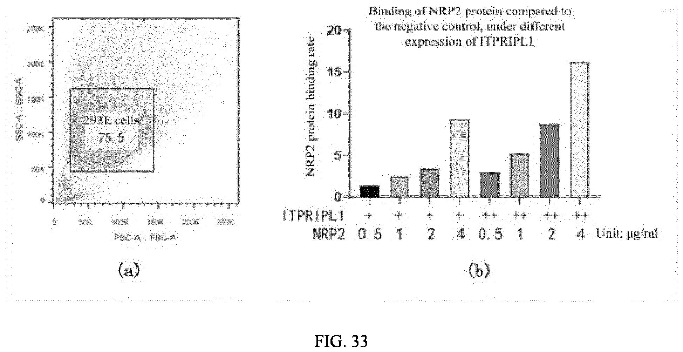

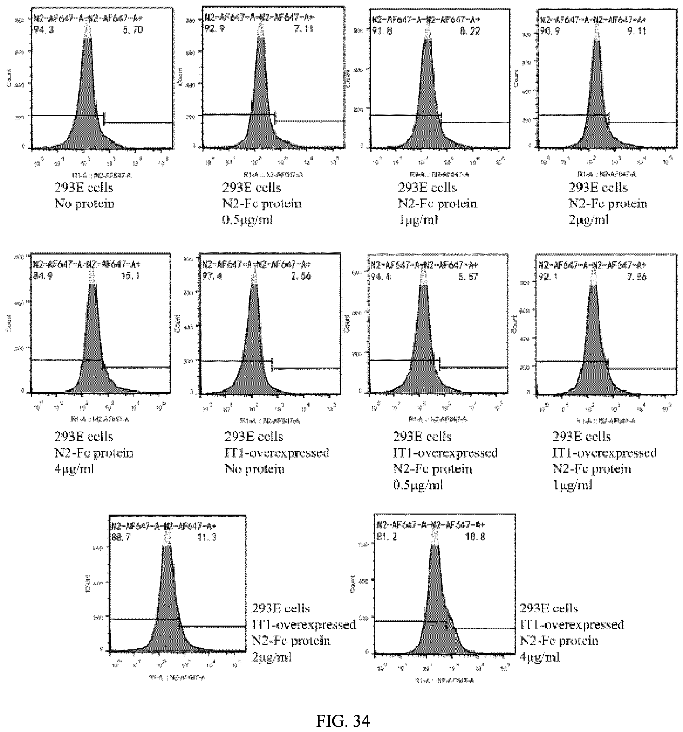

FIG. 33 (a) shows the threshold setting for not classified as dead cells. FIG. 33 (b) reflects the binding of cells with different expression of ITPRIPL1 to NRP2 protein. FIG. 34 shows the specific staining and protein binding under each condition in FIG. 33 (b), respectively. The above results show that NRP2 can bind to 293E with a certain expression of ITPRIPL1, and can bind to 293E cells overexpressing ITPRIPL1 more strongly.

### Example 13: Isolated ITPRIPL1-RBD protein has the ability to transmit inhibitory signals to differentiated THP1 macrophages expressing the NRP2 protein

The luciferin reporter assay demonstrates that the free isolated purified protein from the ITPRIPL1 extracellular domain (IT1-RBD) can inhibit the IFN proliferation signaling of THP1-dual cells completed by PMA-induced differentiation under the condition of inactivation.

The cultured THP1-dual cells (Invivogen, CA, USA) were counted, centrifuged at 1000 rpm for 5 minutes, and then the cells were resuspended with antibiotic-free RPMI-1640 medium (Gibco, CA, USA) to adjust the cell number to 2× 10⁶/ml. 200 µl of the cells and 20 ng/ml of PMA reagent (Invivogen, CA, USA) were added into each well of a transparent 96-well plate (Thermo Fisher, MA, USA) for induction, cultured in an incubator for 3 hours, and then washed with PBS and the medium was exchanged. 72 hours later, the medium was exchanged again, 0/1/2/4/8 µg/ml of free isolated purified protein from the ITPRIPL1 extracellular domain were respectively added and cultured in a cell incubator for 18-24 hours. After the completion of reaction, a non-transparent 96-well plate was taken, into each well of which were added 50 µl of Quanti-luc reagent (Invivogen, CA, USA) and 10 µl of the cell mixture and mixed well, and then the signals were detected immediately with a multimode microplate reader.

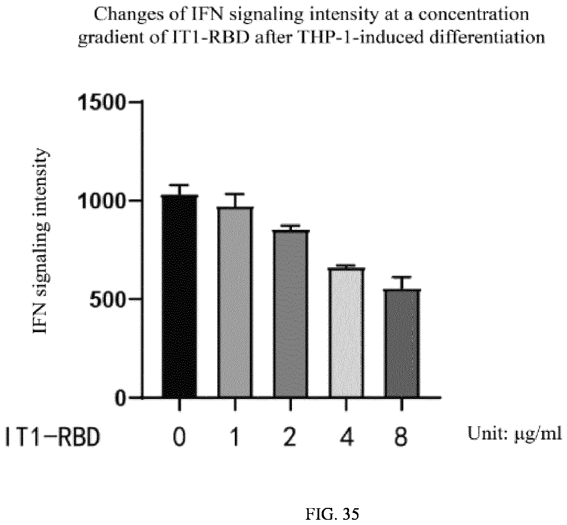

As shown in FIG. 35, the above results show that the free isolated purified protein from the ITPRIPL1 extracellular domain (IT1-RBD) can inhibit the IFN proliferation signaling of THP1-dual cells completed by PMA-induced differentiation under the condition of inactivation. It can be concluded from the above that the isolated ITPRIPL1-RBD protein has the ability to transmit inhibitory signals to differentiated THP1 macrophages expressing the NRP2 protein.

### Example 14: the purified IT1-RBD-Fc protein has the functions of inhibiting T cell signals and killing

### 1. Separation and purification of IT1-RBD-Fc protein.

Based on the published sequence (NCBI reference sequence NM_001008949.3), from which an extracellular domain (HPLMVSDRMDLDTLARSRQLEKRMSEEMRLLEMEFEERKRAAEQRQKAENFWTGDTSSD QLVLGKKDMGWPFQADGQEG) was selected as IT1-RBD1, i.e., all parts in the extracellular domain with signal peptides removed; DRMDLDTLARSRQLEKRMSEEMRLLEMEFEERKRAAEQRQKAENFWTGDTSSDQ was selected as IT1-RBD2, i.e., multiple conserved sequence amino acids at the N-terminus and multiple conserved and non-conserved sequence amino acids at the C-terminus were removed on the basis of RBD1 (for verifying the importance of conserved amino acid sites of mammals on the function); MDLDTLARSRQLEKRMSEEMRLLEMEFEERKRAAEQRQKAEN was selected as IT1-RBD3 (only the sequence predicted to be the Alpha helical secondary structure was reserved, verifying whether the secondary structure was the minimal sequence necessary for the function), followed by a tandem Fc sequence that did not contain the CH1 region (PKSCDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK), corresponding plasmids were construct with pcDNA3.1 as the vector. The mutated derivative sequences of the above several ITPRIPL1 extracellular domains are shown in FIG. 54.

The sequence in which the ITPRIPL1 extracellular domain exerted its function was characterized through this example. Protein-protein docking using the AlphaFold predicted structure of the functional fragment of the ITPRIPL1 extracellular domain and the x-ray diffraction crystal structure (1XIW.pdb) of CD3E was analyzed, indicating that the ITPRIPL1 of multiple species such as human, mouse, rat, grivet, golden monkey, black snub-nosed monkey, Bolivian squirrel monkey, Ma's night monkey, chimpanzee, etc. as shown in FIG. 54 all can bind to CD3E, which is consistent with the results of immunofluorescence and colocalization analysis in Example 2.

Therefore, according to the different sites of different species, an RBD1 derivative sequence with CD3E binding function was obtained: DRMDLDTLARSRQLEKRMSEEMRxLEMEFEERxxxAExxQKxENxWxGxTSxDQ (wherein x represents an amino acid that can be substituted).

Functional detection and analysis of RBD1, RBD2, RBD3 were conducted in this example.

After plasmids were acquired, they were transfected with a PEI reagent (Life-iLab, Shanghai, China) into 293E cells (ATCC, VA, USA), and 120 hours later, the cells were lysed with RIPA lysate (Beyotime, Shanghai, China) and a triple of protease inhibitor-phosphatase inhibitor-PMSF (Consun, Shanghai, China) on ice for 10 minutes, and centrifuged at 12000 rpm for 15 minutes. The supernatants were then aspirated and shaken with 1:100 of protein A magnetic bead (Smart-Lifesciences, Changzhou, China) at 130 rpm for 2 hours at room temperature. The mixture solution was aspirated into a filter column (Millipore, MA, USA) pre-equilibrated with equilibrium liquid (20 mM disodium hydrogen phosphate, 0.15 M sodium chloride, pH 7.0) for washing with the equilibrium liquid for two times, and then eluted with an eluent (0.1 M glycine, pH 3.0): neutralization solution (1 M Tris-HCl, pH 8.5) of 16:1, resulting in the purified IT1-RBD-Fc protein. The concentration of the protein was determined with a Nanodrop spectrophotometer, and it was stored at -20°C.

2. Luciferin reporter assay demonstrates that the purified IT1-RBD-Fc protein can inhibit the NFKB proliferation signaling in Jurkat-dual cells under activation and inactivation conditions of ConA.

The cultured Jurkat-dual cells (Invivogen, CA, USA) were counted, centrifuged at 1000 rpm for 5 minutes and then resuspended with antibiotic-free IMDM medium (Gibco, CA, USA) to adjust the cell number to 2×10⁶/ml. 200 µl of the cells were added into each well of a transparent 96-well plate. Into each well was respectively added 0/4 µg/ml of the purified IT1-RBD1/RBD2/RBD3-Fc protein. After reaction in a cell incubator for two hours, into each well was added 50 µg/ml of Concanavalin A (ConA) (Aladdin, Shanghai, China) as the activation group or added an equal volume of endotoxin-free water as the inactivation group and reacted in the cell incubator for 18-24 hours. After the completion of reaction, a non-transparent 96-well plate (costar, ME, USA) was taken, into each well of which were added 50 µl of Quanti-luc reagent (Invivogen, CA, USA) and 20 µl of the reaction mixture and mixed well, and then the signals were detected immediately with a multimode microplate reader.

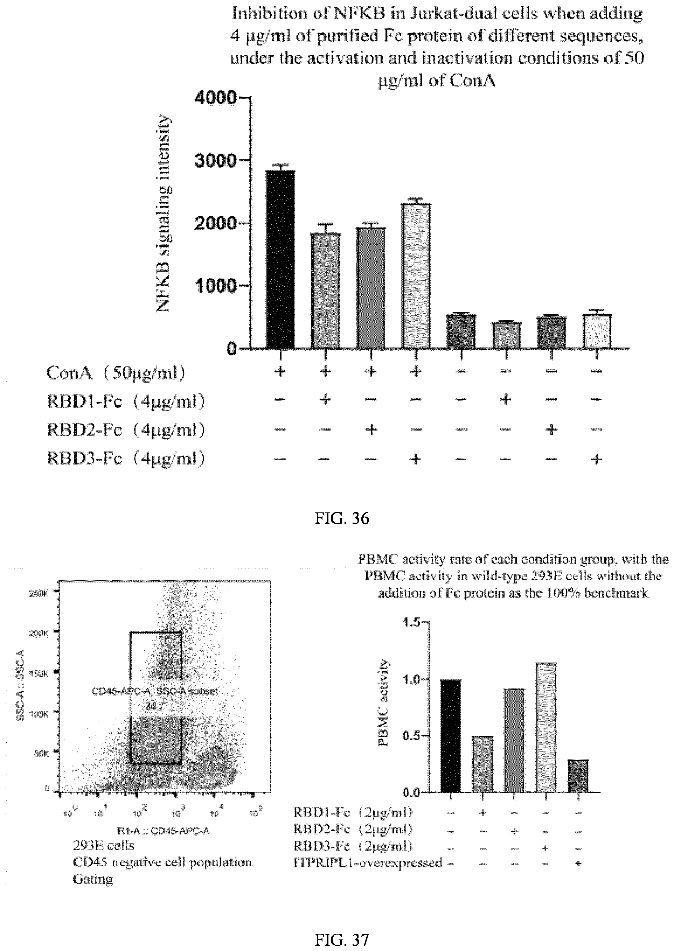

As shown in FIG. 36, it shows the NFKB signaling changes in Jurkat-dual cells under the condition of adding 4 µg/ml of Fc protein of different IT1-RBD segments, under the activation and inactivation conditions of 50 µg/ml of ConA. The above results show that the purified IT1-RBD1-Fc protein can inhibit the NFKB proliferation signaling in Jurkat-dual cells under the activation and inactivation conditions of ConA, and with the gradual shortening of the sequence, the inhibitory effect gradually diminishes.

3. Flow cytometry demonstrates that the purified IT1-RBD1-Fc recombinant protein can reduce the killing of kidney-derived H3K293 cells by human peripheral blood mononuclear cells (PBMCs).

HEK293 cells have been widely used to construct autoimmune disease models for the studies on the disease progression, molecular and cellular biological mechanisms, and pharmacology of kidney autoimmune diseases, autoimmune diseases of the nervous system, systemic lupus erythematosus (SLE), etc. (Stepanenko AA, Gene. 2015 Sep 15; 569(2):182-90, Hira S. J Physiol Sci. 2019 Sep; 69(5):723-732., Keskitalo S, Front Immunol. 2019 Dec 5; 10:2770). Furthermore, HEK293 cells have also been used to construct post-transplant rejection disease models (Yi Gao. Int J Clin Exp Med. 2014; 7(11): 4572-4583, Lorber, Marc I, Transplantation: 1999-67(6)-p 897-903, Pabois A, Biochem Pharmacol. 2016 Mar 15; 104:95-107), antiviral infection immune responses (Ismail Cem Yilmaz, Allergy. 2021 Sep 14. doi: 10.1111/all.15091, Elizabeth A Reap, Vaccine. 2007 Oct 16; 25(42):7441-9), and tumor immune responses, etc. (A A Stepanenko, Gene. 2015 Sep 15; 569(2):182-90).

Furthermore, peripheral blood mononuclear cells (PBMCs) derived from autoimmune diseases or normal people have also been used in many studies to construct autoimmune disease models (Yoshikawa N. Horm Metab Res. 1994 Sep; 26(9):419-23.), post-transplant rejection (Transplant Proc. 2016 Oct; 48(8):2840-2844.), anti-infective immune responses (Har-Noy M, J Transl Med. 2020 May 12; 18(1):196, Nakamura-Hoshi M, Sci Rep. 2020 Jul 9; 10(1):11394.), anti-tumor immune responses, etc. (Zhuang X, Cancer Immunol Res. 2019 Jun; 7(6):939-951), in which the target cells involved also include HEK293 cells. In fact, the effect of PBMC-based humanized NSG mouse model is highly correlated with the effect of PBMCs on target cells in vitro. Therefore, an in vitro model based on PBMCs and target cells plays an important role in the disease process and the manifestation of drug efficacy.

By using HEK293 cells as target cells for autoimmunity, transplant rejection, allergies, and anti-tumor reactions, and by using human peripheral blood mononuclear cells (PBMCs) as the effector cells, this example exemplifies the effect of ITPRIPL1-based regulators on the interaction of antigen-presenting cells and T cells as well as the intervention effect on the above different diseases through a representative disease model.

The implementation process is detailed below: CD3 and CD28 antibodies (Invitrogen, CA, USA) were diluted by mixing with PBMCs (ATCC, VA, USA) to a final concentration of 1 µg/ml so as to activate T cells, and then cultured overnight. The next day, the PBMCs and 293E wild-type (ATCC, VA, USA)/ITPRIPL1-overexpressed cells were counted, and the cell number was respectively adjusted to 1×10⁶/ml. Into the control group were added 100 µl of 293E wild-type /ITPRIPL1-overexpressed cells and 100 µl of culture medium. For the experimental groups, each 100 µl of PBMCs and another kind of cells were added into a 96-well plate (costar, ME, USA). Among the experimental groups, into 3 groups of wild-type 293E cells was further respectively added 2 µg/ml of IT1-RBD1/RBD2/RBD3-Fc protein, and incubated in an incubator for 6 hours. The 96-well plate was taken out, from which the cells were aspirated, placed in an EP tube (Axygen, CA, USA), and centrifuged at 400 rcf for 5 minutes, and the supernatant was then discarded. The cells were resuspended and washed with 500 µl of cell staining buffer (Invitrogen, CA, USA), and centrifuged and washed again. The CD45-APC antibodies (Invitrogen, CA, USA) were diluted with the cell staining buffer at 1:20. 200 µl of the mixture solution was added into each EP tube for resuspension, and incubated at room temperature while shaking slowly for 30 minutes. After centrifugation at 400 rcf for 5 minutes, the supernatant was discarded, and the cells were resuspended and washed with 1 ml of binding buffer (Beyotime, Shanghai, China); and centrifuged and washed again. An unstained group without the addition of PBMCs, a double-stained group without the addition of PBMCs, an unstained group with the addition of PBMCs, a single-stained Annexin V-FITC group with the addition of PBMCs, a single-stained PI group with the addition of PBMCs, a double-stained group with the addition of PBMCs, and a double-stained group each with the addition of IT1 protein were set, into which were respectively added 100 µl of the binding buffer, 5 µl of Annexin V-FITC (Beyotime, Shanghai, China) and 10 µl of PI (Beyotime, Shanghai, China) according to the conditions, and incubated at room temperature while shaking slowly for 15 minutes. 400 µl of the binding buffer was respectively further added, and transferred into flow tubes (Falcon, NY, USA) for loading (Miltenyi Biotec, Cologne, Germany).

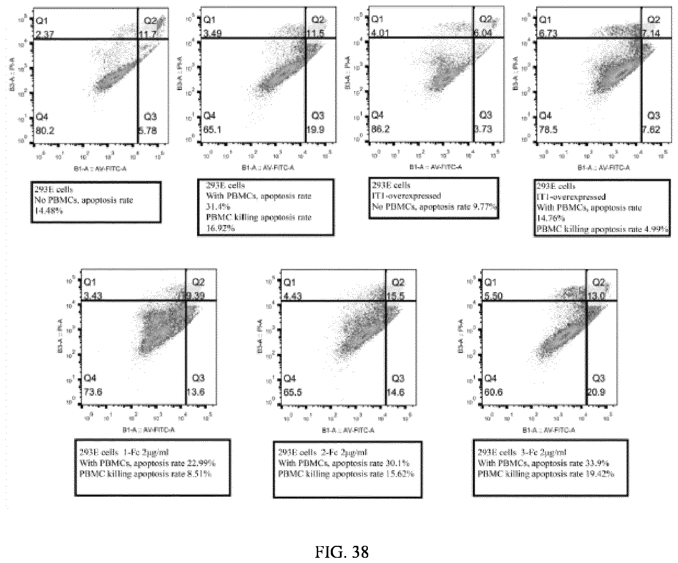

FIG. 37 (a) shows the classification of 293E cells according to CD45. FIG. 37 (b) shows the relative killing activity of PBMCs calculated based on each group of apoptosis data under the conditions of different ITPRIPL1 expression and different proteins. FIG. 38 shows specific apoptosis staining under each condition in FIG. 37 (b). The above results show that the purified IT1-RBD1-Fc recombinant protein can reduce the killing of kidney-derived H3K293 cells by human peripheral blood mononuclear cells (PBMCs), and with the gradual shortening of the sequence, the reduction effect gradually diminishes.

4. Western Blot experiment demonstrates that the ITPRIPL1-RBD recombinant protein and the purified IT1-RBD1-Fc recombinant protein have the function of inhibiting the ZAP70 and Akt pathways downstream of the CD3E.

The cultured Jurkat cells (ATCC, VA, USA) were counted. 4×10⁶ of the cells were taken into a centrifugal tube of 15 ml (Corning, NY, USA) and centrifuged at 800 rpm for 4 minutes, and the supernatant was then discarded. The cells were resuspended in serum-free RPMI1640 medium (Meilun Biotech, Dalian, China), cultured in an incubator for 3 hours of starvation, centrifuged at 800 rpm for 4 minutes, and resuspended in 4 ml of complete medium (Meilun Biotech, Dalian, China). Each 1 ml of them was taken and added into each well of a 12-well plate (Corning, NY, USA). Into the each well was respectively added: no treatment; 4 µg/ml of IT1-RBD protein; 4 µg/ml of RBD1-Fc protein; 4 µg/ml of RBD2-Fc protein. After mixing well and culturing in the incubator for 10 min, the plate was taken out, and the cells were transferred into an EP tube of 1.5 ml (Axygen, CA, USA), centrifuged at 800 rpm for 4 minutes, resuspended and washed with PBS and centrifuged, and washed again. After the completion of centrifugation, the supernatant was discarded. The RIPA lysate (Beyotime, Shanghai, China) and a triple of protease inhibitor-phosphatase inhibitor-PMSF (Consun, Shanghai, China) were formulated at 1:100, and 80 µl of the hybrid lysate was added into each tube of cells. Each EP tube was frozen and thawed on liquid nitrogen-ice for three cycles, and centrifuged at 12000 rpm at 4°C for 15 minutes after the last thawing. After the completion of centrifugation, the supernatants were taken and formulated into a variety of cell samples at a ratio of 4:1 of the supernatant to 5×loading buffer (Beyotime, Shanghai, China), and denatured in a metal bath at 100°C for 10 minutes to produce protein samples. And then, 10% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of Akt, ZAP70 and GAPDH protein. After blocking with rapid blocking buffer (Epizyme, Shanghai, China) for 10 minutes, the corresponding strips were respectively incubated with pAkt specific rabbit antibodies (CST, MA, USA), Akt specific rabbit antibodies (CST, MA, USA), pZAP70 specific rabbit antibodies (CST, MA, USA), GAPDH antibodies (Consun, Shanghai, China) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

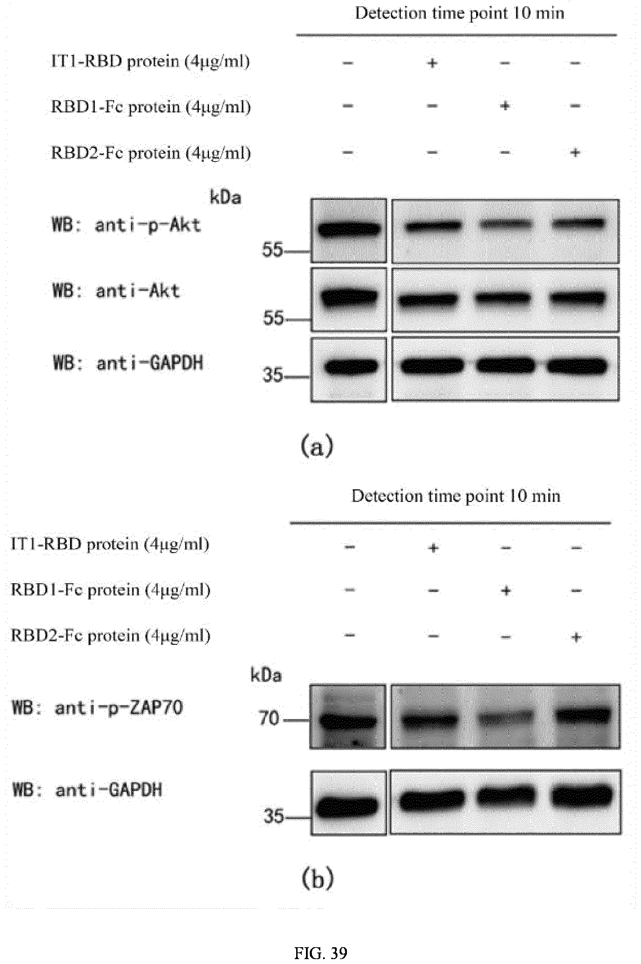

FIG. 39 (a) and (b) respectively shows the expression of the phosphorylated Akt and the phosphorylated ZAP70 after the alignment of GAPDH internal reference in Western blotting, wherein both the phosphorylation of Akt and the phosphorylation of ZAP70 are reduced to some extent after treating with the ITPRIPL1-RBD recombinant protein and the purified IT1-RBD-Fc protein, and the reduction of phosphorylation by the IT1-RBD1-Fc recombinant protein is more obvious than that by the IT1-RBD2-Fc protein. The experimental results show that the ITPRIPL1-RBD recombinant protein and the purified IT1-RBD1-Fc protein have the effect of reducing the phosphorylation of Akt and ZAP70 downstream of the CD3E, thus indicating that they have the function of inhibiting the corresponding T cell pathways.

5. Western Blot experiment demonstrates that CD3E protein has the function of blocking the effect of ITPRIPL1-RBD1-Fc recombinant protein on the phosphorylation of ZAP70 and Akt pathways.

The cultured Jurkat cells (ATCC, VA, USA) were counted. 4×10⁶ of the cells were taken into a centrifugal tube of 15 ml (Corning, NY, USA) and centrifuged at 800 rpm for 4 minutes, and the supernatant was then discarded. The cells were resuspended in serum-free RPMI1640 medium (Meilun Biotech, Dalian, China), cultured in an incubator for 3 hours of starvation, centrifuged at 800 rpm for 4 minutes, and resuspended in 4 ml of complete medium (Meilun Biotech, Dalian, China). Each 1 ml of them was taken and added into each well of a 12-well plate (Corning, NY, USA). Into the each well was respectively added: no treatment; 4 µg/ml of RBD1-Fc protein; 4 µg/ml of RBD1-Fc protein + 4 µg/ml of CD3E protein. After mixing well and culturing in the incubator for 10 min, the plate was taken out, and the cells were transferred into an EP tube of 1.5 ml (Axygen, CA, USA), centrifuged at 800 rpm for 4 minutes, resuspended and washed with PBS and centrifuged, and washed again. After the completion of centrifugation, the supernatant was discarded. The RIPA lysate (Beyotime, Shanghai, China) and a triple of protease inhibitor-phosphatase inhibitor-PMSF (Consun, Shanghai, China) were formulated at 1:100, and 80 µl of the hybrid lysate was added into each tube of cells. Each EP tube was frozen and thawed on liquid nitrogen-ice for three cycles, and centrifuged at 12000 rpm at 4°C for 15 minutes after the last thawing. After the completion of centrifugation, the supernatants were taken and formulated into a variety of cell samples at a ratio of 4:1 of the supernatant to 5×loading buffer (Beyotime, Shanghai, China), and denatured in a metal bath at 100°C for 10 minutes to produce protein samples. And then, 10% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of Akt, ZAP70, ERK and GAPDH protein. After blocking with rapid blocking buffer (Epizyme, Shanghai, China) for 10 minutes, the corresponding strips were respectively incubated with pAkt specific rabbit antibodies (CST, MA, USA), Akt specific rabbit antibodies (CST, MA, USA), pZAP70 specific rabbit antibodies (CST, MA, USA), ZAP70 specific rabbit antibodies (CST, MA, USA), pERK specific rabbit antibodies (CST, MA, USA), ERK specific rabbit antibodies (CST, MA, USA), GAPDH antibodies (Consun, Shanghai, China) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

FIG. 41 shows the Western Blot experimental results after changing the corresponding phosphorylation pathways. As shown in the figure, the phosphorylation of Akt, ZAP70, ERK was all significantly reduced after the addition of ITPRIPL1-RBD1-Fc, with ZAP70 being the most obvious; and the downward trend of the phosphorylation was counteracted with the addition of CD3E protein. The experimental results show that CD3E can block the modulation of phosphorylation pathway by the ITPRIPL1-RBD1-Fc protein, and ITPRIPL1-RBD-Fc regulates the phosphorylation pathway of T cells through CD3.

### Example 15: Regulation of T cell functions by ITPRIPL1 requires correct conformational changes in CD3 and PRS segments

### 1. Construction of CD3 mutant Jurkat cells

The cultured Jurkat cells (ATCC, VA, USA) were counted. 2×10⁵ of the cells were taken into each well of a 24-well plate (Corning, NY, USA), and plated overnight. 500 µl of complete medium (Meilun Biotech, Dalian, China) was mixed with knockout lentivirus (GenePharma, Shanghai, China) against CD3 at a MOI value of 100 for viral infection. 48 hours later, the medium was changed. After continuing the incubation for 24 hours, 1 µg/ml of puromycin was added for screening for a period of one week. At the end of screening, CD3-knockout Jurkat cell lines were obtained.

The CD3-knockout Jurkat cells were counted. 2×10⁵ of the cells were taken into each well of a 24-well plate, and plated overnight. 500 µl of complete medium was mixed with CD3-K76T (unable to make correct conformational changes) and CD3-ΔPRS (with changes in the PRS segments)-overexpressed viruses at a MOI value of 100 for viral infection. 48 hours later, the medium was changed. After continuing the incubation for 24 hours, 600 µg/ml of Geneticin (G418) (Gibco, CA, USA) was added for screening for a period of one week. At the end of screening, CD3-mutated Jurkat cell lines were obtained.

### 2. Western Blot experiment demonstrates that the modulation on the phosphorylation pathway of T cells by ITPRIPL1 requires the conformational changes in CD3 and PRS segments

Wild-type Jurkat cells, CD3-knockout Jurkat cells, CD3-K76T Jurkat cells, and CD3-ΔPRS cells were counted. 1×10⁶ of the cells were taken into each well of a 12-well plate (Corning, NY, USA). After activation by adding 10 µg/ml of OKT3, the wells were respectively subjected to no treatment or treated with 4 µg/ml of ITPRIPL1-RBD1-Fc, and cultured for 10 minutes to harvest the cells. The cells were transferred into EP tubes of 1.5 ml (Axygen, CA, USA), centrifuged at 800 rpm for 4 minutes, resuspended and washed with PBS and centrifuged, and washed again. After the completion of centrifugation, the supernatants were discarded. The RIPA lysate (Beyotime, Shanghai, China) and a triple of protease inhibitor-phosphatase inhibitor-PMSF (Consun, Shanghai, China) were formulated at 1:100, and 80 µl of the hybrid lysate was added into each tube of cells. Each EP tube was frozen and thawed on liquid nitrogen-ice for three cycles, and centrifuged at 12000 rpm at 4°C for 15 minutes after the last thawing. After the completion of centrifugation, the supernatants were taken and formulated into a variety of cell samples at a ratio of 4:1 of the supernatant to 5×loading buffer (Beyotime, Shanghai, China), and denatured in a metal bath at 100°C for 10 minutes to produce protein samples. And then, 10% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of Akt, ZAP70, ERK and GAPDH protein. After blocking with rapid blocking buffer (Epizyme, Shanghai, China) for 10 minutes, the corresponding strips were respectively incubated with pAkt specific rabbit antibodies (CST, MA, USA), Akt specific rabbit antibodies (CST, MA, USA), pZAP70 specific rabbit antibodies (CST, MA, USA), ZAP70 specific rabbit antibodies (CST, MA, USA), pERK specific rabbit antibodies (CST, MA, USA), ERK specific rabbit antibodies (CST, MA, USA), GAPDH antibodies (Consun, Shanghai, China) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

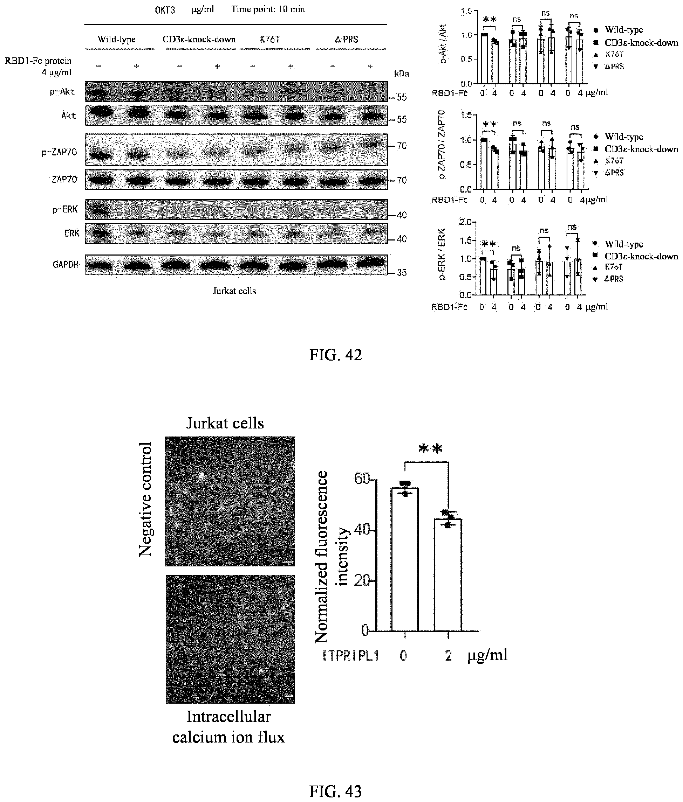

FIG. 42 shows the changes in the phosphorylation pathway of the CD3 mutant Jurkat cells under the action of ITPRIPL1-RBD1-Fc protein as shown by Western blotting. The results show that, the reduced phosphorylation of wild-type Jurkat cells caused by ITPRIPL1 was not observed in CD3-knockout/K76T-mutated/ΔPRS-mutated Jurkat cells, demonstrating that the modulation on the phosphorylation pathway of T cells by ITPRIPL1 requires the conformational changes in CD3 and PRS segments.

### 3. Fluorescence staining experiment of intracellular calcium ion flux demonstrates that ITPRIPL1 has the effect of reducing the activity of T cells

The cultured Jurkat cells (ATCC, VA, USA) were counted. 5×10⁵ of the cells were taken into each well of a 12-well plate (Corning, NY, USA), and treated with PBS or 2 µg/ml of ITPRIPL1-RBD protein for 24 hours. After the treatment was completed, 4 µmol of Fluo-8 AM (Abeam, MA, USA) intracellular calcium ion indicator was given for staining, and incubated in an incubator in dark for 1 hour. After washing with HHBS buffer (Solarbio, Beijing, China) twice, the plate was observed under a fluorescence microscope.

FIG. 43 shows the signal intensity of intracellular calcium ion flux observed under the fluorescence microscope. The experimental results show that, ITPRIPL1 has the function of significantly reducing the calcium ion flux within the Jurkat cells, demonstrating that ITPRIPL1 has the effect of reducing the activity of T cells.

### 4. Fluorescence staining experiment of intracellular calcium ion flux demonstrates that the effect of ITPRIPL1 to reduce the activity of T cells relies on the conformational changes of CD3 and PRS segments

Wild-type Jurkat cells, CD3-knockout Jurkat cells, CD3-K76T Jurkat cells, CD3-ΔPRS cells were counted. 5×10⁵ of cells were taken into each well of a 12-well plate (Corning, NY, USA), and treated with PBS or 4 µg/ml of ITPRIPL1-RBD1-Fc protein for 24 hours. After the treatment was completed, 4 µmol of Fluo-8 AM (Abeam, MA, USA) intracellular calcium ion indicator was given for staining, and incubated in an incubator in dark for 1 hour. After washing with HHBS buffer (Solarbio, Beijing, China) twice, the plate was observed under a fluorescence microscope .

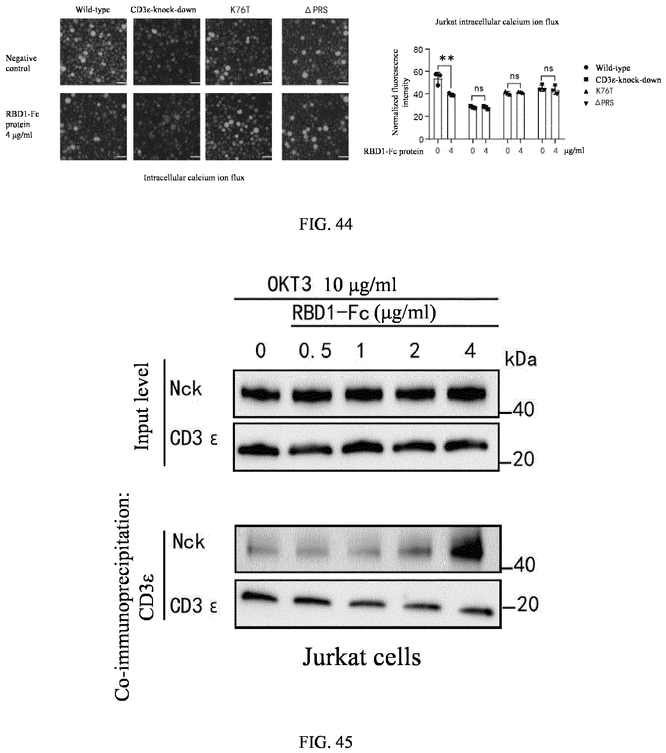

FIG. 44 shows the signal intensity of intracellular calcium ion flux observed under the fluorescence microscope. The experimental results show that, no significant effect of ITPRIPL1 on the calcium ion flux within wild-type Jurkat cells was observed in CD3-knockout/K76T-mutated/ΔPRS-mutated Jurkat cells, demonstrating that the signaling regulation of T cells by ITPRIPL1 requires the conformational changes of CD3 and PRS segments.

### Example 16: ITPRIPL1 regulates the signaling and function of T cells by regulating the binding of CD3-Nck

1. Western Blot experiment demonstrates that ITPRIPL1-RBD1-Fc protein can increase the binding signal of CD3 to Nck in a concentration-dependent manner.

The cultured Jurkat cells (ATCC, VA, USA) were counted. 5×10⁶ of the cells were taken into a centrifugal tube of 15 ml (Corning, NY, USA) and centrifuged at 800 rpm for 4 minutes and the supernatant was then discarded. The cells were resuspended in serum-free RPMI1640 medium (Meilun Biotech, Dalian, China), cultured in an incubator for 3 hours of starvation, centrifuged at 800 rpm for 4 minutes, and resuspended in 5 ml of complete medium (Meilun Biotech, Dalian, China). Each 1 ml of them was taken and added into each well of a 12-well plate (Corning, NY, USA), into which was firstly added 10 µg/ml of OKT3 for activation. And at the same time, into the each well was respectively added: no treatment; 0.5/1/2/4 µg/ml of RBD1-Fc protein. After mixing well and culturing in the incubator for 5 min, the plate was taken out, and the cells were transferred into an EP tube of 1.5 ml (Axygen, CA, USA), centrifuged at 800 rpm for 4 minutes, resuspended and washed with PBS and centrifuged, and washed again. After the completion of centrifugation, the supernatant was discarded. The immunoprecipitation lysate (Thermo Fisher, MA, USA) and a triple of protease inhibitor-phosphatase inhibitor-PMSF (Consun, Shanghai, China) were formulated at 1:100, and 200 µl of the hybrid lysate was added into each tube of cells. A portion of the cell samples were centrifuged, mixed with loading buffer (Beyotime, Shanghai, China) and denatured in a metal bath at 100°C to obtain an input level of protein samples; the remaining cell samples were immunoprecipitated with CD3E specific mouse antibodies (Santa cruz biotechnology, CA, USA), washed with PBS, mixed with the loading buffer (Beyotime, Shanghai, China) and denatured in a metal bath at 100°C to obtain immunoprecipitated protein samples. The supernatant after centrifugation was formulated with 5×loading buffer (Beyotime, Shanghai, China) at a ratio of 4:1 into various cell samples, and denatured in a metal bath at 100°C for 10 minutes to obtain protein samples. 10% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of CD3E and Nck protein. After blocking with rapid blocking buffer (Epizyme, Shanghai, China) for 10 minutes, the corresponding strips were respectively incubated with CD3E specific rabbit antibodies (CST, MA, USA) and Nck specific rabbit antibodies (CST, MA, USA) at 4°C overnight. The next day, after washing with TBST, the strips were incubated with specific anti-rabbit secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

As shown in FIG. 45, the above results show that ITPRIPL1-RBD1-Fc protein can upregulate the binding of CD3 to Nck in a concentration-dependent manner. It can be concluded from the above that ITPRIPL1 can increase the binding signal of CD3 to Nck in a concentration-dependent manner.

2. Proximity ligation assay demonstrates that ITPRIPL1-RBD1-Fc protein can increase the binding signal of CD3 to Nck.

The cultured Jurkat cells (ATCC, VA, USA) were counted. 2×10⁶ of the cells were taken into a centrifugal tube of 15 ml (Corning, NY, USA), centrifuged at 800 rpm for 4 minutes and the supernatant was then discarded. The cells were resuspended in serum-free RPMI1640 medium (Meilun Biotech, Dalian, China), cultured in an incubator for 3 hours of starvation, centrifuged at 800 rpm for 4 minutes, and resuspended in 2 ml of complete medium (Meilun Biotech, Dalian, China). Each 1 ml of them was taken and added into each well of a 12-well plate (Corning, NY, USA), into which was firstly added 10 µg/ml of OKT3 for activation. And at the same time, into the each well was respectively added: no treatment; 4 µg/ml of RBD1-Fc protein. After culturing in the incubator for 5 min, Jurkat cells were taken out, and subjected to relative operations with the reagents in the proximity ligation assay kit (Merck, NJ, USA) as well as CD3E specific mouse antibodies (Santa cruz biotechnology, CA, USA) and Nck specific rabbit antibodies (CST, MA, USA) according to the instructions provided with the kit. After mounting was completed, the slide was observed under a fluorescence microscope.

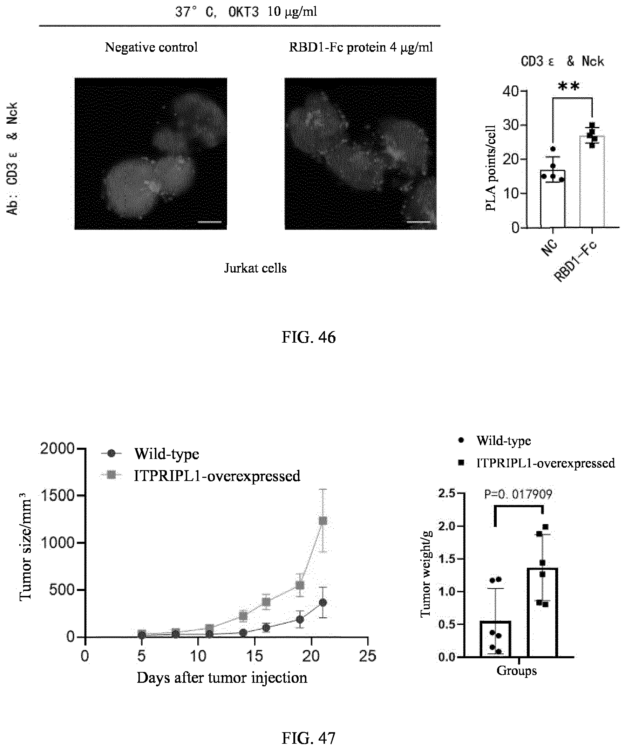

As shown in FIG. 46, the proximity ligation assay shows that the ITPRIPL1-RBD1-Fc protein can significantly increase the binding of CD3 to Nek, thereby regulating the signaling and function of T cells.

### Example 17: ITPRIPL1 has the functions of in vivo regulating immunity and the function of T cells, as well as promoting the immune evasion of tumors

### 1. Construction of humanized CD3ε mouse MC38 subcutaneous xenograft tumor in vivo model

The constructed full-length ITPRIPL1-Flag plasmid and the empty pcDNA3.1 plasmid was respectively transfected into MC38 cells (Kerafast, MA, USA), cultured in an incubator for 24-48 hours, and screened by adding 200 µg/ml of Geneticin (G418) (Gibco, CA, USA). 10-14 days later, after the empty pcDNA3.1 plasmid-transfected group of cells all died, MC38-ITPRIPL1 stably transfected cell lines were obtained.

6-8-week-old humanized CD3ε mice (Model Organisms Center, Shanghai, China) were selected and randomly grouped according to the weight, with 6 mice in each group. MC38 wild-type and MC38-ITPRIPL1 stably transfected cell lines were counted, and resuspended with PBS to a cell density of 1.5×10⁷/ml. The mice were shaved, and 1.5 ×10⁶ wild-type or ITPRIPL1-overexpressed MC38 cells were subcutaneously inoculated into the right armpit to construct in vivo models of humanized CD3ε mouse MC38 subcutaneous xenograft tumor.

### 2. Overexpression of ITPRIPL1 significantly increases the tumor growth in mice

After completing the construction of humanized CD3ε mouse MC38 subcutaneous xenograft tumor in vivo models, the tumor size was measured with a vernier caliper from the fifth day after inoculation. The long diameter and short diameter of the tumor were measured each time, and the tumor size was calculated following the formula of 1/2*A*a*a. Measurement was conducted every three days, and the tumor sizes were recorded. On day 23 after inoculation, all mice were sacrificed. After removing the tumors, the tumor weight was weighed and statistically analyzed.

As shown in FIG. 47, both the tumor size and tumor weight show that the overexpression of ITPRIPL1 can significantly increase the tumor growth, with an in vivo function. Due to the widely upregulation of the expression of ITPRIPL1 in human tumors (see other examples of the present disclosure), the results of this example show that ITPRIPL1 play a role of promoting the immune evasion in human tumors. Therefore, the inhibitors against ITPRIPL1 have the effect of inhibiting tumor growth, and the regulator of ITPRIPL1 has an application value in the preparation of drugs for treating tumors.

### 3. Flow cytometry demonstrates that the overexpression of ITPRIPL1 inhibits the activity of T cells in mice

At least 1 ml of peripheral blood was obtained from mice by means of cardiac blood sampling. Mouse PBMC cells were obtained by using a mouse peripheral blood PBMC separation kit (Solarbio, Beijing, China) according to the corresponding instruction of the kit.

The mouse PBMCs were placed into EP tubes (Axygen, CA, USA), centrifuged at 400 rcf for 5 minutes and the supernatants were then discarded. The cells were resuspended and washed with 500 µl of cell staining buffer (Invitrogen, CA, USA), and centrifuged and washed again. Mouse CD8-APC antibodies (Biolegend, CA, USA), mouse CD69-APC antibodies (Biolegend, CA, USA), and mouse CD137-APC antibodies (Biolegend, CA, USA) were diluted with the cell staining buffer at 1:20. 200 µl of the mixture solution was added into each EP tube for resuspension, and incubated at room temperature while shaking slowly for 30 minutes. After centrifugation at 400 ref for 5 minutes, the supernatant was discarded, and the cells were resuspended and washed with 500 µl of the cell staining buffer; and centrifuged and washed again. 200 µl of the cell staining buffer was respectively added, and transferred into flow tubes (Falcon, NY, USA) for loading (Miltenyi Biotec, Cologne, Germany).

As shown in FIG. 72, according to the fluorescence positive rate of cells, RBD1-bound antibody can significantly increase the expression of CD8, CD25, CD139. The experimental results show that RBD1-bound antibody can relieve the inhibition of ITPRIPL1 on the activity of T cells in mice.

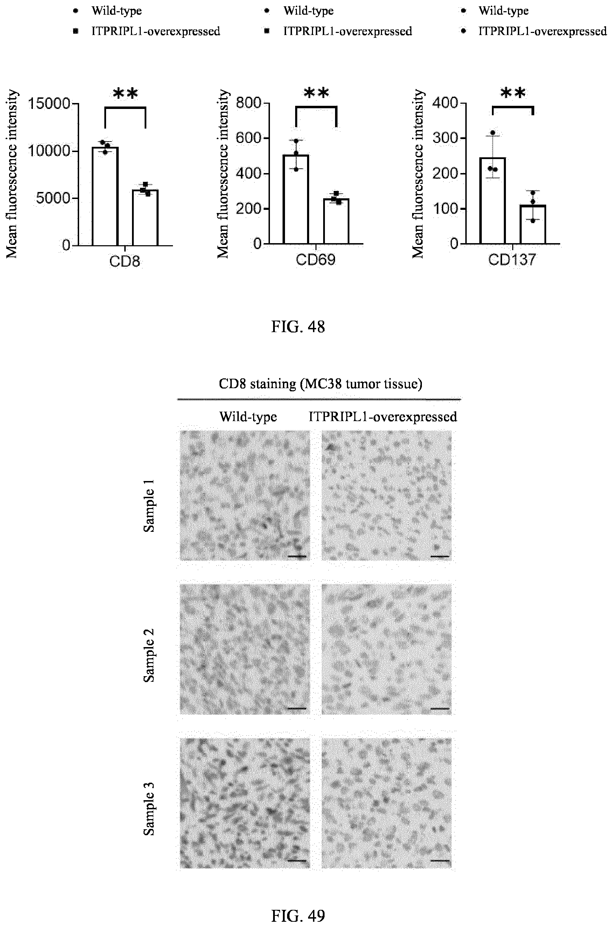

As shown in FIG. 48, according to the mean fluorescence intensity, the overexpression of ITPRIPL1 can significantly reduce the expression of CD8, CD25, CD139. The experimental results show that the overexpression of ITPRIPL1 can inhibit the activity of T cells in mice.

### 4. Immunohistochemical staining demonstrates that the overexpression of ITPRIPL1 reduces the infiltration of T cells in tumors

MC38 tumor tissues were stripped for sectioning and paraffin embedding treatment (Biossci, Wuhan, China). The resulting paraffin sections were subjected to dewaxing, hydration, antigen retrieval or other treatment, and then incubated with mouse CD8 antibodies (CST, MA, USA) in a wet box overnight. The next day, after rewarming, secondary antibodies were incubated according to the reagent instructions, stained with DAB (Solarbio, Beijing, China) and hematoxylin (Solarbio, Beijing, China), and then air-dried in a reverse alcohol concentration gradient and mounted. After the mounting was completed, the slide was observed under a fluorescence microscope and photographed under natural light.

As shown in FIG. 49, the overexpression of ITPRIPL1 can significantly reduce the positive rate of CD8 in tumor tissues. The experimental results show that the overexpression of ITPRIPL1 can reduce the infiltration of T cells in MC38 tumor tissues.

### 5. Construction of ITPRIPL1 heterozygous/homozygous knockout mouse in vivo models

By using the CRISPR/Cas9 technique, Itpripl1 gene protein reading frame shift and function deletion were caused by introducing mutations through non-homologous recombination repairment. The brief process was as below: Cas9 mRNA and gRNA were obtained by means of in vitro transcription; Cas9 mRNA and gRNA were microinjected into the fertilized eggs of C57BL/6J mice to obtain mice of F0 generation. The positive mice of F0 generation identified by PCR amplification and sequencing were mated with C57BL/6J mice to obtain six positive mice of F1 generation, that were, heterozygous knockout and homozygous knockout ITPRIPL1 mouse.

### 6. Flow cytometry demonstrates that ITPRIPL1-knockout enhances the activity of T cells

Blood was sampled from mouse models, from which PBMCs were separated. The cells in the cell suspension were counted. After counting, the cell suspension was diluted with PBS solution to adjust the concentration of the test cells to 10⁶/mL. 200 µl of the cell suspension was taken and centrifuged at 1000 rpm for 5 min (at 4°C). The cells were resuspended in 100 µl of PBS; the test tubes were set as below:

Negative control tube: 100 µl of cell suspension;
CD39 test tube: 100 µl of cell suspension adding with 2 µl of PD1 antibody, mixing well gently;
CD73 test tube: 100 µl of cell suspension adding with 2 µl of PD-L1 antibody, mixing well gently;

The antibodies are all from (Thermo Fisher, MA, USA).

Incubation at 4°C in dark for 30 minutes. At the end of incubation, testing with a flow cytometer.

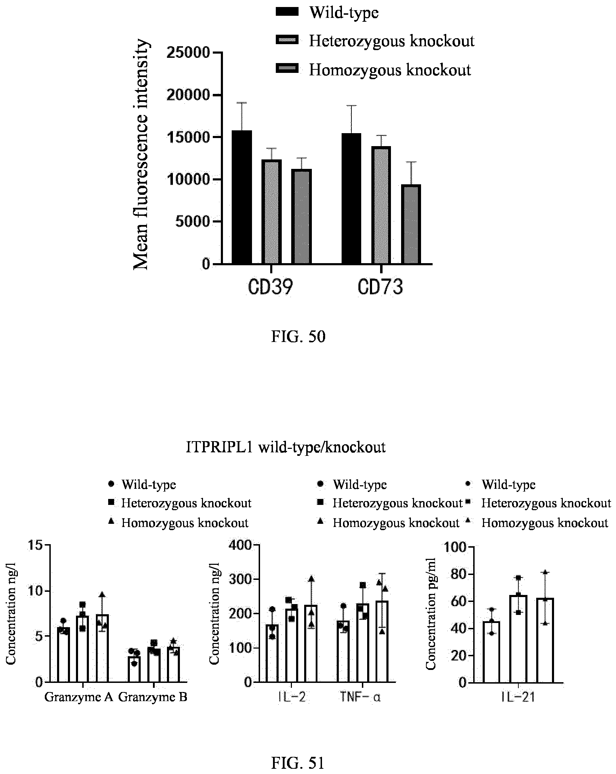

As shown in FIG. 50, after knockout of ITPRIPL1, the inhibitory surface molecules CD39, CD73 all significantly decreased, indicating that the knockout of ITPRIPL1-knockout can enhance the in vivo activity of T cells, thus verifying the negative regulation effect of ITPRIPL1 itself on T cells.

### 7. ELISA assay demonstrates that the knockout of ITPRIPL1 increases the secretion of immuno-activated cytokines

Mouse PBMCs were operated and treated according to the instructions of ELISA kits for cytokines such as granzyme A, granzyme B, IL-2, TNF-alpha, IL-21 (Abnova, CA, USA), obtaining the corresponding experimental results.

As shown in FIG. 51, the knockout of ITPRIPL1 increases the secretion of cytokines such as granzyme A, granzyme B, IL-2, TNF-alpha, IL-21, indicating that the in vivo secretion of immuno-activated factors increases after the knockout of ITPRIPL1, thus suggesting the immunosuppressive effect of ITPRIPL1 itself.

### 8. Immunohistochemical staining demonstrates that the knockout of ITPRIPL1 increases the infiltration of T cells in testicular tissues

The testicular tissues of mice were stripped for staining and sectioning (JRDUN, Shanghai, China), and observed under a fluorescence microscope for the corresponding results.

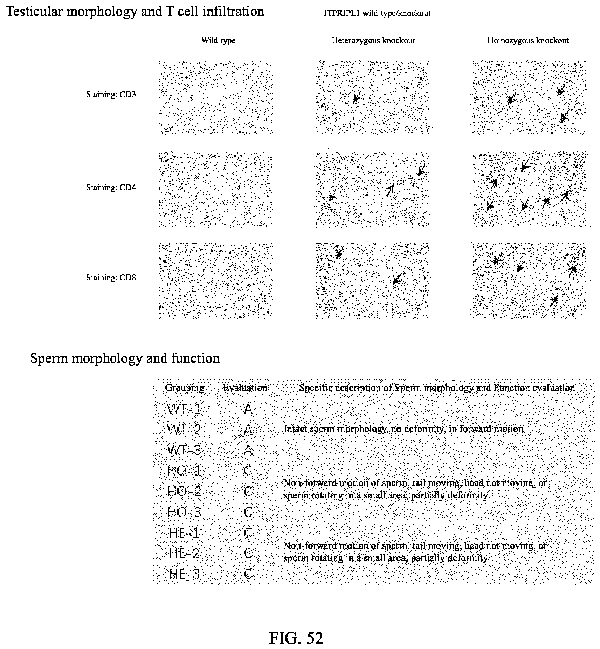

As shown in FIG. 52, the immunohistochemical staining demonstrates that after the knockout of ITPRIPL1, the positive rates of CD3, CD4, CD8 in testis all increase, suggesting the increase in the infiltration of T cells. Correspondingly, heterozygous and homozygous knockout mice have abnormal sperm morphology, abnormal motion mode and significantly reduced vitality, which are consistent with the significant inflammatory infiltration occurring in the testis, thus strongly supporting the autoimmune disorder in testicular tissues themselves. The above results indicate that ITPRIPL1 negatively regulates the activity of T cells under physiological conditions in the testis, and that ITPRIPL plays a key role in maintaining the immune privilege state of the testis. Therefore, the expression of ITPRIPL1 may be used to predict the occurrence of testicular autoimmune diseases and autoimmune infertility, and regulate the activity of ITPRIPL1 so as to be used for the intervention and treatment of autoimmune infertility.

### Example 18: ITPRIPL1 as a biomarker for indicating the presence of tumor cells in the body, predicting the progression and stages of tumors, and distinguishing the boundaries between tumors and normal tissues.

The present disclosure innovatively reveals that ITPRIPL1 plays an important role in the immune evasion of tumors, and tumors with elevated expression of ITPRIPL1 are suitable to be treated with the inhibitor of ITPRIPL1 to inhibit the tumor growth. Therefore, it is very important to detect the expression of ITPRIPL1. The present disclosure exemplifies a method of detecting the expression of ITPRIPL1 using its specific antibodies, and reveals that the high expression of ITPRIPL1 can be used to indicate the presence of tumor cells in the body, and can precisely mark the boundaries between tumor tissues and normal tissues. Furthermore, ITPRIPL1 is also significantly correlated with the progression and stages of tumors. In particular:

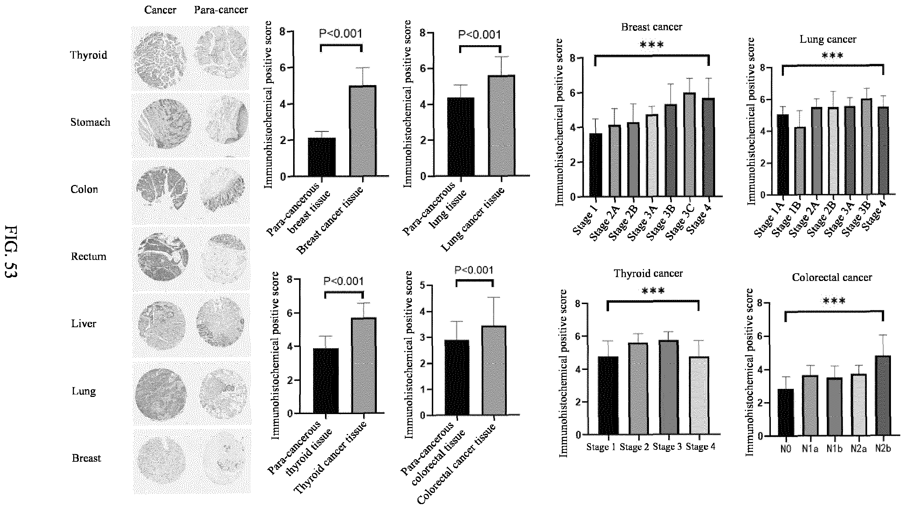

The procedures for producing ITPRIPL1 polyclonal antibodies were as previously described. ITPRIPL1 monoclonal antibodies were further prepared by hybridoma screening and isolation and culture. Immunohistochemical staining was performed on microarrays of a variety of tumor tissues (Shanghai Outdo Biotech) with the antibodies of ITPRIPL1, and the images were scanned and the expression level and distribution of ITPRIPL1 were statistically analyzed. As shown in FIG. 53, the protein expression level of ITPRIPL1 in many common cancers were significantly increased, and were obviously higher than that in para-cancerous tissues. At the same time, ITPRIPL1 was significantly correlated with the progression and stages of the tumor. For example, the expression at the progressive stage of breast cancer (stages of 3C, 4) was significantly higher than that at the early stage (stage 1); the expression at the progressive stage of lung cancer (stage 4) was significantly higher than that at the early stage (stage 1A); ITPRIPL1 in colorectal cancer and thyroid cancer had the above characteristic of having higher expression at the progressive stage than at the early stage. The results show that, the ITPRIPL1 biomarker can be used to indicate the presence of tumor cells in the body and predict the progression and stages of tumors.

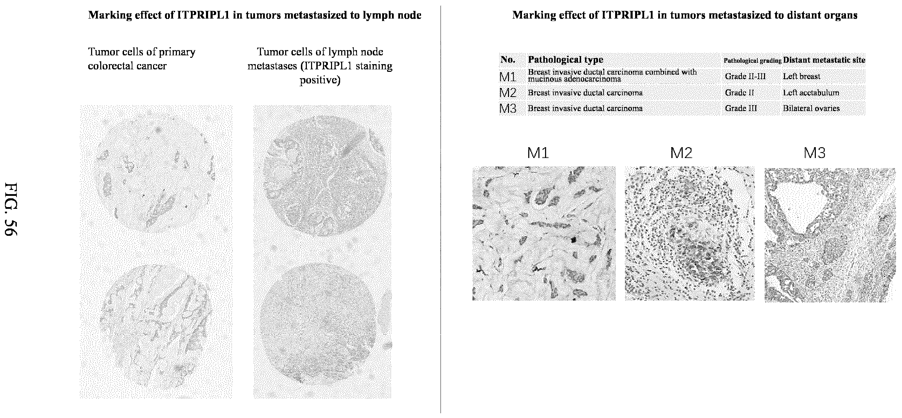

The staining of ITPRIPL1 in tumor tissues showed a very prominent consistency. Firstly, ITPRIPL1 was generally significantly up-regulated in many of the above tumors, with exceptions in only a few tumors. Secondly, there was strong uniformity in the expression of ITPRIPL1 in tumor tissues, i.e., the expression was relatively consistent in different sites of the primary lesions and in different cells of the metastases. This characteristic can be seen in FIG. 56. ITPRIPL1 was significantly expressed in tumor cells that metastasize into lymph nodes, which can be used to distinguish cancer cell tissues and normal lymph node tissues; ITPRIPL1 was also significantly highly expressed in distant metastatic tumor cells, which can used to distinguish metastases and normal tissues. The above characteristics of ITPRIPL1 make it suitable for distinguishing tumor and normal cells and tissues, as well as for precisely marking the boundaries between tumors and normal cells and tissues. Such a marker is suitable for analyzing the tumor size and infiltration extent before and after the treatment. Therefore, the expression of ITPRIPL1 is an important companion diagnostic marker for the ITPRIPL1 regulator drug. At the same time, the lower heterogeneity makes ITPRIPL1 a very promising biomarker for tumor diagnosis.

### Example 19: mouse hybridoma antibody can specifically bind to ITPRIPL1

### 1. Preparation of ITPRIPL1 mouse hybridoma antibody

(1) By using human ITPRIPL1-Fc recombinant protein (as set forth in SEQ ID NO: 21) as the immunogen, C57BL/6 mice were immunized for multiple times to enhance the effect:
   1) primary immunization, 50 µg of antigen per mouse, multi-point subcutaneous injection with Freund's complete adjuvant, at an interval of 3 weeks;
   2) secondary immunization, dosage and route as above, with Freund's incomplete adjuvant, at an interval of 3 weeks;
   3) third immunization, dosage as above, without the adjuvant, intraperitoneal injection at an interval of 3 weeks;
   4) booster immunization, at a dosage of 50 µg, intraperitoneal injection. Three days after the last injection, blood sampling to test its potency.
(2) After the immune effect had been tested to meet the requirements, blood was taken, polyclonal antibodies were separated and purified in a cumulative manner for several times. Specific experimental steps include:
   1) Preparation of a protein G sepharose CL-4B affinity column. 10 mL of protein G sepharose CL-4B packings were prepared, and equal volumes of the packings and TBS buffer solution were mixed in a vacuum flask and stirred. Evacuation was performed for 15 minutes to remove air bubbles in the packings. The protein G sepharose CL-4B packings were slowly added into a glass column while controlling the filling rate at 1 mL/min-2 mL/min with a pump. To avoid column dryness, the column was equilibrated with a pre-cooled TBS buffer solution that was 10 times the bed volume;
   (2) Preparation of polyclonal antibodies. The polyclonal antibodies were slowly thawed in ice water or in a 4°C refrigerator to avoid the aggregation of protein. Solid sodium azide was added to a concentration of 0.05%, and centrifuged at 15,000 × g for 5 min at 4°C. The clarified polyclonal antibodies were removed out and filtered through a filter to remove excess lipid;
   (3) Affinity chromatography. The antibodies were diluted with TBS buffer solution at a ratio of 1:5, and filtered through a filter. The polyclonal antibodies were loaded onto the column at a rate of 0.5 mL/min. To ensure the binding of the polyclonal antibodies to the packings, the column should be loaded twice in succession and the loading effluent should be retained. After washing the column with TBS buffer solution until Aλ, 280 nm was < 0.008, an elution buffer solution at Ph 2.7 was added to elute at a rate of 0.5 mL/min until all protein flew down. The eluent was collected into EP tubes of 1.5 mlwhich had been added with 100 µL of neutralizing buffer solution, mixed well and detected with pH test paper for the pH of the eluent. If the pH was lower than 7, it could be adjusted to about pH 7.4 with a neutralizing buffer so as to avoid the denaturation of the antibodies. Into the column was added 10 mL of elution buffer solution at pH 1.9 to collect the eluent according to the above method until Aλ 280 nm was < 0.008. The protein content in each tube was determined by a spectrophotometer.

### 2. Enzyme-linked immunosorbent assay (ELISA) verifying the binding of each hybridoma antibody to ITPRIPL1

In order to verify the binding capacity of each hybridoma antibody to ITPRIPL1, an enzyme-linked immunosorbent assay (ELISA) was performed to confirm the direct binding of each antibody to ITPRIPL1 protein. An ELISA special plate (costar, ME, USA) was used. Firstly, the plate was coated with 100 µl (1 µg/ml) of ITPRIPL1 recombinant protein (cusabio, Wuhan, China) in ELISA coating buffer (Solarbio, Beijing, China), while for the negative control, the plate was coated with 100 µl of coating buffer free of ITPRIPL1 recombinant protein. The plate was coated at 4°C overnight. After washing with PBST, they were blocked with 100 µl of 5% BSA dissolved in PBS (VWR, PA, USA) in an incubator at 37°C for 90 minutes. After washing with PBST, they were incubated in an incubator at 37°C for 60 minutes for binding. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

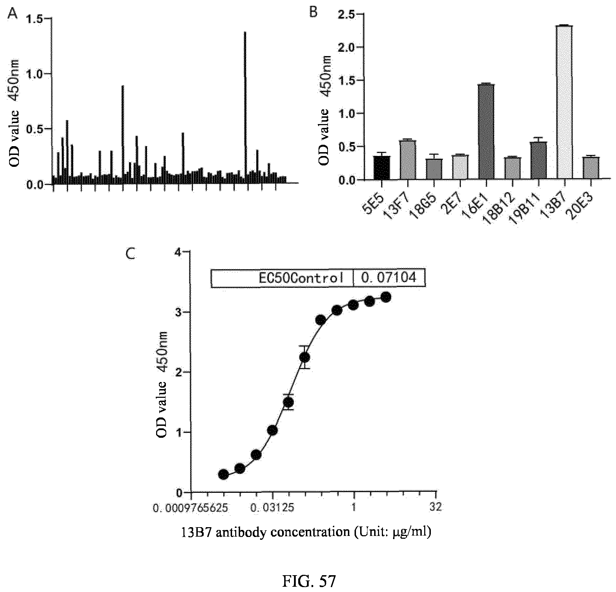

FIG. 57 shows the ELISA experimental results. The upper left figure shows the ELISA results of all the 100 resulting antibodies binding to the ITPRIPL1 protein, and the upper right figure shows the results of individual replicate experiments of 9 strains of antibodies with better binding selected from the results of the upper left figure. The lower figure shows the concentration-dependent binding curve of the 13B7 antibody. It was demonstrated from the experiment that, the capacity of binding to ITPRIPL1 varied among antibodies, indicating the heterogeneity among the antibodies, while 13B7 was the antibody with the strongest binding capacity. The above results demonstrated that each hybridoma can bind to ITPRIPL1 with varying binding capacities.

### 2. Flow cytometry (FACS) verifying the binding of each hybridoma antibody to ITPRIPL1

In order to further verify the binding capacity of each hybridoma antibody to ITPRIPL1, flow cytometry (FACS) was used to confirm the direct binding of each antibody to ITPRIPL1 protein. Jurkat cells (ATCC, VA, USA) with high endogenous expression of ITPRIPL1 were counted, and the cell number was adjusted to 1×10⁶/ml. Each 100 µl of the cells were added into the wells of a 96-well plate (Thermo Fisher, MA, USA). Into each well was respectively added hybridoma antibody or control serum at a final concentration of 1 µg/ml, and incubated in an incubator for 30 minutes. The 96-well plate was taken out, and the cells of each well were placed in EP tubes (Axygen, CA, USA) and centrifuged at 400 rcf for 5 minutes, and the supernatants were then discarded. The cells were resuspended and washed with 500 µl of cell staining buffer (Invitrogen, CA, USA), and centrifuged and washed again. Anti-mouse Fc segment-Alexa Fluor 488 antibodies (Invitrogen, CA, USA) were diluted with the cell staining buffer at 1:500. 200 µl of the mixture solution was added into each EP tube for resuspension, and incubated at room temperature for 30 minutes. After centrifugation at 400 rcf for 5 minutes, the supernatants were discarded, and the cells were resuspended and washed with 1 ml of the cell staining buffer; and centrifuged and washed again. 300 µl of the cell staining buffer was respectively added, and transferred into flow tubes (Falcon, NY, USA) for loading (Miltenyi Biotec, Cologne, Germany) to test.

FIG. 58 shows the FACS experimental results. It was demonstrated from the experiment that, the capacity of binding to ITPRIPL1 varied among antibodies, indicating the heterogeneity among the antibodies, wherein 13B7 was the antibody with the strongest binding capacity.

### Example 20: 13B7 antibody can specifically bind to ITPRIPL1 with high affinity

### 1. Flow cytometry (FACS) verifying the binding of 13B7 antibody to cells with different expression levels of ITPRIPL1

In order to further verify the binding capacity of 13B7 to ITPRIPL1, flow cytometry (FACS) was used to confirm the direct binding of 13B7 antibody to various cells with different expression levels of ITPRIPL1. The cells with different endogenous expression levels of ITPRIPL1, i.e., HCT116 (ATCC, VA, USA), A549 (ATCC, VA, USA), MC38 (Kerafast, MA, USA), Jurkat (ATCC, VA, USA), Raji cells (ATCC, VA, USA) as well as MC38-ITPRIPL1 stably transfected cell lines, were counted and the cell number was adjusted to 1 × 10⁶/ml. Each 100 µl of the cells were added into the wells of a 96-well plate (Thermo Fisher, MA, USA). Into each well was respectively added the 13B7 antibody at a final concentration of 1 µg/ml, and incubated in an incubator for 30 minutes. The 96-well plate was taken out, and the cells of each well were placed in EP tubes (Axygen, CA, USA) and centrifuged at 400 rcf for 5 minutes, and the supernatants were then discarded. The cells were resuspended and washed with 500 µl of cell staining buffer (Invitrogen, CA, USA), and centrifuged and washed again. Anti-mouse Fc segment-Alexa Fluor 488 antibodies (Invitrogen, CA, USA) were diluted with the cell staining buffer at 1:500. 200 µl of the mixture solution was added into each EP tube for resuspension, and incubated at room temperature for 30 minutes. After centrifugation at 400 ref for 5 minutes, the supernatants were discarded, and the cells were resuspended and washed with 1 ml of the cell staining buffer; and centrifuged and washed again. 300 µl of the cell staining buffer was respectively added, and transferred into flow tubes (Falcon, NY, USA) for loading (Miltenyi Biotec, Cologne, Germany) to test.

FIG. 59 shows the FACS experimental results, which show that the binding capacity of each cell line to the 13B7 antibody is substantially consistent with the expression of ITPRIPL1 protein in each cell line, indicating that the 13B7 antibody can specifically bind to ITPRIPL1 on the cell line expressing the ITPRIPL1.

### 2. Flow cytometry (FACS) verifies that the 13B7 antibody can bind to Jurkat cells in a concentration-dependent manner

In order to verify the binding capacity of 13B7 to ITPRIPL1, flow cytometry (FACS) was used to confirm the concentration-dependent binding of the 13B7 antibody to Jurkat cells. The Jurkat cells with high endogenous expression of ITPRIPL1 were counted, and the cell number was adjusted to 1×10⁶/ml. Each 100 µl of the cells were added into the wells of a 96-well plate (Thermo Fisher, MA, USA). Into each well was respectively added the 13B7 antibody at a final concentration of 0.0625/0.125/0.25/0.5/1/2 µg/ml, and incubated in an incubator for 30 minutes. The 96-well plate was taken out, and the cells of each well were placed in EP tubes (Axygen, CA, USA) and centrifuged at 400 rcf for 5 minutes, and the supernatants were then discarded. The cells were resuspended and washed with 500 µl of cell staining buffer (Invitrogen, CA, USA), and centrifuged and washed again. Anti-mouse Fc segment-Alexa Fluor 488 antibodies (Invitrogen, CA, USA) were diluted with the cell staining buffer at 1:500. 200 µl of the mixture solution was added into each EP tube for resuspension, and incubated at room temperature for 30 minutes. After centrifugation at 400 rcf for 5 minutes, the supernatants were discarded, and the cells were resuspended and washed with 1 ml of the cell staining buffer; and centrifuged and washed again. 300 µl of the cell staining buffer was respectively added, and transferred into flow tubes (Falcon, NY, USA) for loading (Miltenyi Biotec, Cologne, Germany) to test.

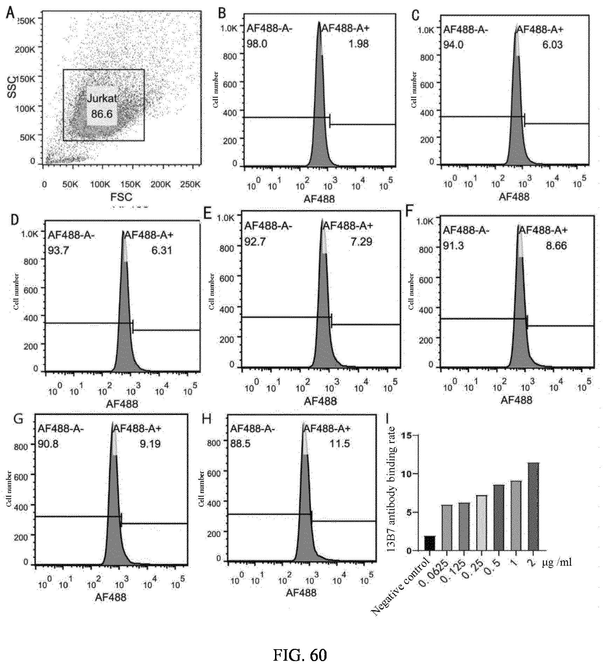

FIG. 60 shows the FACS experimental results, showing that Jurkat cells can bind to the 13B7 antibody with high affinity in a concentration-dependent manner.

### 3. Protein Western Blot verifies that the 13B7 antibody can specifically bind to the ITPRIPL1 protein of cells with different expression levels of ITPRIPL1

Jurkat, HCT116, MC38 cells with different endogenous expression profiles of ITPRIPL1 were counted. 1×10⁶ cells were respectively taken, washed with PBS and centrifuged, and then lysed with 60 µl of RIPA lysate (Beyotime, Shanghai, China) and a triple of protease inhibitor-phosphatase inhibitor-PMSF (Consun, Shanghai, China) at a ratio of 1:100 on ice for 15 minutes. They were frozen and thawed with liquid nitrogen for three cycles. After centrifugation, mixing with loading buffer (Beyotime, Shanghai, China) and denaturation in a metal bath at 100°C, an input level of protein sample was produced. And then, 12.5% of PAGE gel (Epizyme, Shanghai, China) was formulated in a gel plate (Bio-Rad, CA, USA) according to the instructions. The formulated gel was placed in an electrophoresis cell (Bio-Rad, CA, USA), the power (Bio-Rad, CA, USA) was turned on to let the strips run through the stacking gel at a constant voltage of 80 V and run through the separating gel at a constant voltage of 120 V. When the strips run to the bottom of the separating gel, the film was transferred in an electrophoretic transfer cell (Bio-Rad, CA, USA) by a method of tank blot at a constant current of 350 mA for 90 minutes. After the film transfer was completed, the film was sheared according to the mass of the ITPRIPL1 protein and the internal reference GAPDH protein. After blocking with rapid blocking buffer (Epizyme, Shanghai, China) for 10 minutes, the corresponding ITPRIPL1 and GAPDH strips were respectively diluted with the 13B7 antibody at a final concentration of 1 µg/ml and incubated with GAPDH-HRP antibodies (Consun, Shanghai, China) at 4°C overnight. The next day, after washing with TBST, the strips were incubated with specific anti-mouse secondary antibodies (Consun, Shanghai, China) that were diluted with 5% skimmed milk (Sangon, Shanghai, China) dissolved in TBS at room temperature for 1 hour, then washed with TBST, placed in a hybrid luminescent fluid (Share-Bio, Shanghai, China) for 1 minute, and exposed under a Gel-Imager (Bio-Rad, CA, USA).

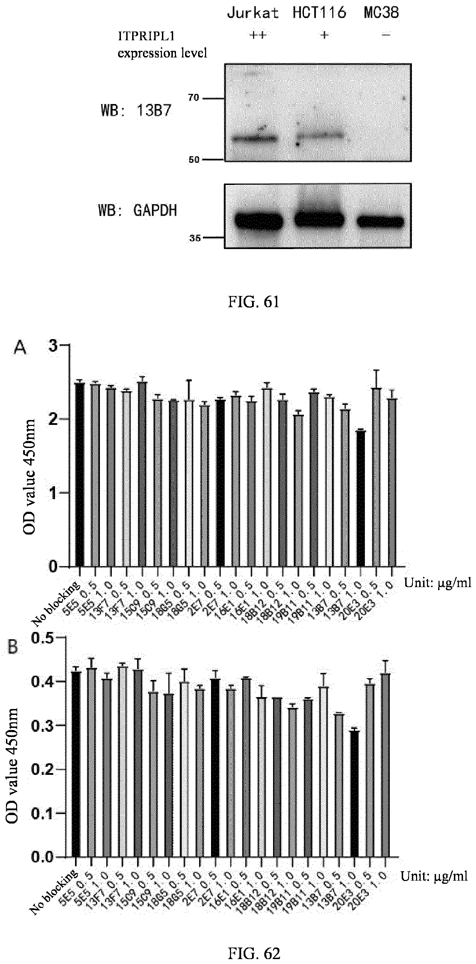

FIG. 61 shows the Western Blot experimental results. As shown in the figure, according to the indication of the GAPDH internal reference, the 13B7 antibody can specifically show a band at the location of the molecular weight of the ITPRIPL1 protein, which is consistent with the endogenous expression of ITPRIPL1 in the various cells, indicating that the 13B7 antibody can specifically bind to ITPRIPL1 protein.

### Example 21: Determination of the blocking effect of each hybridoma antibody on the binding of ITPRIPL1 to CD3E/SEMA3G

In order to verify whether there is a blocking effect of each hybridoma antibody on the binding of ITPRIPL1 to CD3E or SEMA3G, an enzyme-linked immunosorbent assay (ELISA) was used to verify the blocking effect of the antibodies. An ELISA special plate (costar, ME, USA) was used. Firstly, the plate was coated with 1 µg/ml of ITPRIPL1-Fc-tagged recombinant protein (Abclonal, Wuhan, China) or 1 µg/ml of CD3E-Fc-tagged protein (Aero, Beijing, China) dissolved in 100 µl of ELISA coating buffer (Solarbio, Beijing, China), while for the negative control, the plate was coated with 100 µl of coating buffer free of proteins. The plate was coated at 4°C overnight. After washing with PBST, they were blocked with 100 µl of 5% BSA dissolved in PBS (VWR, PA, USA) in an incubator at 37°C for 90 minutes. After washing with PBST, into the ITPRIPL1-Fc protein-coated wells were added SEMA3G-His-tagged protein (cusabio, Wuhan, China) at a final concentration of 1 µg/ml and hybridoma antibodies at a final concentration of 1/2 µg/ml at the same time; into the CD3E-Fc protein-coated wells were added ITPRIPL1-His-tagged protein (cusabio, Wuhan, China) at a final concentration of 1 µg/ml and hybridoma antibodies at a final concentration of 1/2 µg/ml at the same time; they were bound in an incubator at 37°C for 60 minutes. After washing with PBST, they were incubated with PBS-diluted specific anti-His segment antibodies (Abeam, MA, USA) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 62 shows the ELISA experimental results. The upper figure shows the blocking of each hybridoma antibody during the ITPRIPL1-CD3E binding, and the lower figure shows the blocking of each hybridoma antibody during the ITPRIPL1-SEMA3G binding. The experimental results show that the various hybridoma antibodies can block the binding of ITPRIPL1 to CD3E or SEMA3G to different extent, indicating the heterogeneity among the antibodies, in which the 18B12 and 13B7 antibodies have the best binding effect.

### Example 22: monoclonal antibodies can bind to ITPRIPL1 with higher affinity

1. Immunosorbent assay (ELISA) verifies the binding capacity of each monoclonal antibody to ITPRIPL1

After monoclonal purification of each hybridoma antibody, 16 monoclonal antibodies with better growth were obtained. In order to verify the binding capacity of each monoclonal antibody to ITPRIPL1, an enzyme-linked immunosorbent assay (ELISA) was used to verify the direct binding of each antibody to the ITPRIPL1 protein. An ELISA special plate (costar, ME, USA) was used. Firstly, the plate was coated with 100 µl (1 µg/ml) of ITPRIPL1 recombinant protein (cusabio, Wuhan, China) in ELISA coating buffer (Solarbio, Beijing, China), while for the negative control, the plate was coated with 100 µl of coating buffer free of protein. The plate was coated at 4°C overnight. After washing with PBST, they were blocked with 100 µl of 5% BSA dissolved in PBS (VWR, PA, USA) in an incubator at 37°C for 90 minutes. After washing with PBST, different monoclonal antibodies at a final concentration of 1 µg/ml were added and bound in an incubator at 37°C for 60 minutes. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

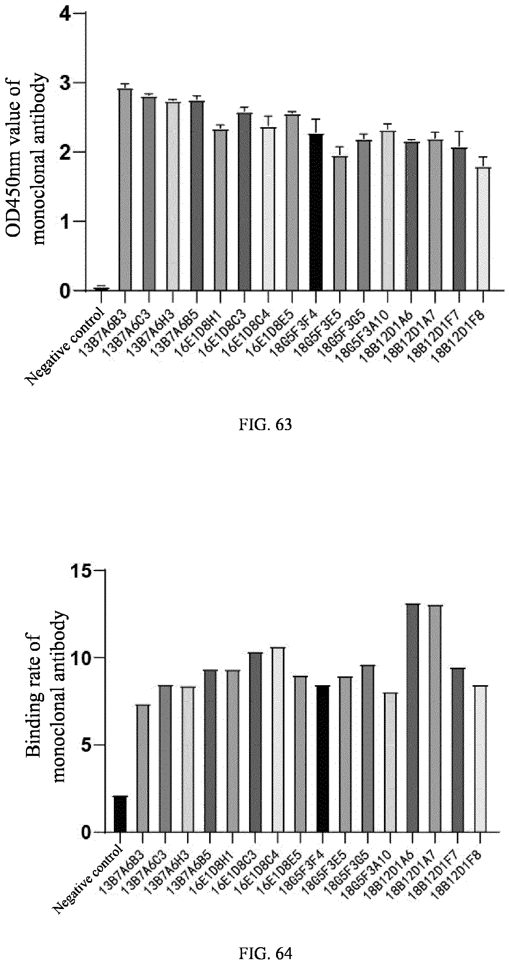

FIG. 63 shows the ELISA experimental results. The experimental results show that there are some differences in the binding capacity of each monoclonal antibody to ITPRIPL1, while the overall binding capacity to ITPRIPL1 is significantly stronger than that before monoclonal purification, indicating that the monoclonal antibody can bind to ITPRIPL1 with higher affinity.

### 2. Flow cytometry (FACS) verifying the binding of each monoclonal antibody to ITPRIPL1

In order to further verify the binding capacity of each monoclonal antibody to ITPRIPL1, flow cytometry (FACS) was used to confirm the direct binding of each antibody to ITPRIPL1 protein. Jurkat cells with high endogenous expression of ITPRIPL1 were counted, and the cell number was adjusted to 1×10⁶/ml. Each 100 µl was added into a 96-well plate (Thermo Fisher, MA, USA). Into each well was respectively added monoclonal antibody or control serum at a final concentration of 1 µg/ml, and incubated in an incubator for 30 minutes. The 96-well plate was taken out, and each well of cells was placed in EP tubes (Axygen, CA, USA) and centrifuged at 400 rcf for 5 minutes, and the supernatants were then discarded. The cells were resuspended and washed with 500 µl of cell staining buffer (Invitrogen, CA, USA), and centrifuged and washed again. Anti-mouse Fc segment-Alexa Fluor 488 antibodies (Invitrogen, CA, USA) were diluted with the cell staining buffer at 1 :500. 200 µl of the mixture solution was added into each EP tube for resuspension, and incubated at room temperature for 30 minutes. After centrifugation at 400 rcf for 5 minutes, the supernatant was discarded, and the cells were resuspended and washed with 1 ml of the cell staining buffer; and centrifuged and washed again. 300 µl of the cell staining buffer was respectively added, and transferred into flow tubes (Falcon, NY, USA) for loading (Miltenyi Biotec, Cologne, Germany) to test.

The results were shown in FIG. 64, showing that there are some differences in the binding capacity of each monoclonal antibody to ITPRIPL1, indicating the heterogeneity among the antibodies, while the overall binding capacity is stronger than that before monoclonal purification. The above results demonstrate that each hybridoma can bind to the ITPRIPL1 protein with different binding capacities, and the binding capacity to ITPRIPL1 is stronger after monoclonal purification.

### Example 23: Determination of the blocking effect of each hybridoma antibody on the binding of ITPRIPL1 to CD3E/SEMA3G

In order to verify whether there is a blocking effect of the resulting monoclonal antibodies on the binding of ITPRIPL1 to CD3E or SEMA3G, an enzyme-linked immunosorbent assay (ELISA) was used to verify the blocking effect of the antibodies. An ELISA special plate (costar, ME, USA) was used. Firstly, the plate was coated with ITPRIPL1-Fc-tagged recombinant protein (Abclonal, Wuhan, China) at a final concentration of 1 µg/ml or CD3E-Fc-tagged protein (Aero, Beijing, China) at a final concentration of 1 µg/ml which was dissolved in 100 µl of ELISA coating buffer (Solarbio, Beijing, China), while for the negative control, the plate was coated with 100 µl of coating buffer free of proteins. The plate was coated at 4°C overnight. After washing with PBST, they were blocked with 100 µl of 5% BSA dissolved in PBS (VWR, PA, USA) in an incubator at 37°C for 90 minutes. After washing with PBST, into the ITPRIPL1-Fc protein-coated wells were added SEMA3G-His-tagged protein (cusabio, Wuhan, China) at a final concentration of 1 µg/ml and monoclonal antibodies at a final concentration of 1 µg/ml at the same time; into the CD3E-Fc protein-coated wells were added ITPRIPL1-His-tagged protein (cusabio, Wuhan, China) at a final concentration of 1 µg/ml and monoclonal antibodies at a final concentration of 1/2 µg/ml at the same time; they were bound in an incubator at 37°C for 60 minutes. After washing with PBST, they were incubated with PBS-diluted specific anti-His segment antibodies (Abeam, MA, USA) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 65 shows the ELISA experimental results. The experimental results show that, there are still some differences in the blocking effect of each monoclonal antibody on the binding of ITPRIPL1 to CD3E/SEMA3G, wherein 13B7A6H3/18B12D1A6/18B12D1F7 has a better blocking effect, which is consistent with the conclusion before monoclonal purification.

### Example 24: Determination of the binding of hybridoma antibodies to monoclonal antibody and polypeptide segments

Based on the protein sequence of the extracellular segment of ITPRIPL1, polypeptide segments with 1/3 overlapping sequence were designed, with a total of 17 segments (Genscript, Nanjing, China). Their sequences were shown in SEQ ID NOs: 42-58. The resulting polypeptide segments were dissolved in DMSO at a final concentration of 400 µg/ml. An enzyme-linked immunosorbent assay (ELISA) was used to verify each hybridoma antibody, and 4 strains of monoclonal antibodies were selected to verify the binding of polypeptide segments. An ELISA special plate (costar, ME, USA) was used. Firstly, the plate was coated with polypeptide segments at a final concentration of 1 µg/ml in 100 µl of ELISA coating buffer (Solarbio, Beijing, China), while for the negative control, the plate was coated with 100 µl of the coating buffer free of protein. The plate was coated at 4°C overnight. After washing with PBST, they were blocked with 100 µl of 5% BSA dissolved in PBS (VWR, PA, USA) in an incubator at 37°C for 90 minutes. After washing with PBST, different hybridoma antibodies or monoclonal antibodies at a final concentration of 1 µg/ml were added and bound in an incubator at 37°C for 60 minutes. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

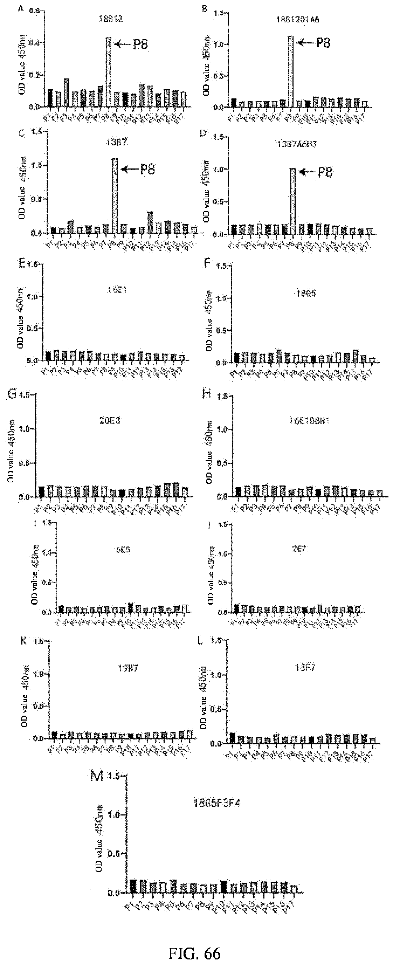

FIG. 66 shows the ELISA experimental results. When the binding reading to a specific polypeptide segment is more than three times higher than that of the background and other polypeptide segments, it is considered to be bound to a specific peptide segment. The experimental results show that, the monoclonal antibodies produced by 13B7/18B12 with good blocking effect all bind to P8, while hybridoma antibodies and monoclonal antibodies with no or poor blocking effect have no specific binding peptide segments, indicating that P8 is the dominant binding site.

### Example 25: Determination of the promoting effect of ITPRIPL1 monoclonal antibodies on the killing of tumor cells by PBMC cells

Flow cytometry demonstrates that monoclonal antibodies with the effect of blocking the binding of ITPRIPL1 to CD3E/SEMA3G can promote the killing of tumor cells by PBMCs.

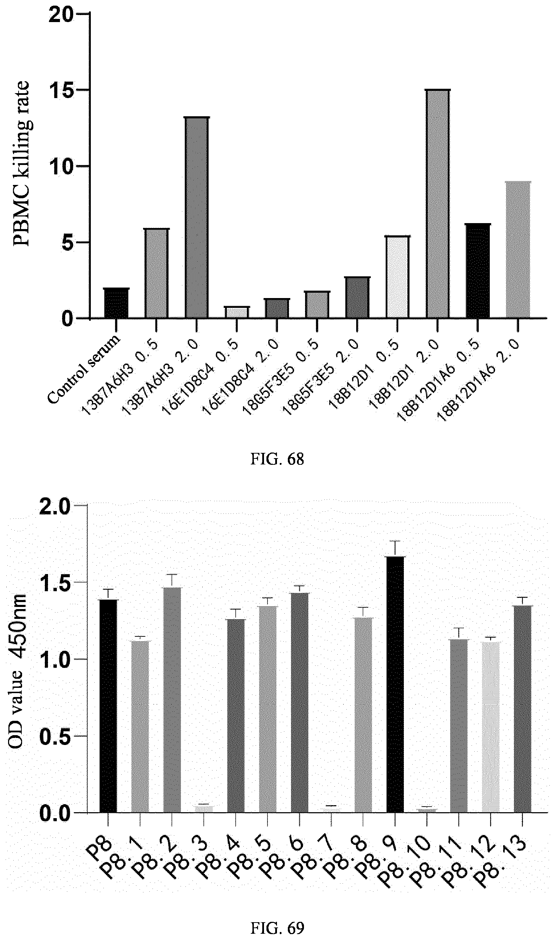

Resuscitated PBMC cells were activated with 1 µg/mL of Anti-CD3/CD28 (Invitrogen, CA, USA) 24 hours in advance. PBMC cells and Raji cells were counted, and the cell number was respectively adjusted to 4×10⁶/ml or 1×10⁶/ml. Each 100 µl was added into a 96-well plate (Thermo Fisher, MA, USA). At the same time, two concentrations of different monoclonal antibodies or control serum were added, mixed and then co-incubated in an incubator for 6 hours. The 96-well plate was taken out, and each well of cells was placed in EP tubes (Axygen, CA, USA) and centrifuged at 400 rcf for 5 minutes, and the supernatants were then discarded. The cells were resuspended and washed with 500 µl of cell staining buffer (Invitrogen, CA, USA), and centrifuged and washed again. After centrifugation at 400 rcf for 5 minutes, the supernatant was discarded, and the cells were resuspended and washed with 1 ml of binding buffer (Beyotime, Shanghai, China); and centrifuged and washed again. Experimental groups and a control group were set, into which were optionally added 100 µl of the binding buffer, 5 µl of Annexin V-FITC (Beyotime, Shanghai, China) and 10 µl of PI (Beyotime, Shanghai, China) respectively, and incubated at room temperature for 15 minutes. 400 µl of the binding buffer was further respectively added, and transferred into flow tubes (Falcon, NY, USA) for loading (Miltenyi Biotec, Cologne, Germany) to test, with the results shown in FIG. 67 and 68. The results show that, compared with the control, the monoclonal antibody 13B7A6H3/18B12D1A6, which has the effect of blocking the binding of ITPRIPL1 to CD3E/SEMA3G and can bind to P8 (SEQ ID NO: 49), significantly increases the killing of Raji cells by PBMCs.

It can be known from sequencing that, for the monoclonal antibody 13B7A6H3, the heavy chain sequence is as set forth in SEQ ID NO: 22, the light chain sequence is as set forth in SEQ ID NO: 23, the sequence of the heavy chain variable region VH is as set forth in SEQ ID NO: 24, the sequence of the light chain variable region VL is as set forth in SEQ ID NO: 25, the sequence of the heavy chain complementarity determining region HCDR1 is as set forth in SEQ ID NO: 26, the sequence of the heavy chain complementarity determining region HCDR2 is as set forth in SEQ ID NO: 27, the sequence of the heavy chain complementarity determining region HCDR3 is as set forth in SEQ ID NO: 28, the sequence of the light chain complementarity determining region LCDR1 is as set forth in SEQ ID NO: 29, the sequence of the light chain complementarity determining region LCDR2 is KV, and the sequence of the light chain complementarity determining region LCDR3 is as set forth in SEQ ID NO: 31; for the monoclonal antibody 18B12D1A6, the heavy chain sequence is as set forth in SEQ ID NO: 32, the light chain sequence is as set forth in SEQ ID NO: 33, the sequence of the heavy chain variable region VH is as set forth in SEQ ID NO: 34, the sequence of the light chain variable region VL is as set forth in SEQ ID NO: 35, the sequence of the heavy chain complementarity determining region HCDR1 is as set forth in SEQ ID NO: 36, the sequence of the heavy chain complementarity determining region HCDR2 is as set forth in SEQ ID NO: 37, the sequence of the heavy chain complementarity determining region HCDR3 is as set forth in SEQ ID NO: 38, the sequence of the light chain complementarity determining region LCDR1 is as set forth in SEQ ID NO: 39, the sequence of the light chain complementarity determining region LCDR2 is KV, and the sequence of the light chain complementarity determining region LCDR3 is as set forth in SEQ ID NO: 41.

By analyzing the similarity of the antibody sequences (FIG. 65C), it can be seen that the sequence of the light chain CDR1 is xSLxNSKGNTH (x represents any amino acids) and it is 81.8% similar to the 13B7A6H3 light chain CDR1, the sequence of the light chain CDR3 is SQSTHxPYT and it is 87.5% similar to the 13B7A6H3 light chain CDR1. Other CDR sequences are the same. Therefore, it can be predicted that, when the similarity of the light chains CDR1 and CDR3 to 13B7A6H3 is higher than 80%, and other CDR sequences are the same as 13B7A6H3, the antibody can still have the activity of binding ITPRIPL1. If the overall similarity of all the CDR sequences is compared, it can be seen that sequences with an overall similarity of 94% to the heavy chain CDR1, CDR2, CDR3 and the light chain CDR1, CDR2, CDR3 of 13B7A6H3 can have the activity of binding ITPRIPL1.

### Example 26: Determination of the effect of point mutation of polypeptide segments on the binding of monoclonal antibodies

### Immunosorbent assay (ELISA) verifying the effect of point mutation of polypeptide segments on monoclonal antibodies

The specific amino acid sequences of the previously obtained polypeptide segment P8 was mutated to alanine (A) one by one, and the original alanine sites were not mutated, and the resulting sequences were as set forth in SEQ ID NOs: 59-71. The resulting polypeptide segments and non-mutated polypeptide segments were dissolved in DMSO at a final concentration of 400 µg/ml, and the binding to the 13B7A6H3 monoclonal antibody was verified by enzyme-linked immunosorbent assay (ELISA). An ELISA special plate (costar, ME, USA) was used. Firstly, the plate was coated with polypeptide segments at a final concentration of 1 µg/ml in 100 µl of ELISA coating buffer (Solarbio, Beijing, China), while for the negative control, the plate was coated with 100 µl of the coating buffer free of protein. The plate was coated at 4°C overnight. After washing with PBST, they were blocked with 100 µl of 5% BSA dissolved in PBS (VWR, PA, USA) in an incubator at 37°C for 90 minutes. After washing with PBST, 13B7A6H3 monoclonal antibody at a final concentration of 1 µg/ml was added and bound in an incubator at 37°C for 60 minutes. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 69 shows the ELISA experimental results. The experimental results show that, after mutations at sites 3 (L), 7 (F), 10 (R) in P8, the 13B7A6H3 antibody no longer binds to polypeptide segments, while mutations at other sites does not affect the binding, indicating that the sites 3 (L), 7 (F), 10 (R) are the key sites for the binding of the 13B7A6H3 monoclonal antibody to ITPRIPL1 protein.

### Example 27: Competitive analysis of ITPRIPL1 monoclonal antibody clusters by epitope mapping

The cluster analysis of ITPRIPL1 monoclonal antibodies was performed by epitope mapping.

The resulting ITPRIPL1 monoclonal antibodies were analyzed by epitope mapping for the competition among the currently obtained ITPRIPL1 monoclonal antibodies and clustered. An ELISA special plate (costar, ME, USA) was used. Firstly, the plate was coated with ITPRIPL1 protein at a final concentration of 0.5 µg/ml in 100 µl of ELISA coating buffer (Solarbio, Beijing, China), while for the negative control, the plate was coated with 100 µl of the coating buffer free of protein. The plate was coated at 4°C overnight. After washing with PBST, they were blocked with 100 µl of 5% BSA dissolved in PBS (VWR, PA, USA) in an incubator at 37°C for 90 minutes. After washing with PBST, 0.5 µg/ml of each ITPRIPL1 monoclonal antibody was added, and at the same time each ITPRIPL1 monoclonal antibody at a final concentration of 0.5 µg/ml was added crossover, and bound in an incubator at 37°C for 60 minutes. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 70 shows the ELISA experimental results of the epitope mapping. The experimental results show that, there is competition between 13B7 and 18B12, there is competition between 13H10, 15C9, and 16E1, and there is no competition between other antibodies, indicating that the current ITPRIPL1 monoclonal antibodies can be divided into four clusters: (1) 13B7, 18B12; (2) 13H10, 15C9, 16E1; (3) 13E8; (4) 18G5.

### Example 28: Construction of humanized ITPRIPL1 antibody and determination of binding site

### 1. Construction of humanized ITPRIPL1 antibody

According to the results of the previous antibody sequencing, the paired sequences SEQ ID NO: 73-82 corresponding to the 13B7A6H3 monoclonal antibody were optimized and re-encoded by using humanized gene expression to obtain a corresponding humanized sequence. The obtained sequence was inserted into the corresponding sequence vector of human Fc segments to obtain a humanized ITPRIPL1 antibody sequence plasmid. The humanized ITPRIPL1 antibody sequence plasmid was transfected into HEK293 cells and cultured. 72 hours later, the supernatant was harvested, mixed with the equilibrated protein A magnetic beads at room temperature for 2 hours, and then flowed through a chromatographic column to be equilibrated/washed and eluted, and the product obtained after neutralization was the humanized ITPRIPL1 antibody.

### 2. Determination of binding activity and site of humanized ITPRIPL1 antibody

An ELISA special plate (costar, ME, USA) was used. Firstly, the plate was coated with ITPRIPL1 P8 polypeptide at a final concentration of 1 µg/ml in 100 µl of ELISA coating buffer (Solarbio, Beijing, China), while for the negative control, the plate was coated with 100 µl of the coating buffer free of protein, and for the positive control, the plate was coated with human ITPRIPL1 P8 polypeptide. The plate was coated at 4°C overnight. After washing with PBST, they were blocked with 100 µl of 5% BSA dissolved in PBS (VWR, PA, USA) in an incubator at 37°C for 90 minutes. After washing with PBST, chimeric and humanized 13B7A6H3 monoclonal antibodies at a final concentration of 0.5 µg/ml were added and bound in an incubator at 37°C for 60 minutes. After washing with PBST, they were incubated with PBS-diluted specific anti-human Fc segment antibodies (Abeam, MA, USA) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

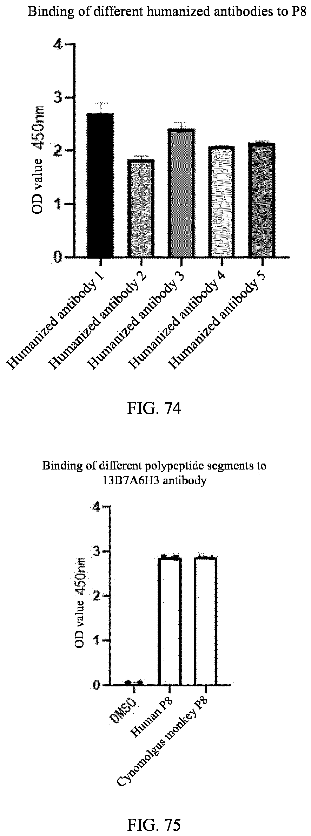

FIG. 74 shows the ELISA experimental results of the binding of humanized antibodies to ITPRIPL1 P8 polypeptide segments. The experimental results show that the resulting humanized antibodies all have activities and can specifically bind to ITPRIPL1 polypeptide segment sites to which 13B7A6H3 binds, demonstrating that the antibodies have been constructed successfully.

### Example 29: Determination of the binding of monoclonal ITPRIPL1 antibodies to cynomolgus monkey ITPRIPL1 protein

### Immunosorbent assay (ELISA) verifies that 13B7A6H3 monoclonal antibodies can bind to the corresponding polypeptide segments of cynomolgus monkey ITPRIPL1.

According to NCBI protein database, it can be known that the sequence of cynomolgus monkey ITPRIPL1 protein is SEQ ID NO: 72; wherein the sequence of the cynomolgus monkey corresponding polypeptide segment to human ITPRIPL1 P8 is SEQ ID NO: Corresponding polypeptide was constructed according to the cynomolgus monkey corresponding polypeptide segment. An ELISA special plate (costar, ME, USA) was used. Firstly, the plate was coated with cynomolgus monkey ITPRIPL1 polypeptide at a final concentration of 1 µg/ml in 100 µl of ELISA coating buffer (Solarbio, Beijing, China), while for the negative control, the plate was coated with 100 µl of the coating buffer free of protein, and for the positive control, the plate was coated with human ITPRIPL1 P8 polypeptide. The plate was coated at 4°C overnight. After washing with PBST, they were blocked with 100 µl of 5% BSA dissolved in PBS (VWR, PA, USA) in an incubator at 37°C for 90 minutes. After washing with PBST, 13B7A6H3 monoclonal antibodies at a final concentration of 0.5 µg/ml were added and bound in an incubator at 37°C for 60 minutes. After washing with PBST, they were incubated with PBS-diluted specific anti-mouse Fc segment antibodies (Consun, Shanghai, China) in an incubator at 37°C for 30 minutes for binding. After washing with PBST, a color developing solution (Invitrogen, CA, USA) was added at 100 µl per well, and the plate was placed in the incubator to react for 5-30 minutes, then 50 µl of stop solution (Sangon, Shanghai, China) was further added, and the plate was placed under a microplate reader (Thermo Fisher, MA, USA) for color development reading at 450 nm.

FIG. 75 shows the ELISA experimental results of the binding of the cynomolgus monkey ITPRIPL1 polypeptides to the ITPRIPL1 monoclonal antibodies. The experimental results show that, there is no significant difference in the binding of cynomolgus monkey ITPRIPL1 polypeptide and human ITPRIPL1 P8 polypeptide to the 13B7A6H3 monoclonal antibody, indicating that 13B7A6H3 monoclonal antibody can bind to cynomolgus monkey ITPRIPL1 protein.

In summary, the present application discloses a newly identified native membrane protein ITPRIPL1 that binds the CD3ε extracellular domain, and also discloses the altered signaling that occurs following the binding of ITPRIPL1 to CD3ε and controls T cell activation. This indicates that, antibodies that have been identified in the past to bind CD3ε may mimic or affect the natural ligand function of ITPRIPL1 to some extent. The discovery of CD3ε and ITPRIPL1, a pair of immune checkpoint receptor ligands, brings a new insight to the in-depth understanding of the maintenance of the body's immune homeostasis and the immune evasion mechanism of tumor cells.

Although the content of the present application has been described in detail through the preferred examples described above, it should be appreciated that the above description should not be construed as limiting the present application. Various modifications and substitutions to the present application will be apparent to those skilled in the art after reading the foregoing. Therefore, the protection scope of the present application shall be defined by the appended claims.

## Claims

1. A use of a regulator of ITPRIPL1 in the preparation of a drug that regulates immune responses or resists tumors, wherein, the regulator is used to increase or decrease the expression or function of the ITPRIPL1 gene or protein in an organism.

2. The use of a regulator of ITPRIPL1 in the preparation of a drug that regulates immune responses or resists tumors according to claim 1, wherein, the regulator comprises any one of the following:
(1) a gene editing system that enables the knockout or mutation of the ITPRIPL1 gene in cells;
(2) an RNA molecule that reduces the expression level of the ITPRIPL1 gene;
(3) a nucleic acid molecule for being introduced into a cell, the nucleic acid molecule encodes ITPRIPL1 and increases the expression level of ITPRIPL1;
(4) an isolated ITPRIPL1 recombinant protein;
(5) an antibody that recognizes and binds to the ITPRIPL1.

3. The use of a regulator of ITPRIPL1 in the preparation of a drug that regulates immune responses or resists tumors according to claim 2, wherein:
the gene editing system is a CRISPR/Cas9 gene editing system; a target sequence used in the CRISPR/Cas9 gene editing system is selected from any one sequence as set forth in SEQ ID NOs: 11-13, and an oligomeric DNA sequence for encoding sgRNA is selected from SEQ ID NOs:14-19;
the nucleic acid molecule comprises: a sequence as set forth in SEQ ID NO: 8, SEQ ID NO: 9 or SEQ ID NO: 10;
the ITPRIPL1 recombinant protein comprises: a functional fragment capable of binding to CD3ε or NRP2 protein in an extracellular domain of the ITPRIPL1 protein;
the antibody that recognizes and binds to ITPRIPL1 is a polyclonal antibody, a monoclonal antibody, a single-stranded antibody, an antigen binding domain, a bispecific antibody, a multi-specific antibody, or an antigen binding portion in a chimeric antigen receptor.

4. The use of a regulator of ITPRIPL1 in the preparation of a drug that regulates immune responses or resists tumors according to claim 3, wherein, the sequence of the functional fragment is selected from any one of SEQ ID NO: 1 to SEQ ID NO: 4, or a derivative sequence thereof, the derivative sequence comprises DRMDLDTLARSRQLEKRMSEEMRxLEMEFEERxxxAExxQKxENxWxGxTSxDQ ("x" is any amino acid), and the derivation method comprises: substituting, deleting or inserting 1-10 amino acids without changing the function of the sequence; the derivative sequence is preferably DRMDLDTLARSRQLEKRMSEEMRxLEMEFEERxxxAExxQKxENxWxGxTSxDQ ("x" is any amino acid).

5. The use of a regulator of ITPRIPL1 in the preparation of a drug that regulates immune responses or resists tumors according to claim 3 or 4, wherein, the ITPRIPL1 recombinant protein forms a fusion protein with an antibody constant region, or forms a fusion protein with a coagulation factor; alternatively, the ITPRIPL1 recombinant protein is modified by means of: polyethylene glycol modification, glycosylation modification, polysialic acid modification, fatty acid modification, KLH modification, biotin modification.

6. The use of a regulator of ITPRIPL1 in the preparation of a drug that regulates immune responses or resists tumors according to claim 3, wherein, the nucleic acid molecule is introduced into the cell through a drug delivery system which comprises recombinant expression vectors, viruses, lipidosome or nanomaterials.

7. The use of a regulator of ITPRIPL1 in the preparation of a drug that regulates immune responses or resists tumors according to claim 1, wherein, the regulation of immune responses comprises: regulating the functions of antigen presenting cells and T lymphocytes during the processes of autoimmune responses, transplant rejection-suppressing immune responses, allergies, anti-infection immune responses, anti-tumor immune responses.

8. The use of a regulator of ITPRIPL1 in the preparation of a drug that regulates immune responses or resists tumors according to claim 7, wherein, the immune responses comprise: type I diabetes, immunologic infertility, rejection after organ transplantation, allergies, systemic inflammation or cytokine storm, infection.

9. The use of a regulator of ITPRIPL1 in the preparation of a drug that regulates immune responses or resists tumors according to claim 1, wherein, the tumors are ITPRIPL1-associated solid tumors or hematological tumors; the solid tumors comprise: glioma, lung cancer, head and neck cancer, gastric cancer, colorectal cancer, thyroid cancer, esophagus cancer, urothelial carcinoma, testicular cancer, breast cancer, cervical cancer, endometrial cancer, melanoma, pancreatic cancer or liver cancer; the hematological tumors comprise: leukemia or lymphoma.

10. A pharmaceutical composition, wherein, comprising a regulator used in the use according to any one of claims 1 to 9, and a pharmaceutically acceptable carrier.

11. An isolated ITPRIPL1 recombinant protein, wherein, the recombinant protein is a functional fragment capable of binding to CD3ε or NRP2 protein in the extracellular domain of the ITPRIPL1 protein.

12. A use of an antibody that recognizes and binds to ITPRIPL1 in the preparation of a drug that regulates immune responses or resists tumors, wherein, the antibody recognizes and binds to the extracellular domain of the ITPRIPL1 protein.

13. Application of an antibody that specifically recognizes ITPRIPL1 for marking boundaries between cancer tissues and para-cancerous tissues in primary lesions, boundaries between cancer cells that have metastasized to lymph nodes and normal lymphatic tissues, boundaries between cancer cells with distant metastases and normal tissues of the metastatic organ, and marking living cancer tissue cells in other biological samples.

14. The application according to claim 13, wherein, the cancers comprise thyroid cancer, breast cancer, colorectal cancer, lung cancer, esophagus cancer, kidney cancer and other ITPRIPL1-associated tumors.

15. An isolated antigenic ITPRIPL1-binding protein, being capable of binding to an amino acid sequence as set forth in SEQ ID NO: 49 or SEQ ID NO: 1 in the antigenic ITPRIPL 1.

16. The isolated antigenic ITPRIPL1-binding protein according to claim 15, comprising a heavy chain variable region and a light chain variable region, wherein, the heavy chain variable region comprises HCDR1, HCDR2 and HCDR3 in the heavy chain variable region VH as set forth in any one of amino acid sequence SEQ ID NO: 24 or SEQ ID NO: 34; the light chain variable region comprises LCDR1, LCDR2 and LCDR3 in the light chain variable region VL as set forth in any one of amino acid sequence SEQ ID NO: 25 or SEQ ID NO: 35.

17. The isolated antigenic ITPRIPL1-binding protein according to claim 16, wherein the amino acid sequence of the HCDR1 is as set forth in SEQ ID NO: 26, the amino acid sequence of the HCDR2 is as set forth in SEQ ID NO: 27, and the amino acid sequence of the HCDR3 is as set forth in SEQ ID NO: 28.

18. The isolated antigenic ITPRIPL1-binding protein according to claim 16, wherein the amino acid sequence of the HCDR1 is as set forth in SEQ ID NO: 36, the amino acid sequence of the HCDR2 is as set forth in SEQ ID NO: 37, and the amino acid sequence of the HCDR3 is as set forth in SEQ ID NO: 38.

19. The isolated antigenic ITPRIPL1-binding protein according to claim 16, wherein the amino acid sequence of the LCDR1 is more than 80% similar to an amino acid sequence as set forth in SEQ ID NO: 29, the amino acid sequence of the LCDR2 is KV, and the amino acid sequence of the LCDR3 is more than 80% similar to an amino acid sequence as set forth in SEQ ID NO: 31.

20. The isolated antigenic ITPRIPL1-binding protein according to claim 16, wherein the amino acid sequence of the LCDR1 is as set forth in any one of SEQ ID NO: 29 or SEQ ID NO: 39, the amino acid sequence of the LCDR2 is KV, and the amino acid sequence of the LCDR3 is as set forth in any one of SEQ ID NO: 31 or SEQ ID NO: 41.

21. The isolated antigenic ITPRIPL1-binding protein according to claim 16, wherein, the amino acid sequence of the LCDR1 is as set forth in SEQ ID NO: 29, the amino acid sequence of the LCDR2 is KV, and the amino acid sequence of the LCDR3 is as set forth in SEQ ID NO: 31; alternatively, the amino acid sequence of the LCDR1 is as set forth in SEQ ID NO: 39, the amino acid sequence of the LCDR2 is KV, and the amino acid sequence of the LCDR3 is as set forth in SEQ ID NO: 41.

22. The isolated antigenic ITPRIPL1-binding protein according to claim 16, wherein, the amino acid sequence of the heavy chain variable region is as set forth in SEQ ID NO: 24; and the amino acid sequence of the light chain variable region is as set forth in any one of SEQ ID NO: 25 or SEQ ID NO: 35.

23. The isolated antigenic ITPRIPL1-binding protein according to any one of claims 15-22, comprising an antibody heavy chain constant region, and the antibody heavy chain constant region is derived from a human IgG heavy chain constant region.

24. The isolated antigenic ITPRIPL1-binding protein according to any one of claims 15-22, comprising an antibody light chain constant region, and the antibody light chain constant region comprises a human Igκ constant region.

25. The isolated antigenic ITPRIPL1-binding protein according to any one of claims 15-22, comprising an antibody heavy chain HC, and the HC comprises an amino acid sequence as set forth in any one of SEQ ID NO: 22 or 32.

26. The isolated antigenic ITPRIPL1-binding protein according to any one of claims 15-22, comprising an antibody light chain LC, and the LC comprises an amino acid sequence as set forth in any one of SEQ ID NO: 23 or 33.

27. The isolated antigenic ITPRIPL1-binding protein according to any one of claims 15-22, comprising an antibody or an antigen binding fragment thereof, wherein the antigen binding fragment comprises Fab, Fab', F(ab)2, Fv fragment, F(ab')2, scFv and/or di-scFv.

28. The isolated antigenic ITPRIPL1-binding protein according to any one of claims 15-22, the ITPRIPL1 protein comprises human and cynomolgus monkey ITPRIPL1 protein.

29. A chimeric antigen receptor, comprising the isolated antigenic ITPRIPL1-binding protein according to any one of claims 15-28.

30. An immunoconjugate, comprising the isolated antigenic ITPRIPL1-binding protein according to any one of claims 15-28.

31. One or more isolated nucleic acid molecules, encoding the isolated antigenic ITPRIPL1-binding protein according to any one of claims 15-28 or the chimeric antigen receptor according to claim 29, or encoding the polypeptide or protein fragment according to any one of claims 4 and 11.

32. A vector, comprising the nucleic acid molecules according to claim 31.

33. A cell, comprising the nucleic acid molecules according to claim 31 or the vector according to claim 32.

34. A pharmaceutical composition, comprising the isolated antigenic ITPRIPL1-binding protein according to any one of claims 15-28, the chimeric antigen receptor according to claim 29, the immunoconjugate according to claim 30, and optionally a pharmaceutically acceptable adjuvant.

35. The cell according to claim 33 is cultured under a condition enabling the expression of the isolated antigenic ITPRIPL1-binding protein according to any one of claims 15-28.

36. Uses of the isolated antigenic ITPRIPL1-binding protein according to any one of claims 15-28, the chimeric antigen receptor according to claim 29, the immunoconjugate according to claim 30, and/or the pharmaceutical composition according to claim 34 in the preparation of a drug which is used for preventing, alleviating and/or treating tumors.

37. The uses according to claim 36, wherein, the tumors comprise ITPRIPL1-associated tumors.

38. A method for screening and identifying ITPRIPL1-associated tumors, which determines by detecting the expression of ITPRIPL1 in a biological sample from a subject and comparing it with a preset value, the detection comprises detecting with antibodies, nucleic acid probes or PCR reactions involving specific primers, and the biological sample comprises tissue, blood, urine, cerebrospinal fluid, ascitic fluid, pleural fluid, tissue exudate, faeces and other samples.

39. An application of the method according to claim 38 in the preparation of a kit for tumor screening or diagnosis, and an application thereof in the preparation of a companion diagnostic kit for screening a patient for whom a regulator of ITPRIPL1 is indicated.

40. A linear epitope polypeptide of ITPRIPL1, the sequence of which comprises:
(i) an amino acid sequence of SEQ ID NO: 49, i.e., RLLEMEFEERKRAAE;
(ii) or, an amino acid sequence of xxLxxxFxxRxxx (x is any amino acid), in which 1-3 amino acids at both ends can be deleted;
(iii) or, an amino acid sequence obtained by substituting, inserting or deleting 1, 2, 3, 4, 5, 6, 7, 8, 9 or 10 amino acids of SEQ ID NO: 49.

41. A nucleic acid molecule, which is **characterized in that**, the nucleic acid molecule comprises a nucleic acid sequence encoding the peptide according to claim 40 or a complementary sequence thereof.

42. A vector, which is **characterized in that**, the vector comprises the nucleic acid molecule according to claim 41.

43. A host cell, which is **characterized in that**, the cell comprises the vector according to claim 42.

44. A pharmaceutical composition, which is **characterized in that**, the composition comprises a pharmaceutically acceptable carrier and the peptide according to claim 40, the vector according to claim 42 or the cell according to claim 43.

45. The epitope peptide and corresponding encoded nucleic acid, vector, host cell, and pharmaceutical composition according to claim 40-44 and uses thereof in the screening and preparation of a regulator of ITPRIPL1.

46. A method for searching and identifying the function regulator of ITPRIPL1 according to claims 1-36, which is **characterized in that**, one or more of the following properties of test molecules are detected: ability of specifically binding to ITPRIPL1 expressed on the cell surface; effect on the binding of ITPRIPL1 to CD3ε; effect on the binding of ITPRIPL1 to NRP2; effect on the binding of ITPRIPL1 to SEMA3G; effect on the binding of ITPRIPL1 to EBI2; and direct or indirect effect on the function of immune cells or tumor cells, wherein the effects can be those detectable in vitro or in vivo.

47. A use of an isolated antigenic ITPRIPL1-binding protein in the preparation of a drug that regulates immune responses or resists tumors as well as in the detection of the expression of ITPRIPL1 in an individual, wherein the isolated antigenic ITPRIPL1-binding protein is capable of binding to an amino acid sequence as set forth in SEQ ID NO: 49 or SEQ ID NO: 1 in the antigenic ITPRIPL1.
